# EUROPEAN PATENT APPLICATION

(11) **EP 3 309 166 A2**
(43) Date of publication of application: **18.04.2018**
(21) Application number: 17175566.3
(22) Date of filing: 23.01.2014
(51) Int. Cl.: C07J 71/00, C07J 5/00, C07J 1/00, C07J 3/00, C07J 7/00, C07J 9/00, C07J 11/00, C07J 17/00, C07J 21/00, C07J 31/00, C07J 41/00, C07J 43/00, A61K 31/56, A61P 21/00

(54) **11BETA-HYDROXY-STEROIDS FOR USE IN MITOCHONDRIA BIOGENESIS AND DISEASES ASSOCIATED WITH MITOCHONDRIAL DYSFUNCTION OR DEPLETION**

(30) Priority: 23.01.2013 IN 173DE2013
(62) Divisional of application: 14743866.7
(71) Applicant: Sphaera Pharma Pvt. Ltd., Haryana 122051 (IN)
(72) Inventor: Dugar, Sundeep, 122051 Haryana (IN); Schreiner, Frederic George, Los Altos Hills, CA California 94022 (US); Mahajan, Dinesh, 122051 Haryana (IN); Sharma, Amit, 122051 Haryana (IN); Patil, Ishwar Rakesh, 122051 Haryana (IN); Kuila, Bilash, 122051 Haryana (IN)
(74) Representative: ZBM Patents - Zea, Barlocci & Markvardsen

(57) **Abstract**

The present invention provides novel compounds of 11β-hydroxy steroids and compositions and their application as pharmaceuticals for preventing or reversing injury to mitochondria, for treating or preventing diseases relating to mitochondrial dysfunction or depletion, and for inducing regeneration or restructuring of mitochondria as a means of treating diseases relating to abnormalities in mitochondrial structure and function in a human or animal subject. Also disclosed herein are methods for diagnosing injury to mitochondria and for diagnosing the success or failure of therapeutics designed to treat, prevent, or reverse injury to or depletion of mitochondria.

## Description

### FIELD OF THE INVENTION

Disclosed herein are novel compounds of 11β-hydroxy steroids and compositions and their application as pharmaceuticals for preventing or reversing injury to mitochondria, for treating or preventing diseases relating to mitochondrial dysfunction or depletion, and for inducing regeneration or restructuring of mitochondria as a means of treating diseases relating to abnormalities in mitochondrial structure and function in a human or animal subject. Also disclosed herein are methods for diagnosing injury to mitochondria and for diagnosing the success or failure of therapeutics designed to treat, prevent, or reverse injury to or depletion of mitochondria.

### BACKGROUND

Mitochondria are responsible for generating more than 90% of the energy needed by the body to sustain life and support growth. When mitochondrial function fails, less energy is generated within the cell, resulting in cell injury and ultimately cell death. Mitochondria are susceptible to degradation due to oxygen radicals produced by their own metabolic processes. Damaged mitochondria involute and are expelled by the cell. Their replacement by new mitochondria is called mitochondrial biogenesis. The proliferation of mitochondria or their hypertrophy to meet increased metabolic demand is also called mitochondrial biogenesis. It is signified by the expression of additional mitochondrial proteins, particularly those related to oxidative phosphorylation. The capacity for mitochondrial biogenesis is significantly lost with age. Thus many diseases of aging are associated with loss of mitochondria in various tissues, whose specialized function is diminished in the context of diminished mitochondrial function and/or number. Many disease states, such as those that have neuromuscular disease symptoms, sarcopenia, muscular dystrophy, diabetes mellitus, dementia, Parkinson's disease, Huntington's disease, amyotrophic lateral sclerosis (ALS), obesity, hyperlipidemia, heart failure, lupus, and ocular conditions such as age-related macular degeneration (AMD), are associated with progressive mitochondrial loss in various tissues.

In addition, a number of drugs and drug classes also have an effect on mitochondrial function and biogenesis and can affect organ function and even lead to organ degeneration or other side effects which are directly related to the toxic effect of these drugs on the mitochondria.

A non-limiting list of drugs and drug classes that are associated with their mitochondria effect can be found in: Pereira et al., Current Drug Safety, 4:34-54, 2009; Gohil et al., Nature Biotechnol., 28:249-257, 2010; and Wagner et al., Nature Biotechnol., 26:343-351, 2008, each of which is hereby incorporated by reference in its entirety. Reflecting this understanding, the phrase "mitochondrial toxicity" as used herein refers to failure of the mitochondria resulting from the administration of chemical compositions to a subject.

Ischemic and ischemia/reperfusion injury are accompanied by decreases in mitochondrial function and number, leading to apoptotic cell death, necrosis, and functional organ deterioration in ischemic conditions such as myocardial infarction and stroke. Despite considerable advances in the diagnosis and treatment of such conditions, there remains a need for prophylactic and therapeutic approaches for the treatment of these conditions.

Mitochondria are critical to cell function and the effects of mitochondrial disease can be varied and can take on unique characteristics. The severity of the specific defect may be great or small and often affect the operation of the mitochondria and multiple tissues more severely, leading to multi-system diseases. Injury to, or dysfunction of, skeletal muscle mitochondria generally results in muscle weakness and atrophy, termed sarcopenia in severe states. In the case of generalized muscle weakness, reduction in bone density can be generalized, one of the causes of the bone disease known as osteoporosis. Depleted mitochondria in the heart can eventuate in the symptoms of congestive heart failure and eventual death. Loss of mitochondrial density in the brain is associated with neurodegeneration states such as Huntington's disease, Alzheimer's disease, and Parkinson's disease. Generalized loss of mitochondria including liver mitochondria can result in hyperlipidemia, hypertension, and insulin resistance progression to Type 2 diabetes. Liver mitochondria are injured by fructose uptake. Fructose, uric acid, and other agents injurious to liver mitochondria can cause accumulation of intracellular lipids, particularly triglycerides that contribute to the syndrome of hepatic steatosis, and increased synthesis and export of triglycerides that contributes to systemic hyperlipidemia, and ultimately obesity and insulin resistance.

Treatment options are currently limited and there remains a need for prophylactic and therapeutic approaches for the treatment of these conditions associated with chronic mitochondrial dysfunction and toxicity. Thus there is a need for treatments that stimulate mitochondrial function in response to increased metabolic demand and induce mitochondrial replication in response to agents or conditions that cause depletion of mitochondria in one or more tissues. Complicating potential therapies is the fact that the aging process is generally associated with progressive loss of the ability to support mitochondrial biogenesis, for reasons that are unknown.

### DESCRIPTION

It is an object of the invention to provide compounds which are derivatives of steroids and particularly those of 11β-hydroxy-steroids and other related steroids. These may find various uses as described hereinafter, in particular as pharmaceutical compounds.

Hydroxysteroids are hydroxylated compounds with a sterol structure and are known to be produced in cells when the mitochondria are exposed to high levels of endogenous H₂O₂ which then acts via the mitochondrial enzyme, 11β-hydroxylase, to hydroxylate a variety of steroids, including cholesterol, pregnenolone, progesterone, and others. Hydroxylation can occur in numerous positions, including the 7, 16, and 11 positions. These molecules, termed hydroxysteroids, are then sulfated and secreted into the extracellular space, where in the brain they modulate GABA-receptors and calcium channels on the plasma membrane. No intracellular activity of hydroxysteroids has previously been described.

In the present invention, compounds have structural Formula I: 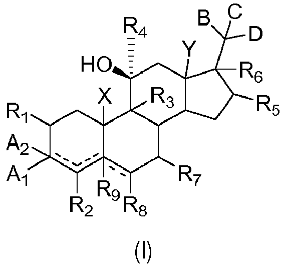 or a salt thereof, wherein:
a dashed line represents an optional double bond or a methylene group attached to the atoms on either side such that a fused cyclopropyl ring results, provided that cumulated double bonds (=C=) are not allowed; n is an integer from 1 to 6;
A₁ is selected from the group consisting of hydrogen, deuterium, halogen, -OH, PO₄³⁻, -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁;
A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom of the heterocylic ring or heteroaryl ring is selected from N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
B and C are each independently selected from the group consisting of hydrogen, -OH, -OR₁₀, -NR₁₀R₁₁, -OQ,-NR₁₀Q, -NR₁₁COR₁, -OCOOR₁, -NR₁₁COOR₁₀, -NR₁₁CONR₁₀;-COR₁₀, -COOR₁₀,-CONR₁₀R₁₁, -NH(CH₂)ₙNH_{2;,} -NR₁₁CO(CH₂)ₙNH₂ and C₁-C₁₂ alkyl, wherein said C₁-C₁₂ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁; or B and C, taken together with the atom to which they are attached, may form a 3-7 membered cycloalkyl, 4-7 membered heterocycloalkyl, or 5-6 membered heteroaryl, wherein heteroatom of the heterocylic ring or heteroaryl ring is selected from N, O or S;, any of which may be optionally substituted with one or more C₁-C₁₂ alkyl; wherein said C₁-C₁₂ alkyl may be optionally substituted with one or more substituents selected from the group consisting of fluorine, chlorine, -OR₁₀, -OCOR₁₀, -NR₁₁COR₁₀, -OCOOR₁₀, -NR₂COOR₁₀, -NR₁₁CONR₁₀R₁₁,-COR₁₀, -COOR₁₀, and -CONR₁₀R₁₁; or B and C, taken together, are =O, =N-OH or =N-NH₂;
D is selected from the group consisting of hydrogen, hydroxyl, methyl, ethyl, propyl, isopropyl, cyclopropyl, amino, hydroxyalkyl, -CD₃, -CF₃, -OR₁₀, -OCF₃, phenyl, 4-6 membered heterocycloalkyl, and 5-6 membered heteroaryl; wherein heteroatom in the said heteroalkyl or heteroaryl ring is selected from the group of N, O or S; wherein said 5-6 membered heterocycloalkyl or 5-6 membered heteroaryl may be optionally substituted with one or more substituents selected from the group consisting of C₁-C₄ alkyl, -R₁₀, -OR₁₀, -NR₁₀R₁₁, fluorine, chlorine, and -CF₃ or a C=O group ; or when taken together with B or C, may represent a double bond; or B, C and D, taken together with the atom to which they are attached, may form a carbonyl group, oxime group, phenyl, 4-6 membered heterocycloalkyl, or 5-6 membered heteroaryl; any of which may be optionally substituted with one or more substituents selected from the group consisting of -C₁-C₄ alkyl, -R₁₀,-OR₁₀, -NR₁₀R₁₁, fluorine, chlorine, -C=O and -CF₃;
R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₃ is selected from the group consisting of hydrogen, fluorine, chlorine, methyl, and -OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₈ and Rg are each selected from hydrogen, hydroxy, methoxy, amines, alkyl or acyl.
R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, amino, substituted amino, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl; Q is selected from the group consisting of: 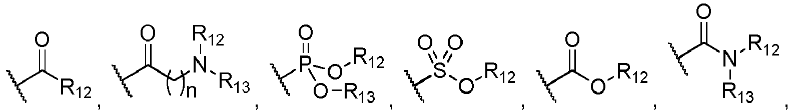 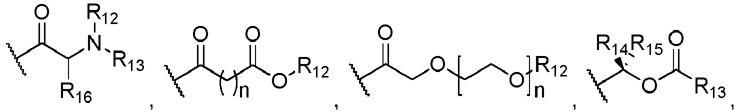 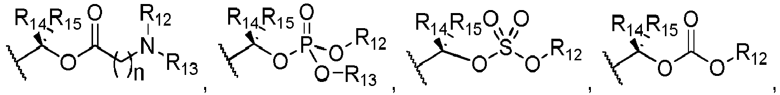 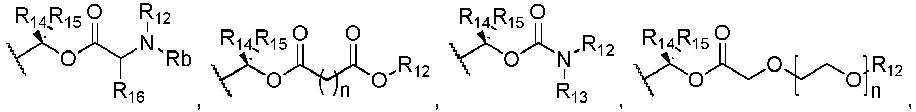 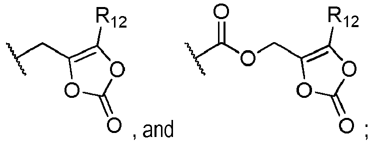 and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀, - NR₁₁CONR₁₀R₁₁, -COR₁₀, -COOR₁₀, and -CONR₁₀R₁₁.

Certain compounds disclosed herein may possess useful mitochondrial biogenesis modulating activity, and may be used in the treatment or prophylaxis of a disease or condition in which mitochondrial biogenesis plays an active role. Thus, in broad aspect, certain embodiments also provide pharmaceutical compositions comprising one or more compounds disclosed herein together with a pharmaceutically acceptable carrier, as well as methods of making and using the compounds and compositions. Certain embodiments provide methods for modulating mitochondrial biogenesis. Other embodiments provide methods for treating a mitochondrial biogenesis-mediated disorder in a patient in need of such treatment, comprising administering to said patient a therapeutically effective amount of a compound or composition according to the present invention. Also provided is the use of certain compounds disclosed herein for use in the manufacture of a medicament for the treatment of a disease or condition ameliorated by the modulation of mitochondrial biogenesis.

In the present invention, compounds have structural Formula II: 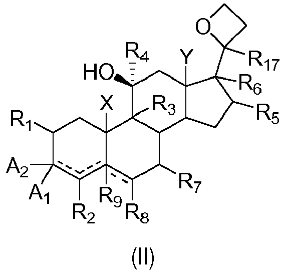 or a salt thereof, wherein:
a dashed line represents an optional double bond or a methylene group attached to the atoms on either side such that a fused cyclopropyl ring results, provided that cumulated double bonds (=C=) are not allowed;
n is an integer from 1 to 6;
m is an integer from 1 to 6;
A₁ is selected from the group consisting of hydrogen, -OH, deuterium, halogen, PO₄³⁻, -SO₄²⁻ -OR₁₀, -NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom in the said heteroalkyl or heteroaryl ring is selected from the group of N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀,-NR₁₁COR₁₀, -OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₃ is selected from the group consisting of hydrogen, fluorine, chlorine, methyl, and -OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₈ and Rg are each selected from hydrogen, hydroxy, methoxy, amines, alkyl or acyl.R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, amino, substituted amino, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl;
Q is selected from the group consisting of: 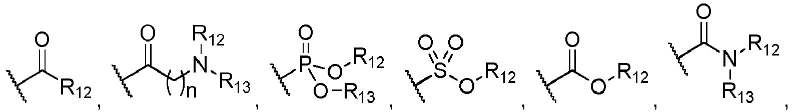 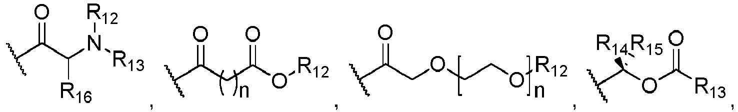 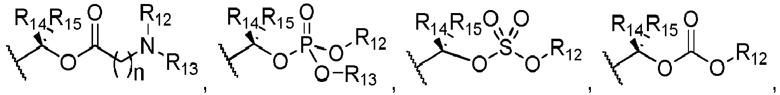 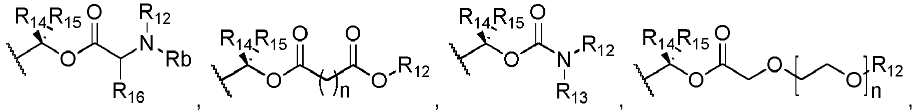 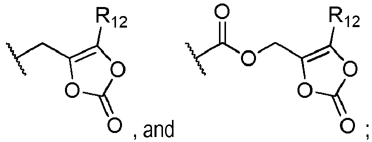 and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀,-NR₁₁CONR₁₀R₁₁, -COR₁₀, -COOR₁₀, and -CONR₁₀R₁₁.
R₆ may be hydrogen.

In the present invention, compounds have structural Formula III: 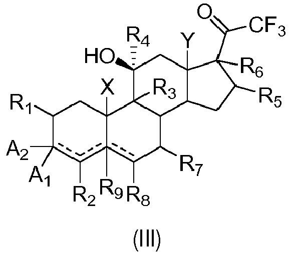 or a salt thereof, wherein:
a dashed line represents an optional double bond or a methylene group attached to the atoms on either side such that a fused cyclopropyl ring results, provided that cumulated double bonds (=C=) are not allowed;
n is an integer from 1 to 6;
A₁ is selected from the group consisting of hydrogen, deuterium, halogen, -OH, PO₄³⁻, -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁;
A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom in the said heteroalkyl or heteroaryl ring is selected from the group of N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀,-NR₁₁COR₁₀, -OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 4-7 membered cycloalkyl, 5-7 membered heterocycloalkyl, and 5-6 membered heteroaryl, wherein heteroatom in the said heteroalkyl or heteroaryl ring is selected from the group of N, O or S; any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₃ is selected from the group consisting of hydrogen, fluorine, chlorine, methyl, and -OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₈ and Rg are each selected from hydrogen, hydroxy, methoxy, amines, alkyl or acyl.
R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl; Q is selected from the group consisting of: 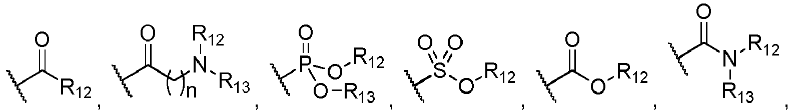 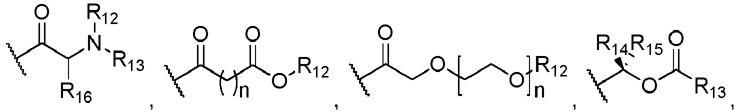 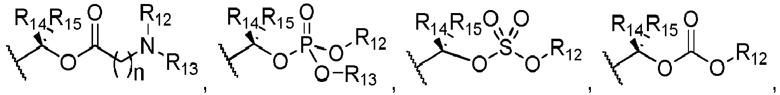 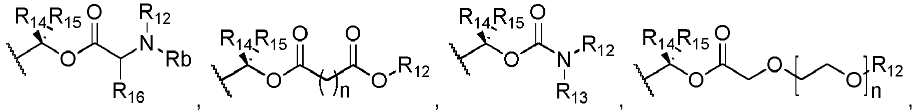 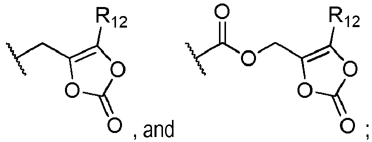 and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀, - NR₁₁CONR₁₀R₁₁, -COR₁₀, -COOR₁₀, and -CONR₁₀R₁₁.
R₆ may be hydrogen.

The compound of the present invention, compounds have structural Formula IV: 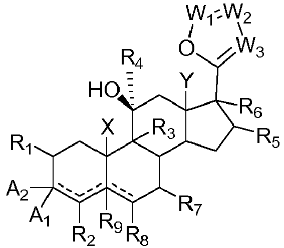 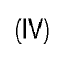 or a salt thereof, wherein:
a dashed line represents an optional double bond or a methylene group attached to the atoms on either side such that a fused cyclopropyl ring results, provided that cumulated double bonds (=C=) are not allowed;
n is an integer from 1 to 6;
A₁ is selected from the group consisting of hydrogen, deuterium, halogen, -OH, PO₄³⁻, -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R_{11,};
A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom of the heterocylic ring or heteroaryl ring is selected from N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₃ is selected from the group consisting of hydrogen, fluorine, chlorine, methyl, and -OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₈ and Rg are each selected from hydrogen, hydroxy, methoxy, amines, alkyl or acyl.
   R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl; Q is selected from the group consisting of: 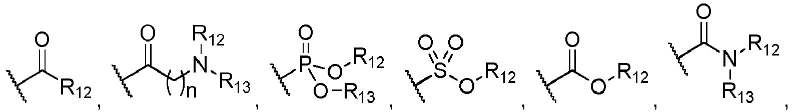 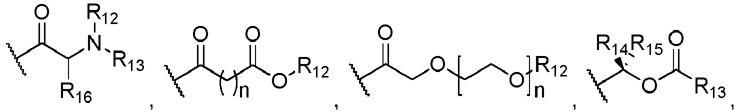 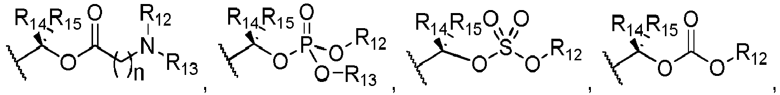 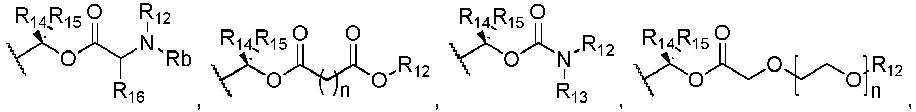 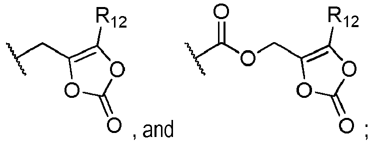
W₁, W₂ and W₃ are each independently selected from the group consisting of CH₂, CH, C, NH, N, NR₁₀, NQ, and O; and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀,-NR₁₁CONR₁₀R₁₁, -COR₁₀, -COOR₁₀, and -CONR₁₀R₁₁.

The present invention further provides compounds have structural Formula V: 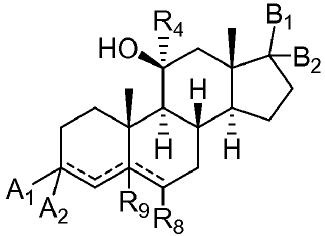 or a salt thereof, wherein:
a dashed line represents an optional double bond or a methylene group attached to the atoms on either side such that a fused cyclopropyl ring results, provided that cumulated double bonds (=C=) are not allowed; and it is understood by a person skilled in the art that the atoms are placed inside the molecule such that their valency is suitably satisfied;
A₁ and A₂ are each independently selected from the group consisting of hydrogen, deuterium, halogen, C₁-C₃ alkyl, hydroxy, C₁-C₃ alkoxy, -NH₂, PO₄³⁻ or -SO₄²⁻;
or A₁ and A₂, taken together, is selected from the group consisting of carbonyl or Oxime; or A₁ and A₂, taken together with the atom to which they are attached, may form a 3-7 membered cycloalkyl, 4-7 membered heterocycloalkyl comprising 1-3 heteroatoms selected from O or N, or 5-6 membered heteroaryl comprising 1-3 hereoatoms; wherein the heteroatom is selected from the group consisting of O, N and S.
R₁ and R₂ are each independently selected from the group consisting of hydrogen, deuterium, halogens, hydroxy, C₁-C₃ alkyl, C₁-C₃ alkoxy or -NH₂; or R₁ and R₂ taken together may be selected from an oxo group or an oxime group.
R₃ is selected from the group consisting of hydrogen, fluorine, chlorine, methyl, and -OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
B₁ and B₂ are each independently selected from the group consisting of hydrogen, deuterium, an -NH₂, a carbonyl, a C₁₋₅ alkoxy, -OH, -OR₃, -COR₃, -CONR₃R₄, -CONH(CH₂)ₙNH₂, -C(R₃)=N-OH or -C(R₃)=N-NH₂; or a C₁₋₅ alkyl optionally substituted with one or more substituents selected from the group consisting of an alkyl, amino, a hydroxyalkyl, a carbonyl group, a C₁₋₅ alkoxy, -OH, -OR₃, -COR₃, -CONR₃R₄, -CONH(CH₂)ₙNH₂,-C(R₃)=N-OH or -NHCOCH₂NH₂; or B₁ is a 3-7 membered cycloalkyl or 4-7 membered heterocycloalkyl or heterocycloalkenyl group comprising 1-3 heteroatoms selected from O or N, or 5-6 membered heteroaryl comprising 1-3 hereoatoms selected from the group consisting of O, N and S; any of which may be optionally substituted with one or more C₁-C₃ alkyl or a carbonyl group; or B₁ and B₂, taken together, is an oxo or an oxime group; or B₁ and B₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of hydrogen, -NH₂, hydroxyl, carbonyl, C₁₋₅ alkyl or a C₁₋₅ alkoxy group;
R₈ and Rg are each selected from hydrogen, hydroxy, methoxy, amines, alkyl or acyl. With the proviso that the 11^{th} position is β in configuration.

The compound of the present invention is selected from the group consisting of Examples 1 to 70.

In certain embodiments, disclosed herein is a method of treatment of a mitochondrial biogenesis-mediated disease comprising the administration of a therapeutically effective amount of a compound as disclosed herein to a patient in need thereof.

The mitochondrial biogenesis-mediated disease is selected from the group consisting of skeletal or cardiac muscle diseases associated with ischemia, or impaired or inadequate blood flow, diseases associated with genetic disorders that directly or indirectly affect the number, structure, or function of mitochondria, diseases associated with impaired neurological function associated with decreased mitochondrial number or function, diseases associated with loss of number, loss of function, or loss of correct, optimally efficient internal organization of skeletal muscle cells or cardiac muscle cells, metabolic diseases, and conditions associated with liver cell injury and altered fatty acid metabolism.

The mitochondrial biogenesis-mediated disease is selected from the group consisting of acute coronary syndrome, myocardial infarction, angina, renal injury, renal ischemia, diseases of the aorta and its branches, injuries arising from medical interventions, atherosclerosis, trauma, diabetes, hyperlipidemia, vascular stenosis, peripheral arterial disease, vasculopathy, and vasculitis.

The mitochondrial biogenesis-mediated disease is selected from the group consisting of Friedreich's ataxia, muscular dystrophy, Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, spinal muscular atrophy, and Emery-Dreifuss muscular dystrophy.

The mitochondrial biogenesis-mediated disease is selected from the group consisting of Huntington's disease, Parkinson's disease, Alzheimer's disease, and amyotrophic lateral sclerosis.

The mitochondrial biogenesis-mediated disease is selected from the group consisting of sarcopenia.

The mitochondrial biogenesis-mediated disease is selected from the group consisting of congestive heart failure, aging, myocarditis, myositis, polymyalgia rheumatic, polymyositis, HIV, cancer and/or the side effects of chemotherapy targeting the cancer, malnutrition, aging, inborn errors of metabolism, trauma, and stroke or other types of neurological impairment.

The mitochondrial biogenesis-mediated disease is selected from the group consisting of hepatic steatosis, hepatic fibrosis, cirrhosis, and hepatocyte or stellate cell injury.

In further embodiments, said method further comprises the administration of another therapeutic agent.

The said agent is selected from the group consisting of hormones which stimulate muscle cell growth, γ-amino butyric acid or its derivatives, dietary protein supplements, anabolic steroids, biological factors known to enhance the growth, strength, endurance, or metabolism of skeletal or cardiac muscle, or recovery of skeletal muscle or cardiac muscle from injury or weakness, compounds known to be associated with increased nitric oxide production which promotes blood flow through muscles, extracts of natural products known to promote muscle strength or endurance, inhibitors of myostatin, stimulators of folistatin expression, compounds known to promote or facilitate mitochochondrial function or biogenesis, a tetracycline antibiotic, glycoprotein IIb/IIIa inhibitor, ADP receptor/P2Y12 inhibitor, prostaglandin analog, COX inhibitor, antiplatelet drug, anticoagulant, heparin, direct factor Xa inhibitor, direct thrombin (II) inhibitor, vasodilator.

The said agent is doxycycline.

The said agent is niacin or allopurinol.

In certain embodiments, disclosed herein are methods of treating or preventing the adverse effects of administration of compounds which exhibit mitochondrial toxicity comprising the administration of a therapeutically effective amount of a compound as disclosed herein to a patient in need thereof.

The adverse effect is selected from the group consisting of abnormal mitochondrial respiration, abnormal oxygen consumption, abnormal extracellular acidification rate, abnormal mitochondrial number, abnormal lactate accumulation, and abnormal ATP levels.

In certain embodiments, disclosed herein are methods of improving muscle structure or function; improving mitochondrial effects associated with exercise; enhancing the capacity for exercise in those limited by age, inactivity, diet, or diseases; enhancing muscle health and function in response to exercise; enhancing muscle health and function in the clinical setting of restricted capacity for exercise; enhancing recovery of muscles from vigorous activity or from injury associated with vigorous or sustained activity, comprising the administration of a therapeutically effective amount of a compound as disclosed herein to a patient in need thereof.

In certain embodiments, disclosed herein are methods of enhancing sports performance and endurance, building muscle shape and strength, or facilitating recovery from the muscle related side effects of training or competition comprising the administration of a therapeutically effective amount of a compound as disclosed herein to a patient in need thereof.

In certain embodiments, disclosed herein are methods of stimulating increased number or function of skeletal muscle cells or contractile muscle cells comprising the administration of a therapeutically effective amount of a compound as disclosed herein to a patient in need thereof.

The said stimulation of muscle cells comprises stimulation of cell division, muscle cell regeneration, activation of muscle satellite cells and their differentiation into adult muscle cells, recovery from injury, increased number or function of mitochondria or processes serving mitochondrial function, increased expression of proteins contributing to contractility, regulation of biochemical or translational processes, mitoses, or transduction of mechanical energy via dystrophin or other attachment processes.

In certain embodiments, disclosed herein is a method of modulation of mitochondrial biogenesis comprising contacting mitochondria with a compound as disclosed herein.

The mitochondrial biogenesis-mediated diseases include sarcopenia, muscular dystrophy, neurodegenerative diseases, liver disease, acute or chronic kidney failure, congestive heart failure, chronic obstructive pulmonary disorder (COPD), peripheral vascular disease, pulmonary hypertension, hyperlipidemia, hypertension, and diabetes.

The present invention comprises administering a compound or composition disclosed herein in an amount effective to stimulate the function, recovery, or regeneration of mitochondria or mitochondrial proteins or function. In further embodiments, the present invention provides methods for preventing or treating adverse events or diseases associated with impaired mitochondrial number or function.

The term "about," as used herein, is intended to qualify the numerical values which it modifies, denoting such a value as variable within a margin of error. When no particular margin of error, such as a standard deviation to a mean value given in a chart or table of data, is recited, the term "about" should be understood to mean that range which would encompass the recited value and the range which would be included by rounding up or down to that figure as well, taking into account significant figures.

The term "acyl," as used herein, alone or in combination, refers to a carbonyl attached to an alkenyl, alkyl, aryl, cycloalkyl, heteroaryl, heterocycle, or any other moiety were the atom attached to the carbonyl is carbon. An "acetyl" group refers to a -C(O)CH₃ group. An "alkylcarbonyl" or "alkanoyl" group refers to an alkyl group attached to the parent molecular moiety through a carbonyl group. Examples of such groups include methylcarbonyl and ethylcarbonyl. Examples of acyl groups include formyl, alkanoyl and aroyl.

The term "alkenyl," as used herein, alone or in combination, refers to a straight-chain or branched-chain hydrocarbon radical having one or more double bonds and containing from 2 to 20 carbon atoms. In certain embodiments, said alkenyl will comprise from 2 to 6 carbon atoms. The term "alkenylene" refers to a carbon-carbon double bond system attached at two or more positions such as ethenylene [(-CH=CH-),(-C::C-)]. Examples of suitable alkenyl radicals include ethenyl, propenyl, 2-methylpropenyl, 1,4-butadienyl and the like. Unless otherwise specified, the term "alkenyl" may include "alkenylene" groups.

The term "alkoxy," as used herein, alone or in combination, refers to an alkyl ether radical, wherein the term alkyl is as defined below. Examples of suitable alkyl ether radicals include methoxy, ethoxy, n-propoxy, isopropoxy, n-butoxy, iso-butoxy, sec-butoxy, tert-butoxy, and the like.

The term "alkyl," as used herein, alone or in combination, refers to a straight-chain or branched-chain alkyl radical containing from 1 to 20 carbon atoms. In certain embodiments, said alkyl will comprise from 1 to 10 carbon atoms. In further embodiments, said alkyl will comprise from 1 to 6 carbon atoms. Alkyl groups may be optionally substituted as defined herein. Examples of alkyl radicals include methyl, ethyl, n-propyl, isopropyl, n-butyl, isobutyl, sec-butyl, tert-butyl, pentyl, iso-amyl, hexyl, octyl, noyl and the like. The term "alkylene," as used herein, alone or in combination, refers to a saturated aliphatic group derived from a straight or branched chain saturated hydrocarbon attached at two or more positions, such as methylene (-CH₂-). Unless otherwise specified, the term "alkyl" may include "alkylene" groups.

The term "alkylamino," as used herein, alone or in combination, refers to an alkyl group attached to the parent molecular moiety through an amino group. Suitable alkylamino groups may be mono- or dialkylated, forming groups such as, for example, N-methylamino, N-ethylamino, N,N-dimethylamino, N,N-ethylmethylamino and the like.

The term "alkylidene," as used herein, alone or in combination, refers to an alkenyl group in which one carbon atom of the carbon-carbon double bond belongs to the moiety to which the alkenyl group is attached.

The term "alkylthio," as used herein, alone or in combination, refers to an alkyl thioether (R-S-) radical wherein the term alkyl is as defined above and wherein the sulfur may be singly or doubly oxidized. Examples of suitable alkyl thioether radicals include methylthio, ethylthio, n-propylthio, isopropylthio, n-butylthio, iso-butylthio, sec-butylthio, tert-butylthio, methanesulfonyl, ethanesulfinyl, and the like.

The term "alkynyl," as used herein, alone or in combination, refers to a straight-chain or branched chain hydrocarbon radical having one or more triple bonds and containing from 2 to 20 carbon atoms. In certain embodiments, said alkynyl comprises from 2 to 6 carbon atoms. In further embodiments, said alkynyl comprises from 2 to 4 carbon atoms. The term "alkynylene" refers to a carbon-carbon triple bond attached at two positions such as ethynylene (-C:::C-, -C≡C-). Examples of alkynyl radicals include ethynyl, propynyl, hydroxypropynyl, butyn-1-yl, butyn-2-yl, pentyn-1-yl, 3-methylbutyn-1-yl, hexyn-2-yl, and the like. Unless otherwise specified, the term "alkynyl" may include "alkynylene" groups.

The terms "amido" and "carbamoyl,"as used herein, alone or in combination, refer to an amino group as described below attached to the parent molecular moiety through a carbonyl group, or vice versa. The term "C-amido" as used herein, alone or in combination, refers to a -C(O)N(RR') group with R and R' as defined herein or as defined by the specifically enumerated "R" groups designated. The term "N-amido" as used herein, alone or in combination, refers to a RC(O)N(R')- group, with R and R' as defined herein or as defined by the specifically enumerated "R" groups designated. The term "acylamino" as used herein, alone or in combination, embraces an acyl group attached to the parent moiety through an amino group. An example of an "acylamino" group is acetylamino (CH₃C(O)NH-).

The term "amino," as used herein, alone or in combination, refers to -NRR', wherein R and R' are independently selected from the group consisting of hydrogen, alkyl, acyl, heteroalkyl, aryl, cycloalkyl, heteroaryl, and heterocycloalkyl, any of which may themselves be optionally substituted. Additionally, R and R' may combine to form heterocycloalkyl, either of which may be optionally substituted.

The term "aryl," as used herein, alone or in combination, means a carbocyclic aromatic system containing one, two or three rings wherein such polycyclic ring systems are fused together. The term "aryl" embraces aromatic groups such as phenyl, naphthyl, anthracenyl, and phenanthryl.

The term "arylalkenyl" or "aralkenyl," as used herein, alone or in combination, refers to an aryl group attached to the parent molecular moiety through an alkenyl group.

The term "arylalkoxy" or "aralkoxy," as used herein, alone or in combination, refers to an aryl group attached to the parent molecular moiety through an alkoxy group.

The term "arylalkyl" or "aralkyl," as used herein, alone or in combination, refers to an aryl group attached to the parent molecular moiety through an alkyl group.

The term "arylalkynyl" or "aralkynyl," as used herein, alone or in combination, refers to an aryl group attached to the parent molecular moiety through an alkynyl group.

The term "arylalkanoyl" or "aralkanoyl" or "aroyl,"as used herein, alone or in combination, refers to an acyl radical derived from an aryl-substituted alkanecarboxylic acid such as benzoyl, napthoyl, phenylacetyl, 3-phenylpropionyl (hydrocinnamoyl), 4-phenylbutyryl, (2-naphthyl)acetyl, 4-chlorohydrocinnamoyl, and the like.

The term aryloxy as used herein, alone or in combination, refers to an aryl group attached to the parent molecular moiety through an oxy.

The terms "benzo" and "benz," as used herein, alone or in combination, refer to the divalent radical C₆H₄= derived from benzene. Examples include benzothiophene and benzimidazole.

The term "carbamate," as used herein, alone or in combination, refers to an ester of carbamic acid (-NHCOO-) which may be attached to the parent molecular moiety from either the nitrogen or acid end, and which may be optionally substituted as defined herein.

The term "O-carbamyl" as used herein, alone or in combination, refers to a -OC(O)NRR', group-with R and R' as defined herein.

The term "N-carbamyl" as used herein, alone or in combination, refers to a ROC(O)NR'- group, with R and R' as defined herein.

The term "carbonyl," as used herein, when alone includes formyl [-C(O)H] and in combination is a -C(O)-group.

The term "carboxyl" or "carboxy," as used herein, refers to -C(O)OH or the corresponding "carboxylate" anion, such as is in a carboxylic acid salt. An "O-carboxy" group refers to a RC(O)O- group, where R is as defined herein. A "C-carboxy" group refers to a -C(O)OR groups where R is as defined herein.

The term "cyano," as used herein, alone or in combination, refers to -CN.

The term "cycloalkyl," or, alternatively, "carbocycle," as used herein, alone or in combination, refers to a saturated or partially saturated monocyclic, bicyclic or tricyclic alkyl group wherein each cyclic moiety contains from 3 to 12 carbon atom ring members and which may optionally be a benzo fused ring system which is optionally substituted as defined herein. In certain embodiments, said cycloalkyl will comprise from 5 to 7 carbon atoms. Examples of such cycloalkyl groups include cyclopropyl, cyclobutyl, cyclopentyl, cyclohexyl, cycloheptyl, tetrahydronapthyl, indanyl, octahydronaphthyl, 2,3-dihydro-1H-indenyl, adamantyl and the like. "Bicyclic" and "tricyclic" as used herein are intended to include both fused ring systems, such as decahydronaphthalene, octahydronaphthalene as well as the multicyclic (multicentered) saturated or partially unsaturated type. The latter type of isomer is exemplified in general by, bicyclo[1,1,1]pentane, camphor, adamantane, and bicyclo[3,2,1]octane.

The term "ester," as used herein, alone or in combination, refers to a carboxy group bridging two moieties linked at carbon atoms.

The term "ether," as used herein, alone or in combination, refers to an oxy group bridging two moieties linked at carbon atoms.

The term "halo," or "halogen," as used herein, alone or in combination, refers to fluorine, chlorine, bromine, or iodine.

The term "haloalkoxy," as used herein, alone or in combination, refers to a haloalkyl group attached to the parent molecular moiety through an oxygen atom.

The term "haloalkyl," as used herein, alone or in combination, refers to an alkyl radical having the meaning as defined above wherein one or more hydrogens are replaced with a halogen. Specifically embraced are monohaloalkyl, dihaloalkyl and polyhaloalkyl radicals. A monohaloalkyl radical, for one example, may have an iodo, bromo, chloro or fluoro atom within the radical. Dihalo and polyhaloalkyl radicals may have two or more of the same halo atoms or a combination of different halo radicals. Examples of haloalkyl radicals include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl. "Haloalkylene" refers to a haloalkyl group attached at two or more positions. Examples include fluoromethylene (-CFH-), difluoromethylene (-CF₂-), chloromethylene (-CHCl-) and the like.

The term "heteroalkyl," as used herein, alone or in combination, refers to a stable straight or branched chain, or cyclic hydrocarbon radical, or combinations thereof, fully saturated or containing from 1 to 3 degrees of unsaturation, consisting of the stated number of carbon atoms and from one to three heteroatoms selected from the group consisting of O, N, and S, and wherein the nitrogen and sulfur atoms may optionally be oxidized and the nitrogen heteroatom may optionally be quaternized. The heteroatom(s) O, N and S may be placed at any interior position of the heteroalkyl group. Up to two heteroatoms may be consecutive, such as, for example, -CH₂-NH-OCH₃.

The term "heteroaryl," as used herein, alone or in combination, refers to a 3 to 15 membered unsaturated heteromonocyclic ring, or a fused monocyclic, bicyclic, or tricyclic ring system in which at least one of the fused rings is aromatic, which contains at least one atom selected from the group consisting of O, S, and N. In certain embodiments, said heteroaryl will comprise from 5 to 7 carbon atoms. The term also embraces fused polycyclic groups wherein heterocyclic rings are fused with aryl rings, wherein heteroaryl rings are fused with other heteroaryl rings, wherein heteroaryl rings are fused with heterocycloalkyl rings, or wherein heteroaryl rings are fused with cycloalkyl rings. Examples of heteroaryl groups include pyrrolyl, pyrrolinyl, imidazolyl, pyrazolyl, pyridyl, pyrimidinyl, pyrazinyl, pyridazinyl, triazolyl, pyranyl, furyl, thienyl, oxazolyl, isoxazolyl, oxadiazolyl, thiazolyl, thiadiazolyl, isothiazolyl, indolyl, isoindolyl, indolizinyl, benzimidazolyl, quinolyl, isoquinolyl, quinoxalinyl, quinazolinyl, indazolyl, benzotriazolyl, benzodioxolyl, benzopyranyl, benzoxazolyl, benzoxadiazolyl, benzothiazolyl, benzothiadiazolyl, benzofuryl, benzothienyl, chromonyl, coumarinyl, benzopyranyl, tetrahydroquinolinyl, tetrazolopyridazinyl, tetrahydroisoquinolinyl, thienopyridinyl, furopyridinyl, pyrrolopyridinyl and the like. Exemplary tricyclic heterocyclic groups include carbazolyl, benzidolyl, phenanthrolinyl, dibenzofuranyl, acridinyl, phenanthridinyl, xanthenyl and the like.

The terms "heterocycloalkyl" and, interchangeably, "heterocycle," as used herein, alone or in combination, each refer to a saturated, partially unsaturated, or fully unsaturated monocyclic, bicyclic, or tricyclic heterocyclic group containing at least one heteroatom as a ring member, wherein each said heteroatom may be independently selected from the group consisting of nitrogen, oxygen, and sulfur In certain embodiments, said hetercycloalkyl will comprise from 1 to 4 heteroatoms as ring members. In further embodiments, said hetercycloalkyl will comprise from 1 to 2 heteroatoms as ring members. In certain embodiments, said hetercycloalkyl will comprise from 3 to 8 ring members in each ring. In further embodiments, said hetercycloalkyl will comprise from 3 to 7 ring members in each ring. In yet further embodiments, said hetercycloalkyl will comprise from 5 to 6 ring members in each ring. "Heterocycloalkyl" and "heterocycle" are intended to include sulfones, sulfoxides, N-oxides of tertiary nitrogen ring members, and carbocyclic fused and benzo fused ring systems; additionally, both terms also include systems where a heterocycle ring is fused to an aryl group, as defined herein, or an additional heterocycle group. Examples of heterocycle groups include aziridinyl, azetidinyl, 1,3-benzodioxolyl, dihydroisoindolyl, dihydroisoquinolinyl, dihydrocinnolinyl, dihydrobenzodioxinyl, dihydro[1,3]oxazolo[4,5-b]pyridinyl, benzothiazolyl, dihydroindolyl, dihy-dropyridinyl, 1,3-dioxanyl, 1,4-dioxanyl, 1,3-dioxolanyl, isoindolinyl, morpholinyl, piperazinyl, pyrrolidinyl, tetrahydropyridinyl, piperidinyl, thiomorpholinyl, and the like. The heterocycle groups may be optionally substituted unless specifically prohibited.

The term "hydrazinyl" as used herein, alone or in combination, refers to two amino groups joined by a single bond, i.e., -N-N-.

The term "hydroxy," as used herein, alone or in combination, refers to -OH.

The term "hydroxyalkyl," as used herein, alone or in combination, refers to a hydroxy group attached to the parent molecular moiety through an alkyl group.

The term "imino," as used herein, alone or in combination, refers to =N-.

The term "iminohydroxy," as used herein, alone or in combination, refers to =N(OH) and =N-O-.

The phrase "in the main chain" refers to the longest contiguous or adjacent chain of carbon atoms starting at the point of attachment of a group to the compounds of any one of the formulas disclosed herein.

The term "isocyanato" refers to a -NCO group.

The term "isothiocyanato" refers to a -NCS group.

The phrase "linear chain of atoms" refers to the longest straight chain of atoms independently selected from carbon, nitrogen, oxygen and sulfur.

The term "lower," as used herein, alone or in a combination, where not otherwise specifically defined, means containing from 1 to and including 6 carbon atoms.

The term "lower aryl," as used herein, alone or in combination, means phenyl or naphthyl, either of which may be optionally substituted as provided.

The term "lower heteroaryl," as used herein, alone or in combination, means either 1) monocyclic heteroaryl comprising five or six ring members, of which between one and four said members may be heteroatoms selected from the group consisting of O, S, and N, or 2) bicyclic heteroaryl, wherein each of the fused rings comprises five or six ring members, comprising between them one to four heteroatoms selected from the group consisting of O, S, and N.

The term "lower cycloalkyl," as used herein, alone or in combination, means a monocyclic cycloalkyl having between three and six ring members. Lower cycloalkyls may be unsaturated. Examples of lower cycloalkyl include cyclopropyl, cyclobutyl, cyclopentyl, and cyclohexyl.

The term "lower heterocycloalkyl," as used herein, alone or in combination, means a monocyclic heterocycloalkyl having between three and six ring members, of which between one and four may be heteroatoms selected from the group consisting of O, S, and N. Examples of lower heterocycloalkyls include pyrrolidinyl, imidazolidinyl, pyrazolidinyl, piperidinyl, piperazinyl, and morpholinyl. Lower heterocycloalkyls may be unsaturated.

The term "lower amino," as used herein, alone or in combination, refers to -NRR', wherein R and R' are independently selected from the group consisting of hydrogen, lower alkyl, and lower heteroalkyl, any of which may be optionally substituted. Additionally, the R and R' of a lower amino group may combine to form a five- or six-membered heterocycloalkyl, either of which may be optionally substituted.

The term "mercaptyl" as used herein, alone or in combination, refers to an RS- group, where R is as defined herein.

The term "nitro," as used herein, alone or in combination, refers to NO₂.

The terms "oxy" or "oxa," as used herein, alone or in combination, refer to -O-.

The term "oxo," as used herein, alone or in combination, refers to =O.

The term "perhaloalkoxy" refers to an alkoxy group where all of the hydrogen atoms are replaced by halogen atoms.

The term "perhaloalkyl" as used herein, alone or in combination, refers to an alkyl group where all of the hydrogen atoms are replaced by halogen atoms.

The terms "sulfonate," "sulfonic acid," and "sulfonic," as used herein, alone or in combination, refer the -SO₃H group and its anion as the sulfonic acid is used in salt formation.

The term "sulfanyl," as used herein, alone or in combination, refers to -S-.

The term "sulfinyl," as used herein, alone or in combination, refers to
-S(O)-.

The term "sulfonyl," as used herein, alone or in combination, refers to -S(O)₂-.

The term "N-sulfonamido" refers to a RS(=O)₂NR'- group with R and R' as defined herein.

The term "S-sulfonamido" refers to a -S(=O)₂NRR', group, with R and R' as defined herein.

The terms "thia" and "thio," as used herein, alone or in combination, refer to a -S- group or an ether wherein the oxygen is replaced with sulfur. The oxidized derivatives of the thio group, namely sulfinyl and sulfonyl, are included in the definition of thia and thio.

The term "thiol," as used herein, alone or in combination, refers to an -SH group.

The term "thiocarbonyl," as used herein, when alone includes thioformyl -C(S)H and in combination is a-C(S)- group.

The term "N-thiocarbamyl" refers to an ROC(S)NR'- group, with R and R'as defined herein.

The term "O-thiocarbamyl" refers to a -OC(S)NRR', group with R and R'as defined herein.

The term "thiocyanato" refers to a -CNS group.

The term "trihalomethanesulfonamido" refers to a X₃CS(O)₂NR- group with X is a halogen and R as defined herein.

The term "trihalomethanesulfonyl" refers to a X₃CS(O)₂- group where X is a halogen.

The term "trihalomethoxy" refers to a X₃CO- group where X is a halogen.

The term "trisubstituted silyl," as used herein, alone or in combination, refers to a silicone group substituted at its three free valences with groups as listed herein under the definition of substituted amino. Examples include trimethysilyl, tert-butyldimethylsilyl, triphenylsilyl and the like.

"Deuterated compounds" encompassed within the scope of this invention are the compounds which have selective incorporation of deuterium in place of hydrogen.

Any definition herein may be used in combination with any other definition to describe a composite structural group. By convention, the trailing element of any such definition is that which attaches to the parent moiety. For example, the composite group alkylamido would represent an alkyl group attached to the parent molecule through an amido group, and the term alkoxyalkyl would represent an alkoxy group attached to the parent molecule through an alkyl group.

When a group is defined to be "null," what is meant is that said group is absent.

The term "optionally substituted" means the anteceding group may be substituted or unsubstituted. When substituted, the substituents of an "optionally substituted" group may include, without limitation, one or more substituents independently selected from the following groups or a particular designated set of groups, alone or in combination: lower alkyl, lower alkenyl, lower alkynyl, lower alkanoyl, lower heteroalkyl, lower heterocycloalkyl, lower haloalkyl, lower haloalkenyl, lower haloalkynyl, lower perhaloalkyl, lower perhaloalkoxy, lower cycloalkyl, phenyl, aryl, aryloxy, lower alkoxy, lower haloalkoxy, oxo, lower acyloxy, carbonyl, carboxyl, lower alkylcarbonyl, lower carboxyester, lower carboxamido, cyano, hydrogen, halogen, hydroxy, amino, lower alkylamino, arylamino, amido, nitro, thiol, lower alkylthio, lower haloalkylthio, lower perhaloalkylthio, arylthio, sulfonate, sulfonic acid, trisubstituted silyl, N₃, SH, SCH₃, C(O)CH₃, CO₂CH₃, CO₂H, pyridinyl, thiophene, furanyl, lower carbamate, and lower urea. Two substituents may be joined together to form a fused five-, six-, or seven-membered carbocyclic or heterocyclic ring consisting of zero to three heteroatoms, for example forming methylenedioxy or ethylenedioxy. An optionally substituted group may be unsubstituted (e.g., -CH₂CH₃), fully substituted (e.g., -CF₂CF₃), monosubstituted (e.g., -CH₂CH₂F) or substituted at a level anywhere in-between fully substituted and monosubstituted (e.g., -CH₂CF₃). Where substituents are recited without qualification as to substitution, both substituted and unsubstituted forms are encompassed. Where a substituent is qualified as "substituted," the substituted form is specifically intended. Additionally, different sets of optional substituents to a particular moiety may be defined as needed; in these cases, the optional substitution will be as defined, often immediately following the phrase, "optionally substituted with."

The term R or the term R', appearing by itself and without a number designation, unless otherwise defined, refers to a moiety selected from the group consisting of hydrogen, alkyl, cycloalkyl, heteroalkyl, aryl, heteroaryl and heterocycloalkyl, any of which may be optionally substituted. Such R and R' groups should be understood to be optionally substituted as defined herein. Whether an R group has a number designation or not, every R group, including R, R' and Rⁿ where n=(1, 2, 3, ...n), every substituent, and every term should be understood to be independent of every other in terms of selection from a group. Should any variable, substituent, or term (e.g. aryl, heterocycle, R, etc.) occur more than one time in a formula or generic structure, its definition at each occurrence is independent of the definition at every other occurrence. Those of skill in the art will further recognize that certain groups may be attached to a parent molecule or may occupy a position in a chain of elements from either end as written. Thus, by way of example only, an unsymmetrical group such as -C(O)N(R)- may be attached to the parent moiety at either the carbon or the nitrogen.

Asymmetric centers exist in the compounds disclosed herein. These centers are designated by the symbols "R" or "S," depending on the configuration of substituents around the chiral carbon atom. It should be understood that the invention encompasses all stereochemical isomeric forms, including diastereomeric, enantiomeric, and epimeric forms as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention. Additionally, the compounds disclosed herein can exist in unsolvated as well as solvated forms with pharmaceutically acceptable solvents such as water, ethanol, and the like. In general, the solvated forms are considered equivalent to the unsolvated forms. The term "bond" refers to a covalent linkage between two atoms, or two moieties when the atoms joined by the bond are considered to be part of larger substructure. A bond may be single, double, or triple unless otherwise specified. A dashed line between two atoms in a drawing of a molecule indicates that an additional bond may be present or absent at that position.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "muscular diseases" refers to diseases associated with impaired skeletal muscle or cardiac muscle cell number or function.

The term "combination therapy" means the administration of two or more therapeutic agents to treat a therapeutic condition or disorder described in the present disclosure. Such administration encompasses coadministration of these therapeutic agents in a substantially simultaneous manner, such as in a single capsule having a fixed ratio of active ingredients or in multiple, separate capsules for each active ingredient. In addition, such administration also encompasses use of each type of therapeutic agent in a sequential manner. In either case, the treatment regimen will provide beneficial effects of the drug combination in treating the conditions or disorders described herein. In certain embodiments, a combination of compounds is administered such that the clearance half-life of each compound from the body overlaps at least partially with one another. For example, a first pharmaceutical has a clearance half-life of 1 hour and is administered at time=0, and a second pharmaceutical has a clearance half-life of 1 hour and is administered at time=45 minutes.

The phrase "therapeutically effective" is intended to qualify the amount of active ingredients used in the treatment of a disease or disorder or on the effecting of a clinical endpoint.

The term "therapeutically acceptable" refers to those compounds (or salts, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.

As used herein, reference to "treatment" of a patient is intended to include prophylaxis. Treatment may also be preemptive in nature, i.e., it may include prevention of disease. Prevention of a disease may involve complete protection from disease, for example as in the case of prevention of infection with a pathogen, or may involve prevention of disease progression. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease to a later stage of the disease.

The term "patient" is generally synonymous with the term "subject" and includes all mammals including humans. Examples of patients include humans, livestock such as cows, goats, sheep, pigs, and rabbits, and companion animals such as dogs, cats, rabbits, and horses. Preferably, the patient is a human.

The term "pregnenolone and other related steroids" as used herein refers to any compound which retains the ring structure and the 11-oxo moiety of 11-hydroxy-pregnenolone itself, but which contains one or more substituent groups relative to 11-oxo-pregenolone. The term also includes prodrugs which release 11-hydroxy-pregnenolone when administered to a subject. The term also includes active metabolites of 11β-hydroxypregnenolone such as 11β-hydroxyprogesterone.

The term "11β-hydroxypregnenolone" as used herein refers to a compound having the structural formula:

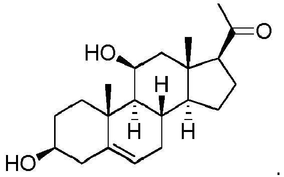

The term "11β-hydroxyprogesterone" as used herein refers to a compound having the structural formula:

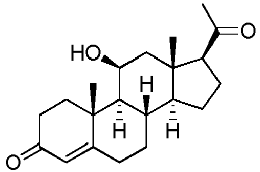

The term "derivative" as used herein to modify the term "11β-hydroxypregnenolone" or "11β-hydroxysreoid" the term "11β-hydroxyprogesterone" refers to any compound which retains the ring structure and stereochemistry of 11β-hydroxypregnenolone or 11β-hydroxyprogesterone, "11β-hydroxysreoid" itself, but which contains one or more substituent groups relative to 11β-hydroxypregnenolone or 11β-hydroxyprogesterone. The term also includes combination molecules or prodrugs that release 11β-hydroxypregnenolone when administered to a subject. Such a combination molecule may include, for example, 11β-hydroxypregnenolone and an agent joined by a hydrolysable linker group.

The term **"HCAEC"** as used herein refer to Human corniary Artery endothelial cells; the term **"BCAEC"** refers to bovine corniary Artery endothelial cells; and the term **"GAPDH"** refers to glyceraldehyde-3-phosphate dehydrogenase.

The term "prodrug" refers to a compound that is made more active in vivo. Certain compounds disclosed herein may also exist as prodrugs, as described in Hydrolysis in Drug and Prodrug Metabolism: Chemistry, Biochemistry, and Enzymology (Testa, Bernard and Mayer, Joachim M. Wiley-VHCA, Zurich, Switzerland 2003). Prodrugs of the compounds described herein are structurally modified forms of the compound that readily undergo chemical changes under physiological conditions to provide the compound. Additionally, prodrugs can be converted to the compound by chemical or biochemical methods in an ex vivo environment. For example, prodrugs can be slowly converted to a compound when placed in a transdermal patch reservoir with a suitable enzyme or chemical reagent. Prodrugs are often useful because, in some situations, they may be easier to administer than the compound, or parent drug. They may, for instance, be bioavailable by oral administration whereas the parent drug is not. The prodrug may also have improved solubility in pharmaceutical compositions over the parent drug. A wide variety of prodrug derivatives are known in the art, such as those that rely on hydrolytic cleavage or oxidative activation of the prodrug. An example, without limitation, of a prodrug would be a compound which is administered as an ester (the "prodrug"), but then is metabolically hydrolyzed to the carboxylic acid, the active entity. Additional examples include peptidyl derivatives of a compound.

The compounds disclosed herein can exist as therapeutically acceptable salts. The present invention includes compounds listed above in the form of salts, including acid addition salts. Suitable salts include those formed with both organic and inorganic acids. Such acid addition salts will normally be pharmaceutically acceptable. However, salts of non-pharmaceutically acceptable salts may be of utility in the preparation and purification of the compound in question. Basic addition salts may also be formed and be pharmaceutically acceptable. For a more complete discussion of the preparation and selection of salts, refer to Pharmaceutical Salts: Properties, Selection, and Use (Stahl, P. Heinrich. Wiley-VCHA, Zurich, Switzerland, 2002).

The term "therapeutically acceptable salt," as used herein, represents salts or zwitterionic forms of the compounds disclosed herein which are water or oil-soluble or dispersible and therapeutically acceptable as defined herein. The salts can be prepared during the final isolation and purification of the compounds or separately by reacting the appropriate compound in the form of the free base with a suitable acid. Representative acid addition salts include acetate, adipate, alginate, L-ascorbate, aspartate, benzoate, benzenesulfonate (besylate), bisulfate, butyrate, camphorate, camphorsulfonate, citrate, digluconate, formate, fumarate, gentisate, glutarate, glycerophosphate, glycolate, hemisulfate, heptanoate, hexanoate, hippurate, hydrochloride, hydrobromide, hydroiodide, 2-hydroxyethansulfonate (isethionate), lactate, maleate, malonate, DL-mandelate, mesitylenesulfonate, methanesulfonate, naphthylenesulfonate, nicotinate, 2-naphthalenesulfonate, oxalate, pamoate, pectinate, persulfate, 3-phenylproprionate, phosphonate, picrate, pivalate, propionate, pyroglutamate, succinate, sulfonate, tartrate, L-tartrate, trichloroacetate, trifluoroacetate, phosphate, glutamate, bicarbonate, para-toluenesulfonate (p-tosylate), and undecanoate. Also, basic groups in the compounds disclosed herein can be quaternized with methyl, ethyl, propyl, and butyl chlorides, bromides, and iodides; dimethyl, diethyl, dibutyl, and diamyl sulfates; decyl, lauryl, myristyl, and steryl chlorides, bromides, and iodides; and benzyl and phenethyl bromides. Examples of acids which can be employed to form therapeutically acceptable addition salts include inorganic acids such as hydrochloric, hydrobromic, sulfuric, and phosphoric, and organic acids such as oxalic, maleic, succinic, and citric. Salts can also be formed by coordination of the compounds with an alkali metal or alkaline earth ion. Hence, the present invention contemplates sodium, potassium, magnesium, and calcium salts of the compounds disclosed herein, and the like.

Basic addition salts can be prepared during the final isolation and purification of the compounds by reacting a carboxy group with a suitable base such as the hydroxide, carbonate, or bicarbonate of a metal cation or with ammonia or an organic primary, secondary, or tertiary amine. The cations of therapeutically acceptable salts include lithium, sodium, potassium, calcium, magnesium, and aluminum, as well as nontoxic quaternary amine cations such as ammonium, tetramethylammonium, tetraethylammonium, methylamine, dimethylamine, trimethylamine, triethylamine, diethylamine, ethylamine, tributylamine, pyridine, *N,N*-dimethylaniline, *N-*methylpiperidine, *N*-methylmorpholine, dicyclohexylamine, procaine, dibenzylamine, *N,N-*dibenzylphenethylamine, 1-ephenamine, and *N,N'*-dibenzylethylenediamine. Other representative organic amines useful for the formation of base addition salts include ethylenediamine, ethanolamine, diethanolamine, piperidine, and piperazine.

A salt of a compound can be made by reacting the appropriate compound in the form of the free base with the appropriate acid.

While it may be possible for the compounds of the subject invention to be administered as the raw chemical, it is also possible to present them as a pharmaceutical formulation. Accordingly, provided herein are pharmaceutical formulations which comprise one or more of certain compounds disclosed herein, or one or more pharmaceutically acceptable salts, esters, prodrugs, amides, or solvates thereof, together with one or more pharmaceutically acceptable carriers thereof and optionally one or more other therapeutic ingredients. The carrier(s) must be "acceptable" in the sense of being compatible with the other ingredients of the formulation and not deleterious to the recipient thereof. Proper formulation is dependent upon the route of administration chosen. Any of the well-known techniques, carriers, and excipients may be used as suitable and as understood in the art; *e.g.,* in Remington's Pharmaceutical Sciences. The pharmaceutical compositions disclosed herein may be manufactured in any manner known in the art, *e.g.,* by means of conventional mixing, dissolving, granulating, dragee-making, levigating, emulsifying, encapsulating, entrapping or compression processes.

The formulations include those suitable for oral, parenteral (including subcutaneous, intradermal, intramuscular, intravenous, intraarticular, and intramedullary), intraperitoneal, transmucosal, transdermal, rectal and topical (including dermal, buccal, sublingual and intraocular) administration although the most suitable route may depend upon for example the condition and disorder of the recipient. The formulations may conveniently be presented in unit dosage form and may be prepared by any of the methods well known in the art of pharmacy. Typically, these methods include the step of bringing into association a compound of the subject invention or a pharmaceutically acceptable salt, ester, amide, prodrug or solvate thereof ("active ingredient") with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the compounds disclosed herein suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

Pharmaceutical preparations which can be used orally include tablets, push-fit capsules made of gelatin, as well as soft, sealed capsules made of gelatin and a plasticizer, such as glycerol or sorbitol. Tablets may be made by compression or molding, optionally with one or more accessory ingredients. Compressed tablets may be prepared by compressing in a suitable machine the active ingredient in a free-flowing form such as a powder or granules, optionally mixed with binders, inert diluents, or lubricating, surface active or dispersing agents. Molded tablets may be made by molding in a suitable machine a mixture of the powdered compound moistened with an inert liquid diluent. The tablets may optionally be coated or scored and may be formulated so as to provide slow or controlled release of the active ingredient therein. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Specific sustained release formulations of the compounds disclosed herein are described in U.S. Patent 6,410,052, which is hereby incorporated by reference.

The compounds may be formulated for parenteral administration by injection, *e.g*., by bolus injection or continuous infusion. Formulations for injection may be presented in unit dosage form, *e.g*., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. The formulations may be presented in unit-dose or multi-dose containers, for example sealed ampoules and vials, and may be stored in powder form or in a freeze-dried (lyophilized) condition requiring only the addition of the sterile liquid carrier, for example, saline or sterile pyrogen-free water, immediately prior to use. Extemporaneous injection solutions and suspensions may be prepared from sterile powders, granules and tablets of the kind previously described.

Formulations for parenteral administration include aqueous and non-aqueous (oily) sterile injection solutions of the active compounds which may contain antioxidants, buffers, bacteriostats and solutes which render the formulation isotonic with the blood of the intended recipient; and aqueous and non-aqueous sterile suspensions which may include suspending agents and thickening agents. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

For buccal or sublingual administration, the compositions may take the form of tablets, lozenges, pastilles, or gels formulated in conventional manner. Such compositions may comprise the active ingredient in a flavored basis such as sucrose and acacia or tragacanth.

The compounds may also be formulated in rectal compositions such as suppositories or retention enemas, e.g., containing conventional suppository bases such as cocoa butter, polyethylene glycol, or other glycerides.

Certain compounds disclosed herein may be administered topically, that is by non-systemic administration. This includes the application of a compound disclosed herein externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration. In an embodiment, the compounds of the present invention may be used in a dosage in the range of 0.01 to 100mg/Kg.

Formulations suitable for topical administration include liquid or semi-liquid preparations suitable for penetration through the skin to the site of inflammation such as gels, liniments, lotions, creams, ointments or pastes, and drops suitable for administration to the eye, ear or nose. The active ingredient for topical administration may comprise, for example, from 0.001% to 10% w/w (by weight) of the formulation. In certain embodiments, the active ingredient may comprise as much as 10% w/w. In other embodiments, it may comprise less than 5% w/w. In certain embodiments, the active ingredient may comprise from 2% w/w to 5% w/w. In other embodiments, it may comprise from 0.1% to 1 % w/w of the formulation.

For administration by inhalation, compounds may be conveniently delivered from an insufflator, nebulizer pressurized packs or other convenient means of delivering an aerosol spray. Pressurized packs may comprise a suitable propellant such as dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. Alternatively, for administration by inhalation or insufflation, the compounds according to the invention may take the form of a dry powder composition, for example a powder mix of the compound and a suitable powder base such as lactose or starch. The powder composition may be presented in unit dosage form, in for example, capsules, cartridges, gelatin or blister packs from which the powder may be administered with the aid of an inhalator or insufflator.

The unit dosage formulations are those containing an effective dose, as herein below recited, or an appropriate fraction thereof, of the active ingredient.

It should be understood that in addition to the ingredients particularly mentioned above, the formulations described above may include other agents conventional in the art having regard to the type of formulation in question, for example those suitable for oral administration may include flavoring agents.

Compounds may be administered orally or via injection at a dose of from 0.1 to 500 mg/kg per day. The dose range for adult humans is generally from 5 mg to 2 g/day. Tablets or other forms of presentation provided in discrete units may conveniently contain an amount of one or more compounds which is effective at such dosage or as a multiple of the same, for instance, units containing 5 mg to 500 mg, usually around 10 mg to 200 mg.

The amount of active ingredient that may be combined with the carrier materials to produce a single dosage form will vary depending upon the host treated and the particular mode of administration.

The compounds can be administered in various modes, e.g. orally, topically, or by injection. The precise amount of compound administered to a patient will be the responsibility of the attendant physician. The specific dose level for any particular patient will depend upon a variety of factors including the activity of the specific compound employed, the age, body weight, general health, sex, diets, time of administration, route of administration, rate of excretion, drug combination, the precise disorder being treated, and the severity of the indication or condition being treated. Also, the route of administration may vary depending on the condition and its severity.

In certain instances, it may be appropriate to administer at least one of the compounds described herein (or a pharmaceutically acceptable salt, ester, or prodrug thereof) in combination with another therapeutic agent. By way of example only, if one of the side effects experienced by a patient upon receiving one of the compounds herein is hypertension, then it may be appropriate to administer an anti-hypertensive agent in combination with the initial therapeutic agent. Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for diabetes involving administration of one of the compounds described herein, increased therapeutic benefit may result by also providing the patient with another therapeutic agent for diabetes. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of certain compounds of the invention with agents which allow or enhance improvements in the number, structure or function of skeletal muscle cells or cardiac muscle cells.

Or, by way of example only, the therapeutic effectiveness of one of the compounds described herein may be enhanced by administration of an adjuvant (i.e., by itself the adjuvant may only have minimal therapeutic benefit, but in combination with another therapeutic agent, the overall therapeutic benefit to the patient is enhanced). Or, by way of example only, the benefit of experienced by a patient may be increased by administering one of the compounds described herein with another therapeutic agent (which also includes a therapeutic regimen) that also has therapeutic benefit. By way of example only, in a treatment for diabetes involving administration of one of the compounds described herein, increased therapeutic benefit may result by also providing the patient with another therapeutic agent for diabetes. In any case, regardless of the disease, disorder or condition being treated, the overall benefit experienced by the patient may simply be additive of the two therapeutic agents or the patient may experience a synergistic benefit.

Specific, non-limiting examples of possible combination therapies include use of compounds or compositions disclosed herein in combination with agents which allow or enhance improvements in the number, structure, or function of skeletal muscle cells or cardiac muscle cells, cofactors that enhance mitochondrial biogenesis, and factors that enhance the production of NO in response to the stimulation of eNOS or nNOS.

In further embodiments, such agents include hormones which stimulate muscle cell growth, γ-amino butyric acid or its derivatives, dietary protein supplements, anabolic steroids, biological factors known to enhance the growth, strength, endurance, or metabolism of skeletal or cardiac muscle, or recovery of skeletal muscle or cardiac muscle from injury or weakness, compounds known to be associated with increased nitric oxide production which promotes blood flow through muscles, extracts of natural products known to promote muscle strength or endurance, inhibitors of myostatin, and stimulators of folistatin expression.

In further embodiments, hormones which stimulate muscle cell growth include, but are not limited to, growth hormone, growth hormone analogs, growth hormone releasing peptides or analogs thereof, growth hormone secretagogues, growth hormone precursors, or other hormones such as somatatropin or mechano growth factor.

In further embodiments, γ-amino butyric acid derivatives include, but are not limited to, 4-amino-3-phenylbutyric acid and neurotransmitters that benefit muscles by modulating the pituitary gland.

In further embodiments, dietary protein supplements include, but are not limited to, casein, whey, soy, egg white, hemp, rice, and pea proteins, amino acids precursors or derivatives thereof with known attributes of potentiating muscle growth, such as leucine, valine, isovaline, beta alanine, glutamine, glutamine dipeptide, or glycocyamine.

In further embodiments anabolic steroids, include, but are not limited to, testosterone or related steroid compounds with muscle growth inducing properties, such as cyclostanazol or methadrostenol, prohomones or derivatives thereof, modulators of estrogen, and selective androgen receptor modulators (SARMS).

In further embodiments, biological factors known to enhance the growth, strength, endurance, or metabolism of skeletal or cardiac muscle, or recovery of skeletal muscle or cardiac muscle from injury or weakness, include, but are not limited to, alpha-lipoic acid, taurine, caffeine, magnesium, niacin, folic acid, ornithine, vitamin B6, B12, or D, aspartate, creatine and its diverse salts such creatine monohydrate, betaine, N-acetyl cysteine, beta-hydroxyl methyl butyrate, lecithin, choline, phospholipid mixtures, phosphatidyl serine, carnitine, L-carnitine, acetyl-L-carnitine, and glycine proprionyl-L-carnitine.

In an embodiment, the compounds of the present invention are used for the activation AMPK.

In an embodiment, the compounds of the present invention are used for the subsequent activation transcription factors such as PCG-1 alpha associated with mitochondrial biogenisis.

In further embodiments, the compounds of the present invention are used for the activation mitochondrial biogenisis and functions.

In further embodiments, the compounds of the present invention are used for treatment of diseases associated with mitochondrial depletion and/ or dysfunctionselected, but not limited to, the group consisting of skeletal muscle diseases, cardiac muscle diseases associated with ischemia, or impaired or inadequate blood flow, diseases associated with genetic disorders that directly or indirectly affect the number, structure, or function of mitochondria, diseases associated with impaired neurological function associated with decreased mitochondrial number or function, diseases associated with loss of number, loss of function, or loss of correct, optimally efficient internal organization of skeletal muscle cells or cardiac muscle cells, metabolic diseases, and conditions associated with liver cell injury and altered fatty acid metabolism.

In further embodiments, the compounds of the present invention are used for mitochondrial biogenesis-mediated disease is selected from the group consisting of acute coronary syndrome, myocardial infarction, angina, renal injury, renal ischemia, diseases of the aorta and its branches, injuries arising from medical interventions, atherosclerosis, trauma, diabetes, hyperlipidemia, vascular stenosis, peripheral arterial disease, vasculopathy, and vasculitis, Friedreich's ataxia, muscular dystrophy, Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, spinal muscular atrophy, Emery-Dreifuss muscular dystrophy, Huntington's disease, Parkinson's disease, Alzheimer's disease, and amyotrophic lateral sclerosis, sarcopenia, congestive heart failure, aging, myocarditis, myositis, polymyalgia rheumatic, polymyositis, HIV, cancer and/or the side effects of chemotherapy targeting the cancer, malnutrition, aging, inborn errors of metabolism, trauma, and stroke or other types of neurological impairment, hepatic steatosis, hepatic fibrosis, cirrhosis, and hepatocyte or stellate cell injury.

In further embodiments, compounds known to be associated with increased nitric oxide production which promotes blood flow through muscles include, but are not limited to, arginine and citrulline.

In further embodiments, compounds known to promote or facilitate mitochochondrial function or biogenesis, include, but are not limited to, alpha lipoic acid, resveratrol, coenzyme Q10 and its derivatives, forskalin, metformin, acetyl-carnitine, alpha tocopherol, pyruvate, choline, B vitamins, niacin, and biotin.

In further embodiments, extracts of natural products known to promote muscle strength or endurance, include, but are not limited to, guarana, geranium robertianum, cirsium ologophyllum, bauhinia purpureae, yohimbe, bacopa monniera, beet powder, rhodiola, or tea extracts.

In further embodiments, inhibitors of myostatin are proteins, antibodies, peptides, or small molecules.

In further embodiments, stimulators of follistatin expression or function are proteins, peptides, or small molecules.

In further embodiments, compounds disclosed herein may be administered in combination with another agent or agents such as niacin, or inhibitors of xanthine oxidase, such as allopurinol.

In further embodiments, compounds disclosed herein may be administered orally or parenterally.

In any case, the multiple therapeutic agents (at least one of which is a compound disclosed herein) may be administered in any order or even simultaneously. If simultaneously, the multiple therapeutic agents may be provided in a single, unified form, or in multiple forms (by way of example only, either as a single pill or as two separate pills). One of the therapeutic agents may be given in multiple doses, or both may be given as multiple doses. If not simultaneous, the timing between the multiple doses may be any duration of time ranging from a few minutes to four weeks.

Thus, in another aspect, certain embodiments provide methods for treating muscular diseases in a human or animal subject in need of such treatment comprising administering to said subject an amount of a compound disclosed herein effective to reduce or prevent said disorder in the subject, in combination with at least one additional agent for the treatment of said disorder that is known in the art. In a related aspect, certain embodiments provide therapeutic compositions comprising at least one compound disclosed herein in combination with one or more additional agents for the treatment of muscular diseases.

The compositions of the present invention may also be formulated as nutraceutical compositions. The term "nutraceutical composition" as used herein refers to a food product, foodstuff, dietary supplement, nutritional supplement or a supplement composition for a food product or a foodstuff comprising exogenously added compounds as disclosed herein. Details on techniques for formulation and administration of such compositions may be found in Remington, The Science and Practice of Pharmacy 21st Edition (Mack Publishing Co., Easton, PA) and Nielloud and Marti-Mestres, Pharmaceutical Emulsions and Suspensions: 2nd Edition (Marcel Dekker, Inc, New York).

As used herein, the term "food product" refers to any food or feed suitable for consumption by humans or animals. The food product may be a prepared and packaged food (e.g., mayonnaise, salad dressing, bread, grain bar, beverage, etc.) or an animal feed (e.g., extruded and pelleted animal feed, coarse mixed feed or pet food composition). As used herein, the term foodstuff refers to any substance fit for human or animal consumption.

Food products or foodstuffs are for example beverages such as nonalcoholic and alcoholic drinks as well as liquid preparation to be added to drinking water and liquid food, non-alcoholic drinks are for instance soft drinks, sport drinks, fruit juices, such as orange juice, apple juice, and grapefruit juice, lemonades, teas, near-water drinks, milk and other dairy drinks such as for example yogurt drinks, and diet drinks. In another embodiment food products or foodstuffs refer to solid or semi-solid foods comprising a compound according to the invention. These forms can include, but are not limited to baked goods such as cakes and cookies, puddings, dairy products, confections, snack foods, or frozen confections or novelties (e.g., ice cream, milk shakes), prepared frozen meals, candy, snack products (e.g., chips), liquid food such as soups, spreads, sauces, salad dressings, prepared meat products, cheese, yogurt and any other fat or oil containing foods, and food ingredients (e.g., wheat flour).

Animal feed including pet food compositions advantageously include food intended to supply necessary dietary requirements, as well as treats (e.g., dog biscuits) or other food supplements. The animal feed comprising the composition according to the invention may be in the form of a dry composition (for example, kibble), semi-moist composition, wet composition, or any mixture thereof. Alternatively or additionally, the animal feed is a supplement, such as a gravy, drinking water, yogurt, powder, suspension, chew, treat (e.g., biscuits) or any other delivery form.

The term "dietary supplement" refers to a small amount of a compound for supplementation of a human or animal diet packaged in single or multiple dose units.

Dietary supplements do not generally provide significant amounts of calories but may contain other micronutrients (e.g., vitamins or minerals). The term food products or foodstuffs also includes functional foods and prepared food products pre-packaged for human consumption.

The term nutritional supplement refers to a composition comprising a dietary supplement in combination with a source of calories. In some embodiments, nutritional supplements are meal replacements or supplements (e.g., nutrient or energy bars or nutrient beverages or concentrates).

Dietary supplements of the present invention may be delivered in any suitable format. In certain embodiments, dietary supplements are formulated for oral delivery. The ingredients of the dietary supplement of this invention are contained in acceptable excipients and/or carriers for oral consumption. The actual form of the carrier, and thus, the dietary supplement itself, is not critical. The carrier may be a liquid, gel, gelcap, capsule, powder, solid tablet (coated or noncoated), tea, or the like.

Compounds and compositions disclosed herein are administered in an "effective amount." This term is defined hereinafter. Unless dictated otherwise, explicitly or otherwise, an "effective amount" is not limited to a minimal amount sufficient to ameliorate a condition, or to an amount that results in an optimal or a maximal amelioration of the condition. In the case when two or more compounds are administered together, an effective amount of one such compound may not be, in and of itself, be an effective amount, but may be an effective amount when used together with additional compounds.

In certain embodiments, the effective amount is an amount which stimulates mitochondrial function in cells. Such stimulation of mitochondrial function in cells may comprise stimulation of one or more of mitochondrial respiration and mitochondrial biogenesis. The methods and compositions described herein can assist in prevention of the consequences of mitochondrial toxicity which has not yet occurred, as well as provide for the active therapy of mitochondrial toxicity that may have already occurred.

While the phrase "administered together" as used herein may refer to the provision of chemical compositions in the same pharmaceutical composition, the phrase as used herein is not intended to imply that this must be so. Rather, two or more chemical compositions are "administered together" if the T_{1/2} for the clearances of each composition from the body overlaps at least partially with one another. For example, if a first pharmaceutical has a T_{1/2} for clearance of 1 hour and is administered at time=0, and a second pharmaceutical has a T_{1/2} for clearance of 1 hour and is administered at time=45 minutes, such pharmaceuticals are considered administered together. Conversely, if the second drug is administered at time=2 hours, such pharmaceuticals are not considered administered together.

Routes of administration for the pharmaceutical compositions of the present invention include parenteral and enteral routes. Enteral routes of administration include delivery by mouth (oral), nasal, rectal, and vaginal routes. Parenteral routes of administration include intravenous, intramuscular, subcutaneous, and intraperitoneal routes. When more than one pharmaceutical composition is being administered, each need not be administered by the same route. In particularly embodiments, 11β-hydroxypregnenolone, or a derivative or pharmaceutically acceptable salt thereof, is administered together intravenously with one or more tetracycline antibiotics such as doxycycline, most preferably in a single pharmaceutical composition.

In certain embodiments, the methods disclosed herein comprise the administration to cells at least 10pM, at least 1.0 nM, or at least 100nM of a compounds disclosed herein.

In further embodiments, the methods disclosed herein comprise the administration of compounds of the disclosure in a total daily dose of about 0.001 mg/kg/dose to about 10 mg/kg/dose, alternately from about 0.3 mg/kg/dose to about 3 mg/kg/dose. In another embodiment the dose range is from about 0.1 to about 1 mg/kg/day. Generally between about 0.01 mg and about 0.1 gram per day can be administered; alternately between about 2.5 mg and about 200 mg can be administered. The dose may be administered in as many divided doses as is convenient.

In further embodiments, the methods disclosed herein comprise the administration of compounds disclosed herein in a range of about 0.1 to about 100 mg per kg body weight.

In further embodiments, the desired concentration is maintained for at least 30 minutes, 1 hour, 3 hours, 6 hours, 12 hours, 24 hours, 48 hours, 72 hours, or more. In yet further embodiments, the desired concentration is achieved at least once during each 12-hour period over at least 24 hours, 48 hours, 72 hours, 1 week, one month, or more; or at least once during each 24-hour period over at least 48 hours, 72 hours, 1 week, one month, or more. In order to maintain a desired concentration for a desired time, multiple doses of one or more compounds may be employed. The dosing interval may be determined based on the clearance half-life for each compound of interest from the body.

In certain embodiments, disclosed herein are methods and compositions for the treatment of diseases associated with loss of number, function, or correct, optimally efficient internal organization of mitochondria within cells.

In further embodiments, disclosed herein are methods and compositions for the treatment of skeletal or cardiac muscle diseases associated with ischemia, or impaired or inadequate blood flow. Examples of such states include, but are not limited to, atherosclerosis, trauma, diabetes, vascular stenosis, peripheral arterial disease, vasculopathy, and vasculitis.

In further embodiments, disclosed herein are methods and compositions for the treatment of diseases associated with genetic disorders that directly or indirectly affect the number, structure, or function of mitochondria, particularly those associated with muscle dysfunction or myopathy. Examples of such states include, but are not limited to, the set of diseases broadly classified as muscular dystrophies and Friedreich's ataxia. In further embodiments, said muscular dystrophy is selected from the group consisting of Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy.

In further embodiments, disclosed herein are methods and compositions for the therapeutic treatment of diseases associated with impaired neurological function associated with decreased mitochondrial number or function. Examples include, but are not limited to, Huntington's disease, Parkinson's disease, Alzheimer's disease, and amyotrophic lateral sclerosis (ALS).

In certain embodiments, disclosed herein are methods and compositions for the treatment of diseases associated with loss of number, loss of function, or loss of correct, optimally efficient internal organization of skeletal muscle cells or cardiac muscle cells. Such diseases may eventuate in a state of functionally significant muscle wasting, which, in its most pronounced form, is termed sarcopenia. Sarcopenia may be secondary to a variety of disorders, including aging, muscular dystrophy, diabetes, or other abnormal metabolic conditions, infection, inflammation, autoimmune disease, cardiac dysfunction, or severe disuse syndromes, or inactivity associated with arthritis. Examples of such diseases include, but are not limited to, congestive heart failure, aging, myocarditis, myositis, polymyalgia rheumatic, polymyositis, HIV, cancer and/or the side effects of chemotherapy targeting the cancer, malnutrition, aging, inborn errors of metabolism, trauma, and stroke or other types of neurological impairment.

In certain embodiments, disclosed herein are methods and compositions for use to enhance sports performance and endurance, to build muscle shape and strength, and to facilitate recovery from the muscle related side effects of training or competition, such as soreness, weakness, cramping, pain, or injury.

In certain embodiments, a subject is selected for treatment with a compound or composition disclosed herein based on the occurrence of one or more physiological manifestations of skeletal or cardiac muscle injury or dysfunction in the subject. Such manifestations include elevations in biomarkers known to be related to injury of the heart or skeletal muscle. Examples of such biomarkers include, but are not limited to, elevated plasma levels of cardiac or skeletal muscle enzymes or proteins, such as myoglobin, troponin, or creatine phosphokinase, lactic acidosis, and elevated serum creatinine.

In certain embodiments, a compound or composition as disclosed herein is administered in an amount which stimulates increased number or function of skeletal muscle cells or contractile muscle cells. Such stimulation of muscle cells may comprise stimulation of one or more aspects of muscle cell function, including cell division, muscle cell regeneration, activation of muscle satellite cells and their differentiation into adult muscle cells, recovery from injury, increased number or function of mitochondria or processes serving mitochondrial function, increased expression of proteins contributing to contractility, regulation of biochemical or translational processes, mitoses, or transduction of mechanical energy via dystrophin or other attachment processes. The methods and compositions described herein can assist in prevention of the consequences of muscle injury or dysfunction which have not yet occurred, as well as provide for the active therapy of muscle injury, dysfunction, or diseases which have already occurred.

In yet another aspect, the invention is directed to methods of treating metabolic disease in a subject. These methods comprise administering to a subject in need thereof a compound disclosed herein. In certain embodiments, the subject is selected based on the occurrence of diabetes or hyperlipidemia. In further embodiments the method reduces blood glucose levels and/or lowers blood triglycerides in the subject.

In yet another aspect, the invention is directed to methods of preventing or reversing injury to hepatic mitochondria by agents such as fructose, and thus preventing or reversing hepatic steatosis and other conditions associated with liver cell injury and altered fatty acid metabolism, and thus preventing or reversing hepatic fibrosis, or cirrhosis, associated with sustained hepatocyte or stellate cell injury.

In another embodiment, compounds or compositions disclosed herein may be administered in combination with another agent or agents such as niacin, or inhibitors of xanthine oxidase, such as allopurinol, to treat hyperlipidemia or treat liver injury associated with fructose or other agents associated with intracellular fat accumulation, steatosis, fibrosis, or cirrhosis.

In another embodiment, a decrease in the plasma or tissue levels of 11β-hydroxypregnenolone, 11β-hydroxyprogesterone, or metabolites thereof, such as sulfated, glucuronidated, or methylated derivatives, may be employed as a diagnostic test to determine deficiency states of 11β-hydroxypregnenolone or 11β-hydroxyprogesterone.

In another embodiment, an increase in the plasma or tissue levels of 11β-hydroxypregnenolone, 11β-hydroxyprogesterone, or metabolites thereof, such as sulfated, glucoronidated, or methylated derivatives, may be employed as a diagnostic test to determine therapeutic response to administration of 11β-hydroxypregnenolone, 11β-hydroxyprogesterone, or derivatives thereof.

Changes in the plasma or tissue levels of 11β-hydroxypregnenoione or 11β-hydroxyprogesterone or related hydroxysteroids and metabolites thereof may also be employed in conjunction with biomarkers of muscle injury or regeneration, such as myostatin, follistatin, creatine kinase, and others in order to determine deficiency states of the hydroxysteroid pathway or measure therapeutic response to hydroxysteroid-based therapeutics.

In another embodiment, compounds or compositions disclosed herein may be employed as therapeutics, or hormone replacement, in diseases or conditions associated with deficiency states of 11β-hydroxypregnenolone, 11β-hydroxyprogesterone, or metabolites thereof.

In a first aspect, the present invention provides methods for preventing or treating adverse events associated with the use of chemical compositions such as approved medications in which the adverse event is caused by, or associated with, perturbations in mitochondrial number, function, or structure. The methods comprise administering to a subject in need thereof a compound or composition disclosed herein. In certain embodiments the method reduces symptoms of mitochondrial toxicity due to the subject's exposure to chemical compositions that exhibit mitochondrial toxicity.

In certain embodiments, compounds or compositions disclosed herein are administered in combination with one or more chemical compositions which exhibit mitochondrial toxicity. Such chemical compositions include, but are not limited to, those described above in regard to drug-induced mitochondrial dysfunction of the heart, liver, and kidneys.

In certain embodiments, the chemical composition that exhibits mitochondrial toxicity is identified based on the demonstration of one or more biological effects indicative of mitochondrial toxicity by the chemical composition. Such effects include, but are not limited to, abnormal mitochondrial respiration, abnormal oxygen consumption, abnormal extracellular acidification rate, abnormal mitochondrial number, abnormal lactate accumulation, abnormal ATP levels, etc.

In other embodiments, compounds or compositions disclosed herein are administered based on the occurrence of one or more physiological manifestations of mitochondrial toxicity in the subject. Such manifestations include, but are not limited to, elevations in markers known to relate to injury to the heart, liver, and/or kidney. Non-limiting examples include elevated serum liver enzymes, elevated cardiac enzymes, lactic acidosis, elevated blood glucose, elevated serum creatinine, etc.

In certain embodiments, compounds or compositions disclosed herein are administered in combination with one or more chemical compositions which can increase the biological activity of compounds disclosed herein, particularly with respect to effecting mitochondrial biogenesis, promoting muscle regeneration, and enhancing NO availability via the stimulation of the expression and activity of eNOS and nNOS.

In another embodiment, the present invention provides methods for improving muscle structure or function; methods for improving mitochondrial effects associated with exercise; methods for enhancing the capacity for exercise in those limited by age, inactivity, diet, or any of the aforementioned diseases and conditions; methods for enhancing muscle health and function in response to exercise; methods for enhancing muscle health and function in the clinical setting of restricted capacity for exercise, whether due to injury, inactivity, obesity, or any of the aforementioned diseases and conditions; and/or methods to enhance recovery of muscles from vigorous activity or from injury associated with vigorous or sustained activity.

In related aspects, the present invention includes methods of treating a condition involving decreased mitochondrial function in an animal. These methods comprise delivering to the animal one or more compounds or compositions disclosed herein.

It is an object of the invention to provide compositions and methods for prophylactic and/or therapeutic treatment of diseases and conditions related to apoptosis and cellular necrosis caused by ischemia. In various aspects described hereinafter, the present invention provides compositions and methods for treatment of acute coronary syndromes, including but not limited to myocardial infarction and angina; acute ischemic events in other organs and tissues, including but not limited to renal injury, renal ischemia and diseases of the aorta and its branches; injuries arising from medical interventions, including but not limited to coronary artery bypass grafting (CABG) procedures and aneurysm repair; and metabolic diseases, including but not limited to diabetes mellitus.

It is another object of the invention to provide compositions and methods for prophylactic and/or therapeutic treatment of conditions related to mitochondrial function. In various aspects described hereinafter, the present invention comprises administering one or more compounds as disclosed herein. Stimulation of mitochondrial function in cells may comprise stimulation of one or more of mitochondrial respiration and mitochondrial biogenesis. The methods and compositions described herein can assist in prevention of impaired mitochondria biogenesis and thus prevention of the consequences of impaired mitochondrial biogenesis in various diseases and conditions, as well as provide for the active therapy of mitochondrial depletion that may have already occurred.

In certain embodiments of the present invention, compounds or compositions disclosed herein are administered to the subject together with one or more additional drugs useful in the treatment of ischemic or ischemia/reperfusion events. Exemplary additional drugs include one or more compounds independently selected from the group consisting of tetracycline antibiotics (e.g., doxycycline), glycoprotein IIb/IIIa inhibitors (e.g., eptifibatide, tirofiban, abciximab); ADP receptor/P2Y12 inhibitors (e.g., clopidogrel, ticlopidine, prasgurel); prostaglandin analogues (e.g., betaprost, iloprost, treprostinil); COX inhibitors (e.g., asprin, aloxiprin); other antiplatelet drugs (e.g., ditazole, cloricromen, dipyridamole, indobufen, picotamide, triflusal); anticoagulants (e.g., coumarins, 1,3-indandiones); heparins; direct factor Xa inhibitors; direct thrombin (II) inhibitors (e.g., bivalirudin); and vasodilators (e.g., fendoldopam, hydralazine, nesiritide, nicorandil, nicardipine, nitroglycerine, nitroprusside). This list is not meant to be limiting. In a particularly embodiment, a compound or composition disclosed herein is administered together with one or more tetracycline antibiotics such as doxycycline.

In the case of an ischemic event involving the heart, objective measures include increases in one or more cardiac markers (e.g., CK-MB, myoglobin, cardiac troponin I, cardiac troponin T, B-type Natriuretic peptide, NT-proBNP, etc.); changes in serial ECG tracings; and angiographic results.

In the case of an ischemic event involving the kidneys, objective measures include those defined by Bellomo et al., Crit Care. 8(4):R204-12, 2004, which is hereby incorporated by reference in its entirety. This reference proposes the following classifications for stratifying acute kidney injury patients: "Risk": serum creatinine increased 1.5 fold from baseline OR urine production of <0.5 ml/kg body weight for 6 hours; "Injury": serum creatinine increased 2.0 fold from baseline OR urine production <0.5 ml/kg for 12 h; "Failure": serum creatinine increased 3.0 fold from baseline OR creatinine >355 µmol/L (with a rise of >44) or urine output below 0.3 ml/kg for 24 h.

In a related aspect, the present invention is directed to pharmaceutical compositions for treatment of an acute ischemic or ischemia /reperfusion (IR) event. This composition comprises an effective amount of a compound or composition disclosed herein, and one or more additional drugs useful in the treatment of ischemic or ischemia/reperfusion events. In particularly embodiments, the pharmaceutical composition comprises a compound or composition disclosed herein and one or more tetracycline antibiotics, such as doxycycline. In a further embodiment, the composition is formulated for intravenous delivery.

The compounds of the present invention may be illustrated but not limited to the examples as provide in Table 1.

**Table 1: Illustrative compounds of the provisional application**

| **No.** | **IUPAC name** | **Structure** |
|---|---|---|
| 1001. | (3S,8S,9S,10R,11S,13S,14S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3, 11-diol | 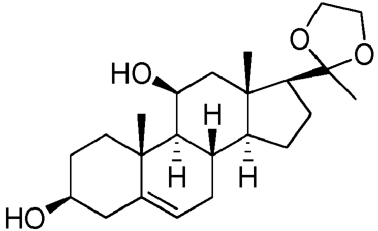 |
| 1002. | 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethanone | 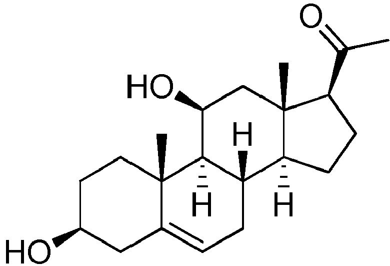 |
| 1003. | (3S,8S,9S,10R,11S,13S,14S,17S)-17-(1-hydroxyethyl)-10,13-dimethyl-2,3,4,7,8,9,10,11,12,13,14,15,16,17-tetradecahydro-1H-cyclopenta[a]phenanthrene-3,11 -diol | 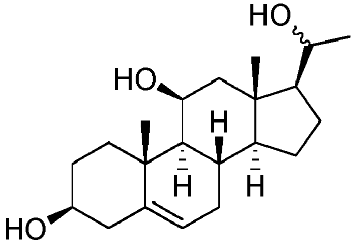 |
| 1004. | (3S,8S,9S,10R,11S,13S,14S,17S)-methyl 3,11-dihydroxy-10,13-dimethyl-2,3,4,7,8,9,10,11,12,13,14,15,16,17-tetradecahydro-1H-cyclopenta[a]phenanthrene-17-carboxylate | 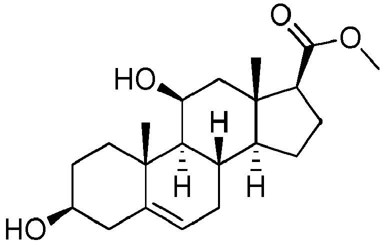 |
| 1005. | (8S,9S,10R,11S,13S,14S,17S)-methyl 11-hydroxy-10,13-dimethyl-3-oxo-2,3,6,7,8,9,10,11,12,13,14,15,16,17-tetradecahydro-1 H-cyclopenta[a]phenanthrene-17-carboxylate | 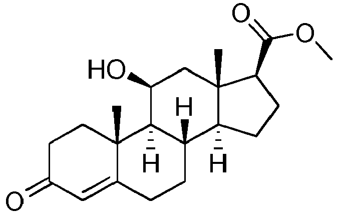 |
| 1006. | 1-((3S,8S,9S,10R,11S,13S,14S,17S)-3,11-dihydroxy-10,13-dimethyl-2,3,6,7,8,9,10,11,12,13,14,15,16,17-tetradecahydro-1 H-cyclopenta[a]phenanthren-17-yl)ethanone | 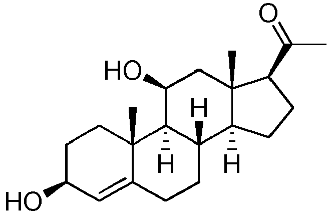 |
| 1007. | (8S,9S,10R,11S,13S,14S,17S)-17-acetyl-11-hydroxy-10,13-dimethyl-6,7,8,9,10,11,12,13,14,15,16,17-dodecahydro-1 H-cyclopenta[a]phenanthren-3(2H)-one | 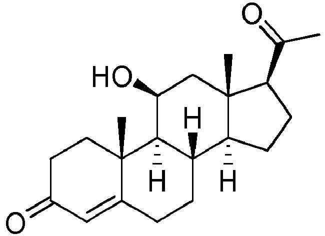 |
| 1008 . | 1-((3S,8S,9S,10S,11S,13S,14S,17S)-3,11-dihydroxy-10,13-dimethylhexadecahydro-1 H-cyclopenta[a]phenanthren-17-yl)ethanone | 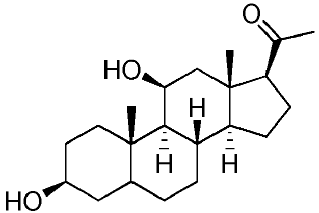 |
| 1009. | (3S,8S,9S,10R,11S,13R,14S,17S)-17-ethyl-10,13-dimethyl-2,3,4,7,8,9,10,11,12,13,14,15,16,17-tetradecahydro-1 H-cyclopenta[a]phenanthrene-3,11-diol | 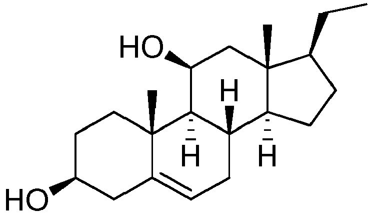 |
| 1010. | (8S,9S,10R,11S,13S,14S,17R)-17-((R)-1,2-dihydroxyethyl)-11,17-dihydroxy-10,13-dimethyl-6,7,8,9,10,11,12,13,14,15,16,17 -dodecahydro-1 H-cyclopenta[a]phenanthren-3(2H)-one | 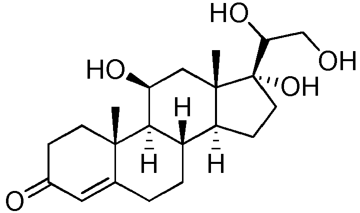 |
| 1011. | (8S,9S,10R,11S,13S,14S)-11-hydroxy-10,13-dimethyl-7,8,9,1 0,11,12,13,14,15, 16-decahydro-1 H-cyclopenta[a]phenanthrene-3,17(2H,6H)-dione | 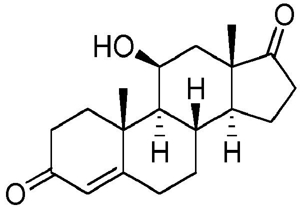 |
| 1012 . | (8S,9S,10R,11S,13S,14S,17S)-11-hydroxy-10,13-dimethyl-17-(3-methyl-1,2,4-oxadiazol-5-yl)-6,7,8,9,10,11,12,13,14,15,16,17 -dodecahydro-1 H-cyclopenta[a]phenanthren-3(2H)-one | 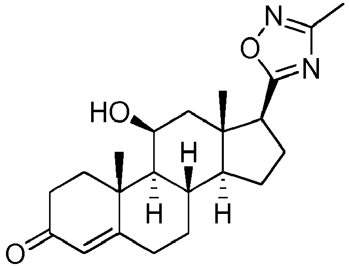 |
| 1013 . | Trimethylammonium (3S,8S,9S,10R,11S,13S,14S,17S)-17-acetyl-11-hydroxy-10,13-dimethyl-2,3,4,7,8,9,10,11,12,13,14,15,16,17 -tetradecahydro-1H-cyclopenta[a]phenanthren-3-yl sulfate | 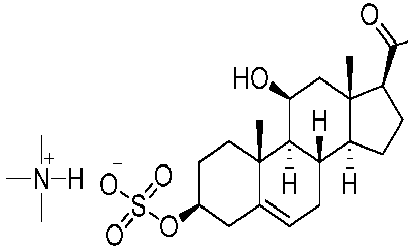 |
| 1014 . | (8S,9S,10R,11S,13S,14S,17S)-11-hydroxy-17-(2-hydroxyacetyl)-10, 13-dimethyl-6,7,8,9,10,11,12,13,14,15,16,17 -dodecahydro-1H-cyclopenta[a]phenanth ren-3(2H)-one | 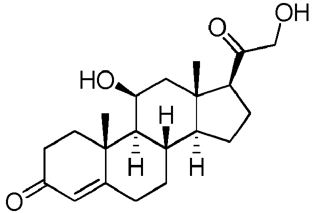 |
| 1015 . | (8S,9S,10R,11S,13S,14S,17R)-11,17-dihydroxy-17-(2-hydroxyacetyl)-10,13-dimethyl-6,7,8,9,10,11,12,13,14,15,16,17 -dodecahydro-1 H-cyclopenta[a]phenanthren-3(2H)-one | 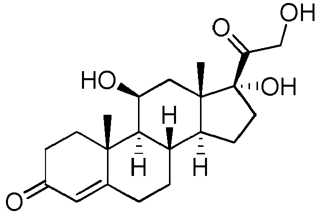 |
| 1016 | 1-((3S,10R,11S,13S,17S)-3,11-dihydroxy-10,13-dimethyl-2,3,4,7,8,9,10,11,12,13,14,15,16,17-tetradecahydro-1 H-cyclopenta[a]phenanthren-17-yl)ethan-1-one | 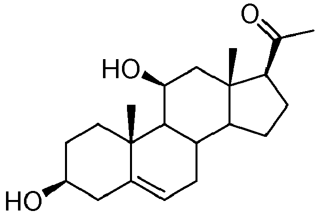 |
| 1017 | (3S,10R,11S,13S,17S)-10,13-dimethyl-2,3,4,7,8,9,10,11,12,13,14,15,16,17-tetradecahydro-1 H-cyclopenta[a]phenanthrene-3, 11,17 -triol | 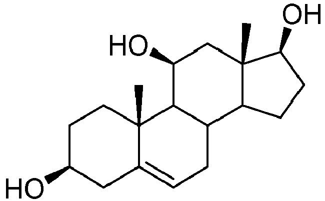 |
| 1018 | (10R,11S,13S,17S)-11,17 -dihydroxy-10, 13-dimethyl-1,2,6,7,8,9,10,11,12,13,14,15,16,17-tetradecahydro-3H-cyclopenta[a]phenanth ren-3-one | 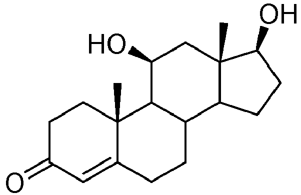 |
| 1019 | (10R,11S,13S)-11-hydroxy-10, 13-dimethyl-1,6,7,8,9,10,11,12,13,14,15,16-dodecahydro-3H-cyclopenta[a]phenanthrene-3,17(2H)-dione | 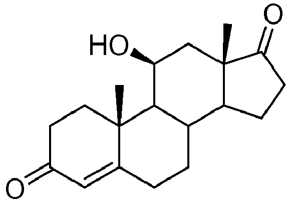 |
| 1020 | (10R,11S,13S)-11-hydroxy-10, 13-dimethyl-3-oxo-2,3,6,7,8,9,10,11,12,13,14,15,16,17-tetradecahydro-1H-cyclopenta[a]phenanthrene-17-carboxylic acid | 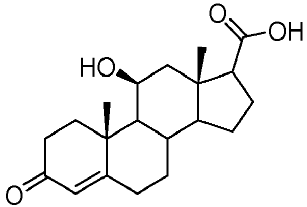 |
| 1021 | (10R,11S,13S)-17-(1-hydroxyethyl)-10, 13-dimethyl-1,2,4,7,8,9,10,11,12,13,14,15,16,17-tetradecahydrospiro[cyclopenta[a]phenanthrene-3,2'-[1,3]dioxolan]-11-ol | 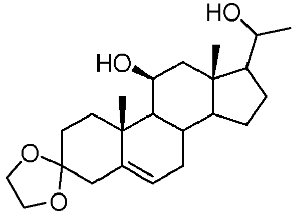 |
| 1022 | (3Z,10R,11S,13S,17E)-11-hydroxy-10,13-dimethyl-1,6,7,8,9,10,11,12,13,14,15,16-dodecahydro-3H-cyclopenta[a]phenanthrene-3,17(2H)-dione dioxime | 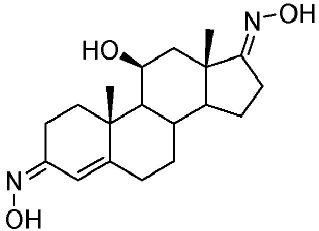 |
| 1023 | (10R,11S,13S,17R)-11,17-dihydroxy-10,13-dimethyl-3-oxo-2,3,6,7,8,9,10,11,12,13,14,15,16,17-tetradecahydro-1H-cyclopenta[a]phenanthrene-17-carboxylic acid | 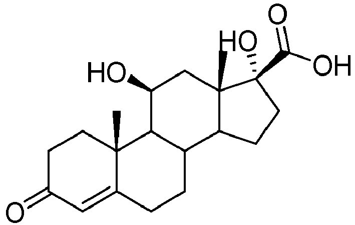 |
| 1024 | (10R,11S,13S,E)-11-hydroxy-17-(hydroxyimino)-10,13-dimethyl-1,2,6,7,8,9,10,11,12,13,14,15,16,17-tetradecahydro-3H-cyclopenta[a]phenanthren-3-one | 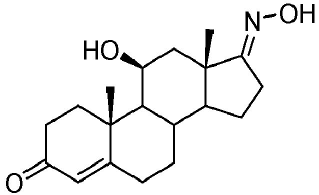 |
| 1025 | (10R,11S,13S,17S)-17-acetyl-11-hydroxy-10,13-dimethyl-1,2,6,7,8,9,10,11,12,13,14,15,16,17-tetradecahydro-3H-cyclopenta[a]phenanthren-3-one | 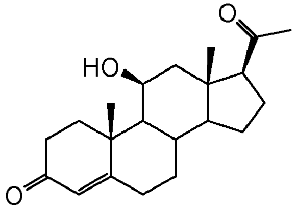 |
| 1026 | (10S,11S,13S,17S)-17-acetyl-11-hydroxy-10,13-dimethylhexadecahydro-3H-cyclopenta[a]phenanth ren-3-one | 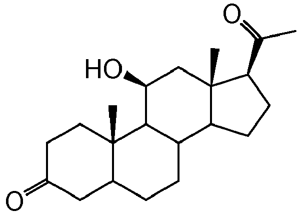 |
| 1027 | (3S,10R,11S,13S,17S)-10,13-dimethyl-2,3,6,7,8,9,10,11,12,13,14,15,16,17-tetradecahydro-1H-cyclopenta[a]phenanthrene-3, 11,17 -triol | 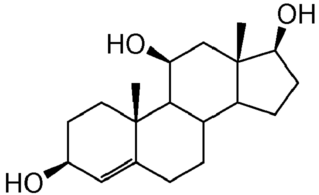 |
| 1028 | (10R,11S,13S,17R)-11-hydroxy-10,13-dimethyl-1,6,7,8,9,10,11,12,13,14,15,16-dodecahydrospiro[cyclopenta[a]phenanthrene-17,2'-oxetane]-3,3'(2H)-dione | 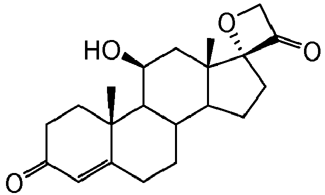 |
| 1029 | 1-((10R,11S,13S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one | 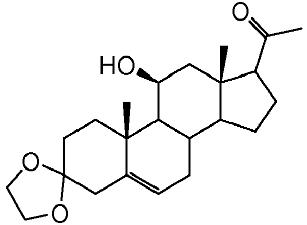 |
| 1030 | (10R,11S,13S)-11-hydroxy-N,N,10,13-tetramethyl-3-oxo-2,3,6,7,8,9,10,11,12,13,14,15,16,17-tetradecahydro-1H-cyclopenta[a]phenanthrene-17-carboxamide | 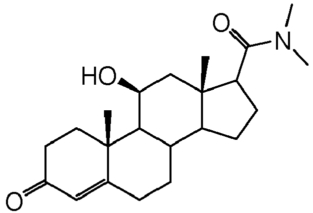 |
| 1031 | (10R,11S,13S,17S,Z)-11-hydroxy-17-((Z)-1-(hydroxyimino)ethyl)-10,13-dimethyl-1,2,6,7,8,9,10,11,12,13,14,15,16,17-tetradecahydro-3H-cyclopenta[a]phenanthren-3-one oxime | 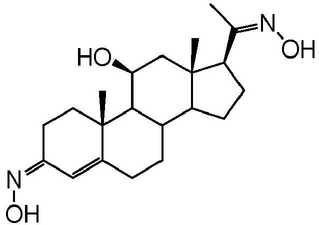 |
| 1032 | (10R,11S,13S)-11-hydroxy-17-(1-hydroxyethyl)-10,13-dimethyl-1,2,6,7,8,9,10,11,12,13,14,15,16,17-tetradecahydro-3H-cyclopenta[a]phenanthren-3-one | 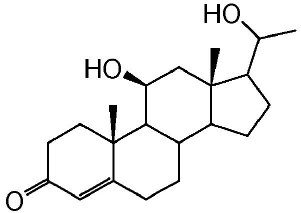 |
| 1033 | (10S,11S,13S)-11-hydroxy-N,N,10,13-tetramethyl-3-oxohexadecahydro-1 H-cyclopenta[a]phenanthrene-17-carboxamide | 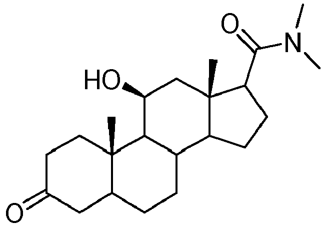 |
| 1034 | (10S,11S,13S)-11-hydroxy-10,13-dimethyltetradecahydro-3H-cyclopenta[a]phenanthrene-3,17(2H)-dione | 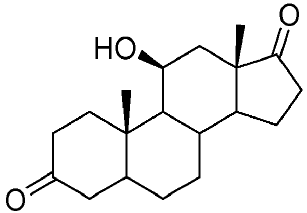 |
| 1035 | (10R,11S,13S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one | 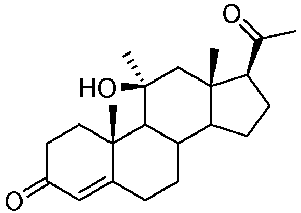 |
| 1036 | (3S,10R,11S,13S)-17-(1-hydroxyethyl)-10,13-dimethyl-2,3,4,7,8,9,10,11,12,13,14,15,16,17-tetradecahydro-1H-cyclopenta[a]phenanthrene-3,11-diol | 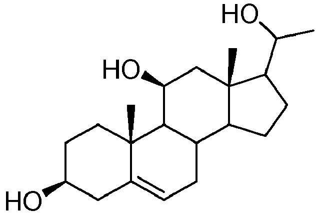 |
| 1037 | (3S,8S,9S,10R,11S,13S,14S,17S)-17-(1-hydroxyethyl)-10,13-dimethyl-2,3,6,7,8,9,10,11,12,13,14,15,16,17-tetradecahydro-1 H-cyclopenta[a]phenanthrene-3,11-diol | 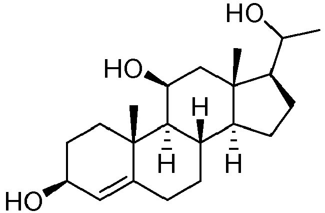 |
| 1038 | 1-((3S,10R,11S,13S,17S)-11-hydroxy-3-methoxy-10,13-dimethyl-2,3,4,7,8,9,10,11,12,13,14,15,16,17-tetradecahydro-1H-cyclopenta[a]phenanthren-17-yl)ethan-1-one | 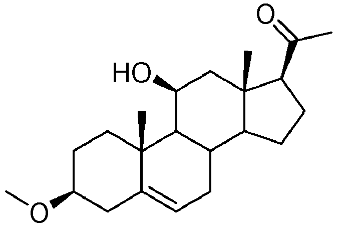 |
| 1039 | (10S,11S,13S)-17-(1-hydroxyethyl)-10,13-dimethylhexadecahydrospiro[cyclopenta[a]phenanthr ene-3,2'-[1,3]dioxolan]-11-ol | 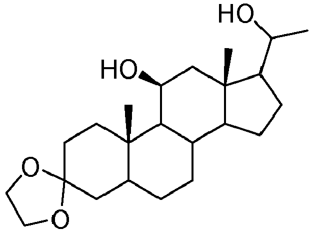 |
| 1040 | (3S,10S,11S,13S,17S)-17-(1-hydroxyethyl)-10,13-dimethylhexadecahydro-1 H-cyclopenta[a]phenanthrene-3,11-diol | 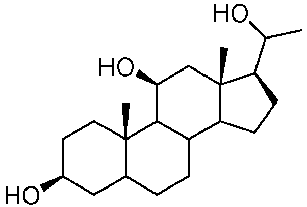 |
| 1041 | (3S,10R,11S,13R,17S)-17-ethyl-10,13-dimethyl-2,3,4,7,8,9,10,11,12,13,14,15,16,17 -tetradecahydro-1H-cyclopenta[a]phenanthrene-3,11-diol | 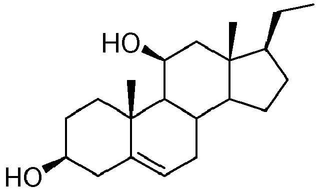 |
| 1042 | 1-((3S,10S,11S,13S,17S)-3,11-dihydroxy-10,13-dimethylhexadecahydro-1 H-cyclopenta[a]phenanthren-17-yl)ethan-1-one | 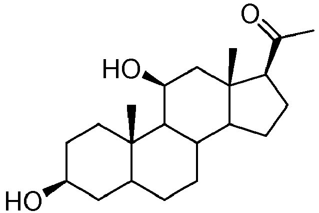 |
| 1043 | (3S,10R,11S,13S,17S)-3,11-dihydroxy-N,N,10,13-tetramethyl-2,3,4,7,8,9,10,11,12,13,14,15,16,17-tetradecahydro-1H-cyclopenta[a]phenanthrene-17-carboxamide | 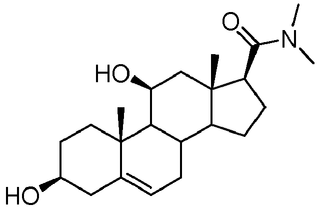 |
| 1044 | 1-((8S,9S,10R,11S,13S,14S,17S)-11-hydroxy-10,13-dimethyl-2,7,8,9,10,11,12,13,14,15,16,17-dodecahydro-1H-cyclopenta[a]phenanthren-17-yl)ethan-1-one | 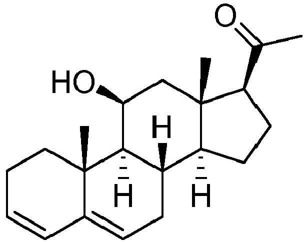 |
| 1045 | 1-((3S,10R,11S,13S,17S)-3,11-dihydroxy-10,13-dimethyl-2,3,4,7,8,9,10,11,12,13,14,15,16,17-tetradecahydro-1 H-cyclopenta[a]phenanthren-17-yl-3-d)ethan-1-one | 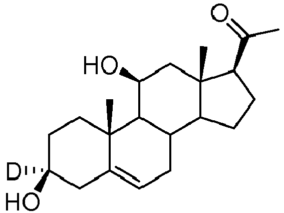 |
| 1046 | (3S,10R,11S,13S,17S)-3,11-dihydroxy-N,N,10,13-tetramethyl-2,3,6,7,8,9,10,11,12,13,14,15,16,17-tetradecahydro-1H-cyclopenta[a]phenanthrene-17-carboxamide | 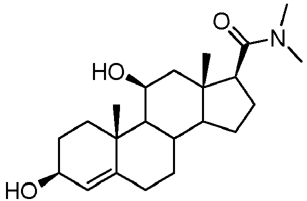 |
| 1047 | 1-((3S,10R,11S,13S,17S)-3,11-dihydroxy-10,11,13-trimethyl-2,3,4,7,8,9,10,11,12,13,14,15,16,17-tetradecahydro-1 H-cyclopenta[a]phenanthren-17-yl)ethan-1-one | 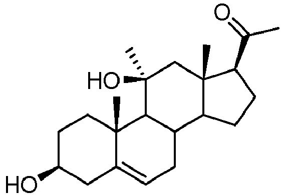 |
| 1048 | (10R,11S,13S)-11-hydroxy-17-(2-hydroxyacetyl)-10,13-dimethyl-1,2,6,7,8,9,10,11,12,13,14,15,16,17-tetradecahydro-3H-cyclopenta[a]phenanthren-3-one | 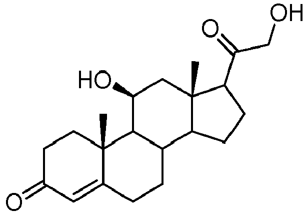 |
| 1049 | (10R,11S,13S,17S)-11-hydroxy-17-(2-hydroxypropan-2-yl)-10,13-dimethyl-1,2,6,7,8,9,10,11,12,13,14,15,16,17-tetradecahydro-3H-cyclopenta[a]phenanth ren-3-one | 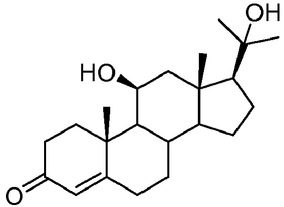 |
| 1050 | (E)-1-((3S,10R,11S,13S)-3,11-dihydroxy-10,13-dimethyl-2,3,4,7,8,9,10,11,12,13,14,15,16,17-tetradecahydro-1 H-cyclopenta[a]phenanthren-17-yl)ethan-1-one oxime | 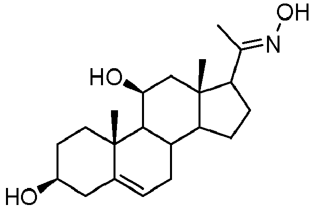 |
| 1051 | 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-11-d)ethan-1-one | 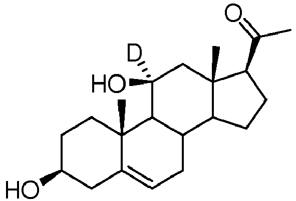 |
| 1052 | (3S,10R,11S,13S,17S)-17-((E)-1-hydrazonoethyl)-10,13-dimethyl-2,3,4,7,8,9,10,11,12,13,14,15,16,17-tetradecahydro-1 H-cyclopenta[a]phenanthrene-3,11-diol | 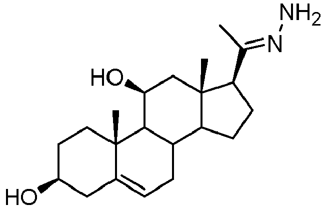 |
| 1053 | 1-((10S,11S,13S,17S)-11-hydroxy-10,13-dimethylhexadecahydro-1 H-cyclopenta[a]phenanthren-17-yl)ethan-1-one | 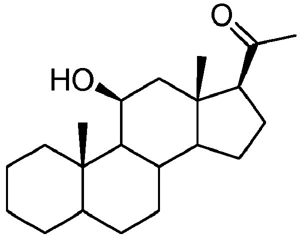 |
| 1054 | (3S,10S,11S,13S)-17-(1-aminoethyl)-10,13-dimethylhexadecahydro-1 H-cyclopenta[a]phenanthrene-3,11-diol | 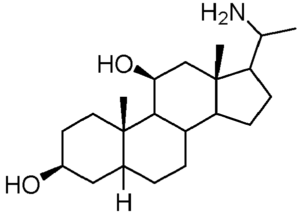 |
| 1055 | 1-((3R,10R,11S,13S,17S)-3-fluoro-11-hydroxy-10,13-dimethyl-2,3,4,7,8,9,10,11,12,13,14,15,16,17-tetradecahydro-1 H-cyclopenta[a]phenanthren-17-yl)ethan-1-one | 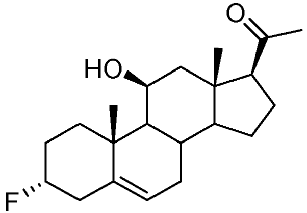 |
| 1056 | (3S,10S,11S,13S)-17-amino-10,13-dimethylhexadecahydro-1 H-cyclopenta[a]phenanthrene-3,11-diol | 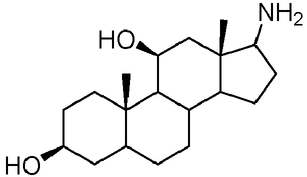 |
| 1057 | (10R,11S,13S)-11-hydroxy-10, 13-dimethyl-17-(piperazine-1-carbonyl)-1,2,6,7,8,9,10,11,12,13,14,15,16,17-tetradecahydro-3H-cyclopenta[a]phenanth ren-3-one | 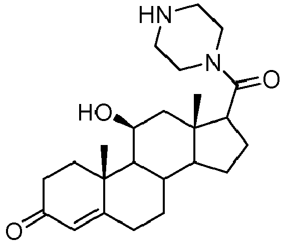 |
| 1058 | (8S,9S,10R,11S,13S,14S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one | 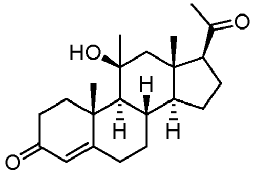 |
| 1059 | 2-amino-N-((1S)-1-((3S,10S,11S,13S,17S)-3,11-dihydroxy-1 0, 13-dimethylhexadecahydro-1 H-cyclopenta[a]phenanthren-17-yl)ethyl)acetamide hydrochloride | 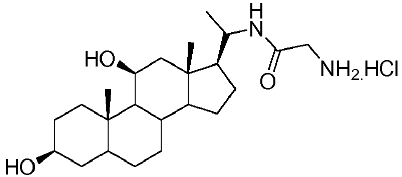 |
| 1060 | (8S,9S,10R,11S,13S,14S,17S)-*N*-(2-aminoethyl)-11-hydroxy-10,13-dimethyl-3-oxo-2,3,6,7,8,9,10,11,12,13,14,15,16-tetradecahydro-*1H-*cyclopenta[a]phenanthrene-17-carboxamide | 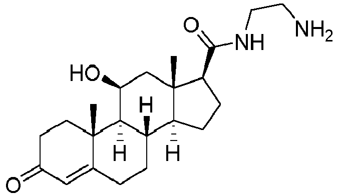 |
| 1061 | 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3,11-d₂)ethan-1-one | 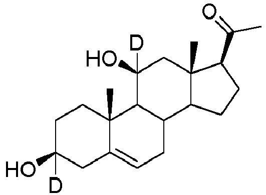 |
| 1062 | (3S,10R,11S,13S,17S)-2,3,6,7,8,9,10,11,12,13,14,15,16,17-tetradecahydro-10,13-dimethyl-1H-cyclopenta[a]phenanthrene-3,11,17-triol | 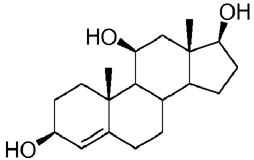 |
| 1063 | (3S,8S,9S,10S,11S,13S,14S,17S)-3,11-dihydroxy-N,10,13-trimethylhexadecahydro-1H-cyclopenta[a]phenanthrene-17-carboxamide | 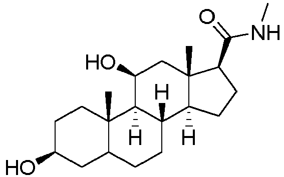 |
| 1064 | (3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide | 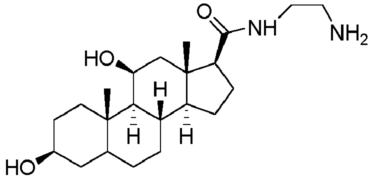 |
| 1065 | (3S,10R,11S,13S,17S)-5-methoxy-10,13-dimethyl-17-(2-methyl-1,3-dioxolan-2-yl)hexadecahydro-1 H-cyclopenta[a]phenanthrene-3,6,11-triol | 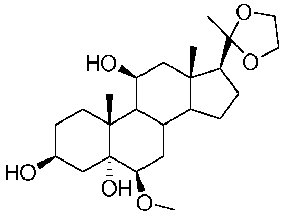 |
| 1066 | 1-((3S,5R,6R,10R,11 S,13S,17S)-3,5,11-trihydroxy-6-methoxy-10,13-dimethylhexadecahydro-1 H-cyclopenta[a]phenanthren-17-yl)ethan-1-one | 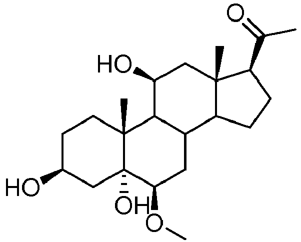 |
| 1069 | 1-((3S,5R,6R,10R,11S,13S,17S)-3,5,6,11-tetrahydroxy-10,13-dimethylhexadecahydro-1H-cyclopenta[a]phenanthren-17-yl)ethan-1-one | 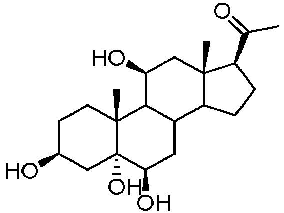 |
| 1070 | 3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol | 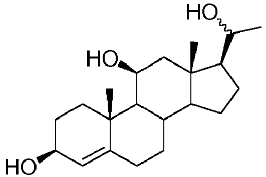 |

The compounds of the present invention include:
I. (3S,8S,9S,10R,11S,13S,14S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol;
II. 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethanone;
III. (3S,8S,9S,10R,11S,13S,14S,17S)-17-(1-hydroxyethyl)-10,13-dimethyl-2,3,4,7,8,9,10,11,12,13,14,15,16,17-tetradecahydro-1H-cyclopenta[a]phenanthrene-3,11-diol;
IV. (3S,8S,9S,10R,11S,13S,14S,17S)-methyl 3,11-dihydroxy-10,13-dimethyl-2,3,4,7,8,9,10,11,12,13,14,15,16,17-tetradecahydro-1H-cyclopenta[a]phenanthrene-17-carboxylate;
V. (8S,9S,10R,11S,13S,14S,17S)-methyl11-hydroxy-10,13-dimethyl-3-oxo-2,3,6,7,8,9,10,11,12,13,14,15,16,17-tetradecahydro-1H-cyclopenta[a]phenanthrene-17-carboxylate;
VI. 1-((3S,8S,9S,10R,11S,13S,14S,17S)-3,11-dihydroxy-10,13-dimethyl-2,3,6,7,8,9,10,11,12,13,14,15,16,17-tetradecahydro-1H-cyclopenta[a]phenanthren-17-yl)ethanone;
VII. (8S,9S,10R,11S,13S,14S,17S)-17-acetyl-11-hydroxy-10,13-dimethyl-6,7,8,9,10,11,12,13,14,15,16,17-dodecahydro-1H-cyclopenta[a]phenanthren-3(2H)-one;
VIII. 1-((3S,8S,9S,10S,11S,13S,14S,17S)-3,11-dihydroxy-10,13-dimethylhexadecahydro-1H-cyclopenta[a]phenanthren-17-yl)ethanone;
IX. (3S,8S,9S,10R,11S,13R,14S,17S)-17-ethyl-10,13-dimethyl-2,3,4,7,8,9,10,11,12,13,14,15,16,17-tetradecahydro-1H-cyclopenta[a]phenanthrene-3,11-diol;
X. (8S,9S,10R,11S,13S,14S,17R)-17-((R)-1,2-dihydroxyethyl)-11,17-dihydroxy-10,13-dimethyl-6,7,8,9,10,11,12,13,14,15,16,17-dodecahydro-1H-cyclopenta[a]phenanthren-3(2H)-one;
XI. (8S,9S,10R,11S,13S,14S)-11-hydroxy-10,13-dimethyl-7,8,9,10,11,12,13,14,15,16-decahydro-1H-cyclopenta[a]phenanthrene-3,17(2H,6H)-dione
XII. (8S,9S,10R,11S,13S,14S,17S)-11-hydroxy-10,13-dimethyl-17-(3-methyl-1,2,4-oxadiazol-5-yl)-6,7,8,9,10,11,12,13,14,15,16,17-dodecahydro-1H-cyclopenta[a]phenanthren-3(2H)-one;
XIII. Trimethylammonium (3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl sulfate;
XIV. (8S,9S,10R,11S,13S,14S,17S)-11-hydroxy-17-(2-hydroxyacetyl)-10,13-dimethyl-6,7,8,9,10,11,12,13,14,15,16,17-dodecahydro-1H-cyclopenta[a]phenanthren-3(2H)-one;
XV. (8S,9S,10R,11 S,13S,14S,17R)-11,17-dihydroxy-17-(2-hydroxyacetyl)-10,13-dimethyl-6,7,8,9,10,11,12,13,14,15,16,17-dodecahydro-1H-cyclopenta[a]phenanthren-3(2H)-one;
XVI. 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one;
XVII. (3S,10R,11S,13S,17S)-10,13-dimethyl-2,3,4,7,8,9,10,11,12,13,14,15,16,17-tetradecahydro-1H-cyclopenta[a]phenanthrene-3,11,17-triol;
XVIII. (10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one;
XIX. (10R,11S,13S)-11-hydroxy-10,13-dimethyl-1,6,7,8,9,10,11,12,13,14,15,16-dodecahydro-3H-cyclopenta[a]phenanthrene-3,17(2H)-dione;
XX. (10R,11S,13S)-11-hydroxy-10,13-dimethyl-3-oxo-2,3,6,7,8,9,10,11,12,13,14,15,16,17-tetradecahydro-1H-cyclopenta[a]phenanthrene-17-carboxylic acid;
XXI. (10R,11S,13S)-17-(1-hydroxyethyl)-10,13-dimethyl-1,2,4,7,8,9,10,11,12,13,14,15,16,17-tetradecahydrospiro[cyclopenta[a]phenanthrene-3,2'-[1,3]dioxolan]-11-ol;
XXII. (3Z,10R,11S,13S,17E)-11-hydroxy-10,13-dimethyl-1,6,7,8,9,10,11,12,13,14,15,16-dodecahydro-3H-cyclopenta[a]phenanthrene-3,17(2H)-dione dioxime;
XXIII. (10R,11S,13S,17R)-11,17-dihydroxy-10,13-dimethyl-3-oxo-2,3,6,7,8,9,10,11,12,13,14,15,16,17-tetradecahydro-1H-cyclopenta[a]phenanthrene-17-carboxylic acid;
XXIV. (10R,11S,13S,E)-11-hydroxy-17-(hydroxyimino)-10,13-dimethyl-1,2,6,7,8,9,10,11,12,13,14,15,16,17-tetradecahydro-3H-cyclopenta[a]phenanthren-3-one;
XXV. (10R,11S,13S,17S)-17-acetyl-11-hydroxy-10,13-dimethyl-1,2,6,7,8,9,10,11,12,13,14,15,16,17-tetradecahydro-3H-cyclopenta[a]phenanthren-3-one;
XXVI. (10S,11S,13S,17S)-17-acetyl-11-hydroxy-10,13-dimethylhexadecahydro-3H-cyclopenta[a]phenanthren-3-one;
XXVII. (3S,10R,11S,13S,17S)-10,13-dimethyl-2,3,6,7,8,9,10,11,12,13,14,15,16,17-tetradecahydro-1H-cyclopenta[a]phenanthrene-3,11,17-triol;
XXVIII. (10R,11S,13S,17R)-11-hydroxy-10,13-dimethyl-1,6,7,8,9,10,11,12,13,14,15,16-dodecahydrospiro[cyclopenta[a]phenanthrene-17,2'-oxetane]-3,3'(2H)-dione;
XXIX. 1-((10R,11S,13S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one;
XXX. (10R,11S,13S)-11-hydroxy-N,N,10,13-tetramethyl-3-oxo-2,3,6,7,8,9,10,11,12,13,14,15,16,17-tetradecahydro-1H-cyclopenta[a]phenanthrene-17-carboxamide;
XXXI. (10R,11S,13S,17S,Z)-11-hydroxy-17-((Z)-1-(hydroxyimino)ethyl)-10,13-dimethyl-1,2,6,7,8,9,10,11,12,13,14,15,16,17-tetradecahydro-3H-cyclopenta[a]phenanthren-3-one oxime;
XXXII. (10R,11S,13S)-11-hydroxy-17-(1-hydroxyethyl)-10,13-dimethyl-1,2,6,7,8,9,10,11,12,13,14,15,16,17-tetradecahydro-3H-cyclopenta[a]phenanthren-3-one;
XXXIII. (10S,11S,13S)-11-hydroxy-N,N,10,13-tetramethyl-3-oxohexadecahydro-1H-cyclopenta[a]phenanthrene-17-carboxamide;
XXXIV. (10S,11S,13S)-11-hydroxy-10,13-dimethyltetradecahydro-3H-cyclopenta[a]phenanthrene-3,17(2H)-dione;
XXXV. (10R,11S,13S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one;
XXXVI. (3S,10R,11 S,13S)-17-(1-hydroxyethyl)-10,13-dimethyl-2,3,4,7,8,9,10,11,12,13,14,15,16,17-tetradecahydro-1H-cyclopenta[a]phenanthrene-3,11-diol;
XXXVII. (3S,8S,9S,10R,11S,13S,14S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol;
XXXVIII. 1-((3S,10R,11S,13S,17S)-11-hydroxy-3-methoxy-10,13-dimethyl-2,3,4,7,8,9,10,11,12,13,14,15,16,17-tetradecahydro-1H-cyclopenta[a]phenanthren-17-yl)ethan-1-one;
XXXIX. (10S,11S,13S)-17-(1-hydroxyethyl)-10,13-dimethylhexadecahydrospiro[cyclopenta[a]phenanthrene-3,2'-[1,3]dioxolan]-11-ol;
XL. (3S,10S,11S,13S,17S)-17-(1-hydroxyethyl)-10,13-dimethylhexadecahydro-1H-cyclopenta[a]phenanthrene-3,11-diol;
XLI. (3S,10R,11S,13R,17S)-17-ethyl-10,13-dimethyl-2,3,4,7,8,9,10,11,12,13,14,15,16,17-tetradecahydro-1H-cyclopenta[a]phenanthrene-3,11-diol;
XLII. 1-((3S,10S,11S,13S,17S)-3,11-dihydroxy-10,13-dimethylhexadecahydro-1H-cyclopenta[a]phenanthren-17-yl)ethan-1-one;
XLIII. (3S,10R,11S,13S,17S)-3,11-dihydroxy-N,N,10,13-tetramethyl-2,3,4,7,8,9,10,11,12,13,14,15,16,17-tetradecahydro-1H-cyclopenta[a]phenanthrene-17-carboxamide;
XLIV. 1-((8S,9S,10R,11S,13S,14S,17S)-11-hydroxy-10,13-dimethyl-2,7,8,9,10,11,12,13,14,15,16,17-dodecahydro-1H-cyclopenta[a]phenanthren-17-yl)ethan-1-one;
XLV. 1-((3S,10R,11 S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3-d)ethan-1-one;
XLVI. (3S,10R,11S,13S,17S)-3,11-dihydroxy-N,N,10,13-tetramethyl-2,3,6,7,8,9,10,11,12,13,14,15,16,17-tetradecahydro-1H-cyclopenta[a]phenanthrene-17-carboxamide;
XLVII. 1-((3S,10R,11S,13S,17S)-3,11-dihydroxy-10,11,13-trimethyl-2,3,4,7,8,9,10,11,12,13,14,15,16,17-tetradecahydro-1H-cyclopenta[a]phenanthren-17-yl)ethan-1-one;
XLVIII. (10R,11S,13S)-11-hydroxy-17-(2-hydroxyacetyl)-10,13-dimethyl-1,2,6,7,8,9,10,11,12,13,14,15,16,17-tetradecahydro-3H-cyclopenta[a]phenanthren-3-one;
XLIX. (10R,11S,13S,17S)-11-hydroxy-17-(2-hydroxypropan-2-yl)-10,13-dimethyl-1,2,6,7,8,9,10,11,12,13,14,15,16,17-tetradecahydro-3H-cyclopenta[a]phenanthren-3-one;
L. (E)-1-((3S,10R,11S,13S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one oxime;
LI. 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-11-d)ethan-1-one;
LII. (3S,10R,11S,13S,17S)-17-((E)-1-hydrazonoethyl)-10,13-dimethyl-2,3,4,7,8,9,10,11,12,13,14,15,16,17-tetradecahydro-1H-cyclopenta[a]phenanthrene-3,11-diol;
LIII. 1-((10S,11S,13S,17S)-11-hydroxy-10,13-dimethylhexadecahydro-1H-cyclopenta[a]phenanthren-17-yl)ethan-1-one;
LIV. (3S,10S,11S,13S)-17-(1-aminoethyl)-10,13-dimethylhexadecahydro-1H-cyclopenta[a]phenanthrene-3,11-diol;
LV. 1-((3R,10R,11S,13S,17S)-3-fluoro-11-hydroxy-10,13-dimethyl-2,3,4,7,8,9,10,11,12,13,14,15,16,17-tetradecahydro-1H-cyclopenta[a]phenanthren-17-yl)ethan-1-one;
LVI. (3S,10S,11S,13S)-17-amino-10,13-dimethylhexadecahydro-1H-cyclopenta[a]phenanthrene-3,11-diol;
LVII. (10R,11S,13S)-11-hydroxy-10,13-dimethyl-17-(piperazine-1-carbonyl)-1,2,6,7,8,9,10,11,12,13,14,15,16,17-tetradecahydro-3H-cyclopenta[a]phenanthren-3-one;
LVIII. (8S,9S,10R,11S,13S,14S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one;
LIX. (8S,9S,10R,11S,13S,14S,178)-*N*-(2-aminoethyl)-11-hydroxy-10,13-dimethyl-3-oxo-2,3,6,7,8,9,10,11,12,13,14,15,16-tetradecahydro-*1H*-cyclopenta[a]phenanthrene-17-carboxamide;
LX. 1-((3S,10R,11S,13S,17S)-3,11-dihydroxy-10,13-dimethyl-2,3,4,7,8,9,10,11,12,13,14,15,16,17-tetradecahydro-1H-cyciopenta[a]phenanthren-17-yl-3,11-d₂)ethan-1-one;
LXI. (3S,8S,9S,10S,11S,13S,14S,17S)-3,11-dihydroxy-N,10,13-trimethylhexadecahydro-1H-cyclopenta[a]phenanthrene-17-carboxamide;
LXII. (3S,10R,11S,13S,17S)-2,3,6,7,8,9,10,11,12,13,14,15,16,17-tetradecahydro-10,13-dimethyl-1H-cyclopenta[a]phenanthrene-3,11,17-triol;
LXIII. (3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide;
LXIV. (3S,10R,11S,13S,17S)-5-methoxy-10,13-dimethyl-17-(2-methyl-1,3-dioxolan-2-yl)hexadecahydro-1H-cyclopenta[a]phenanthrene-3,6,11-triol;
LXV. 1-((3S,5R,6R,10R,11S,13S,17S)-3,5,11-trihydroxy-6-methoxy-10,13-dimethylhexadecahydro-1H-cyclopenta[a]phenanthren-17-yl)ethan-1-one;
LXVI. 1-((3S,5R,6R,10R,11S,13S,17S)-3,5,11-trihydroxy-6-methoxy-10,13-dimethylhexadecahydro-1H-cyclopenta[a]phenanthren-17-yl)ethan-1-one;
LXVII. 1-((3S,5R,6R,10R,11S,13S,17S)-3,5,6,11-tetrahydroxy-10,13-dimethylhexadecahydro-1H-cyclopenta[a]phenanthren-17-yl)ethan-1-one;
LXVIII. 3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol.

The invention is further illustrated by the following examples. The following examples are representative of the disclosure, and provide detailed methods for preparing the compounds of the disclosure, including the preparation of the intermediate compounds. The preparation of particular compounds are described in detail in the following examples, but the artisan will recognize that the chemical reactions described may be readily adapted to prepare a number of other agents of the various embodiments. For example, the synthesis of non-exemplified compounds may be successfully performed by modifications apparent to those skilled in the art, e.g. by appropriately protecting interfering groups, by changing to other suitable reagents known in the art, or by making routine modifications of reaction conditions. As used herein the symbols and conventions used in these process, schemes and examples, regardless of whether a particular abbreviation is specifically defined, are consistent with those used in the contemporary scientific literature, for example, the Journal of American Chemical Society or the Journal of Biological Chemistry.

For all of the following examples, standard work-up and purification methods known to those skilled in the art may be utilized. Unless otherwise indicated, all temperatures are expressed in °C (degrees Centigrade). All reactions conducted at room temperature unless otherwise noted. Synthetic methodologies illustrated herein are intended to exemplify the applicable chemistry through the use of specific examples and are not indicative of the scope of the disclosure.

### EXAMPLE 1[1001]

### (3S,8S,9S,10R,11S,13S,14S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol

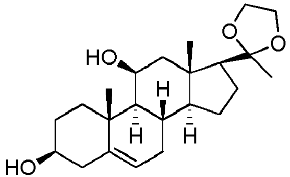

### Step 1

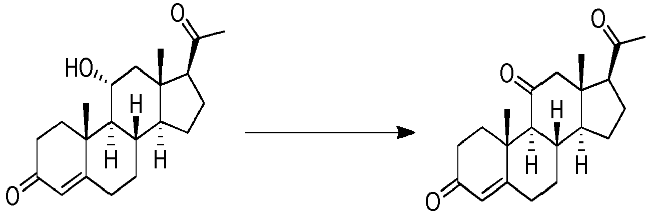

A solution of chromium trioxide (3.3 g, 33.3 mmol) in water (4 mL) was added dropwise to a solution of 17-acetyl-11-hydroxy-10,13-dimethyl-1,2,6,7,8,9,10,11,12,13,14,15,16,17-tetradecahydro-cyclopenta[a]phenanthren-3-one (10 g, 30.3 mmol) in acetic acid (300 ml). The reaction was stirred for 1.5 hr and quenched by the addition of methanol (30 mL), stirring for 0.5 hr. The mixture was concentrated by rotovap at 70 °C, dissolved in ethyl acetate/methanol, washed with saturated sodium bicarbonate and brine, dried over sodium sulfate and concentrated to give 17-acetyl-10,13-dimethyl-1,6,7,8,9,10,12,13,14,15,16,17-dodecahydro-2H-cyclopenta[a]phenanthrene-3,11-dione as an off white solid (9.0 g).

### Step 2

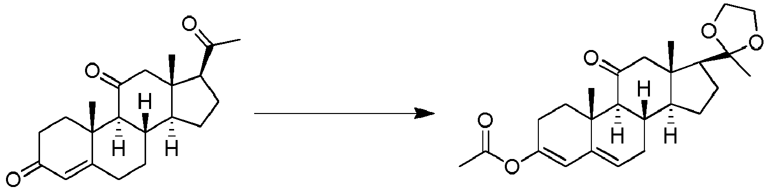

A solution of perchloric acid (10 ul) in ethyl acetate (10 ml) is added to a solution of acetic anhydride (4.8 mL) in ethyl acetate (30 ml) and the final volume is brought up to 50 ml. 17-Acetyl-10,13-dimethyl-1,6,7,8,9,10,12,13,14,15,16,17-dodecahydro-2H-cyclopenta[a]phenanthrene-3,11-dione (500 mg, 1.5 mmol) was added and the reaction was stirred for 7 min then washed with saturated sodium bicarbonate, dried over sodium sulfate, and concentrated by rotovap at 70 °C. The resulting oil was dissolved in methanol (10 ml), treated with pyridine (1 ml) and concentrated by rotovap to an oil which was dissolved in ethyl acetate, washed with 10% citric acid, saturated sodium bicarbonate, and brine, dried over sodium sulfate and concentrated to give the acetate diene as an oil which solidified on standing. A mixture of p-toluenesulfonic acid hydrate (110 mg) and ethylene glycol (2 ml) in benzene (25 ml) was refluxed with removal of water for 0.5 hr. A solution of the acetate diene in benzene (12 ml) was added and the reaction was refluxed for 1.5 hr. The mixture was diluted with ethyl acetate, washed with saturated sodium bicarbonate and brine, and dried over sodium sulfate. The organic extracts was concentrated to to obtain acetic acid 10,13-dimethyl-17-(2-methyl-[1,3]dioxolan-2-yl)-11-oxo-2,7,8,9,10,11,12,13,14,15,16,17-dodecahydro-1H-cyclopenta[a]phenanthren-3-yl ester as a off -white crystalline solid (420 mg).

### Step 3

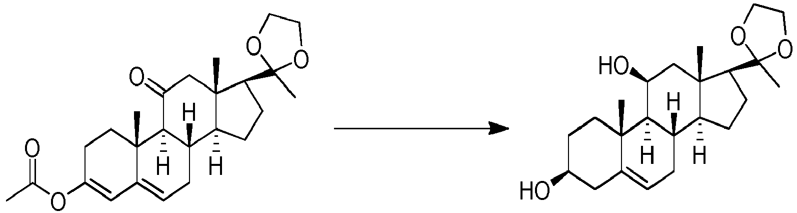

A chilled solution of acetic acid 10,13-dimethyl-17-(2-methyl-[1,3]dioxolan-2-yl)-11-oxo-2,7,8,9,10,11,12,13,14,15,16,17-dodecahydro-1H-cyclopenta[a]phenanthren-3-yl ester (1.59 g) in 7:3 ethanol/THF (125 ml) was added dropwise to an ice cold suspension of sodium borohydride (2.5 g) in 80% ethanol (50 ml). The reaction was stirred in an ice bath for 8 hrs then overnight allowing the ice bath to melt slowly. The reaction was quenched with 10% acetic acid and concentrated by rotovap until a solid forms. The solid is filtered, washed with water and dried to constant weight to give 10,13-dimethyl-17-(2-methyl-[1,3]dioxolan-2-yl)-2,3,4,7,8,9,10,11,12,13,14,15,16,17-tetradecahydro-1H-cyclopenta[a]phenanthrene-3,11-diol as a white solid (797 mg). A second crop is obtained by further concentrating the mother liquor (228 mg). MS (ES-API) *m*/*z* (M+H⁺) 377.8.

### EXAMPLE 2 [1002]

### 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethanone

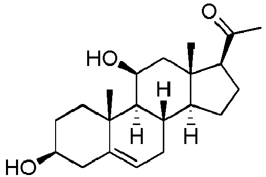

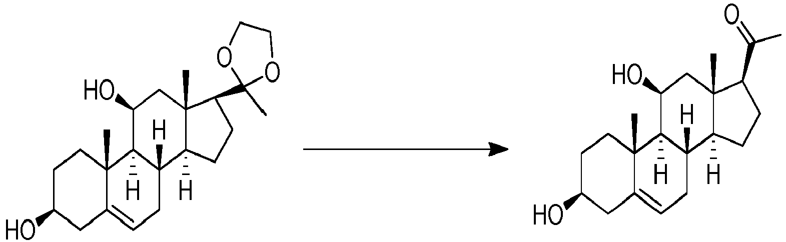

10,13-Dimethyl-17-(2-methyl-[1,3]dioxolan-2-yl)-2,3,4,7,8,9,10,11,12,13,14,15,16,17-tetradecahydro-1H-cyclopenta[a]phenanthrene-3,11-diol (570 mg) was dissolved in 95% acetone (40 ml) and sulfuric acid (10µl, 5%) was added, The resulting mixture was stirred at 0oC for 30 min.. The reaction mixture was concentrated by rotovap until a solid appeared, washed with water and extracted with ethyl acetate. The organic layers were dreid (Na2SO4) concentrated and crude was pudified by column chromatography (100-200 mesh, 2-3 % MeOH/DCM) to give 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one as a white solid (350 mg, 81%). ¹H NMR (400 MHz, DMSO-*d*₆) δ 0.72 (s, 3H), 0.93 (dd, *J* = 11.6 and 3.6 Hz, 1H), 1.04-1.16 (m, 2H), 1.17 (s, 3H), 1.13-1.24 (m, 1H), 1.38 (q, *J* = 12.8 Hz, 1H), 1.46-1.82 (m, 6H), 1.94 (dt, 9.6, *J* = 6.4 and 3.2 Hz, 1H), 1.99 - 2.13 (m, 4H), 2.05 (s, 3H), 2.18 (dd, *J* = 10.8 and 2.8 Hz, 2H), 3.19 - 3.29 (m, 1H), 4.10 (d, *J* = 3.2 Hz, 1H), 4.24 (t, *J* = 3.2 Hz, 1H), 4.57 (d, *J* = 4.4 Hz, 1H), 5.11 (br s, 1H).
ESIMS: 333 (M⁺ + 1)

### EXAMPLE 3 [1003]

### (3S,8S,9S,10R,11S,13S,14S,17S)-17-(1-hydroxyethyl)-10,13-dimethyl-2,3,4,7,8,9,10,11,12,13,14,15,16,17-tetradecahydro-1H-cyclopenta[a]phenanthrene-3,11-diol

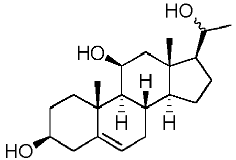

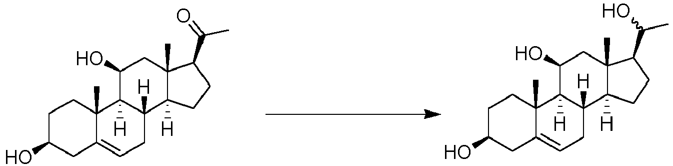

1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one (20 mg, 0.06 mmol) was taken in EtOH (10 ml). NaBH₄ (9 mg, 0.24 mmol) was added and mixture was stirred at room temperature for 2 hrs. The reaction was quenched with brine, extracted with ethyl acetate (50 ml), dried (Na2SO4) concentrated to obtain (3S,8S,9S,10R,11S,13S,14S,17S)-17-(1-hydroxyethyl)-10,13-dimethyl-2,3,4,7,8,9,10,11,12,13,14,15,16,17-tetradecahydro-1H-cyclopenta[a]phenanthrene-3,11-diol
as white solid (20 mg, 98%).ESIMS: 335 (M⁺ + 1)

### EXAMPLE 4 [1004]

### (3S,8S,9S,10R,11S,13S,14S,17S)-methyl 3,11-dihydroxy-10,13-dimethyl-2,3,4,7,8,9,10,11,12,13,14,15,16,17-tetradecahydro-1H-cyclopenta[a]phenanthrene-17-carboxylate

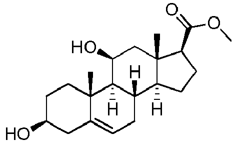

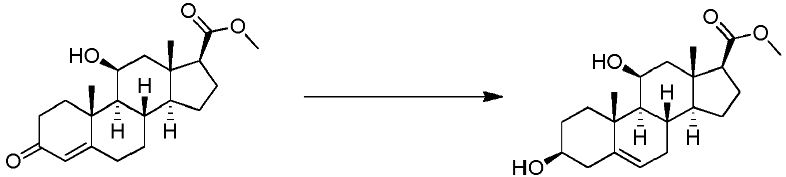

A solution of 70% formic acid (14 ul) in ethyl acetate (10 ml) is added to a solution of acetic anhydride (6.7 mL) in ethyl acetate (50 ml) and the final volume is brought up to 70 ml. 11-Hydroxy-10,13-dimethyl-3-oxo-2,3,6,7,8,9,10,11,12,13,14,15,16,17-tetradecahydro-1H-cyclopenta[a]phenanthrene-17-carboxylic acid methyl ester (700 mg) was added and the reaction was stirred for 7 min then washed with saturated sodium bicarbonate, dried over sodium sulfate, and concentrated by rotovap at 70 °C. The resulting oil was dissolved in methanol (10 ml), treated with pyridine (1 ml) and concentrated by rotovap to an oil which was dissolved in ethyl acetate, washed with 10% citric acid, saturated sodium bicarbonate, and brine, dried over sodium sulfate and concentrated to give the acetate diene as a solid. A chilled solution of the foregoing acetate diene in 7:3 ethanol/THF (125 ml) was added dropwise to an ice cold suspension of sodium borohydride (2.5 g) in 80% ethanol (50 ml). The reaction was stirred in an ice bath for 8 hr then overnight allowing the ice bath to melt slowly. The reaction was quenched with 10% acetic acid and concentrated by rotovap until solid forms. The solid is filtered, washed with water and dried to constant weight to give 3,11-Dihydroxy-10,13-dimethyl-2,3,4,7,8,9,10,11,12,13,14,15,16,17-tetradecahydro-1H-cyclopenta[a]phenanthrene-17-carboxylic acid methyl ester as a white solid.

### EXAMPLE 5 [1005]

### (8S,9S,10R,11S,13S,14S,17S)-methyl 11-hydroxy-10,13-dimethyl-3-oxo-2,3,6,7,8,9,10,11,12,13,14,15,16,17-tetradecahydro-1H-cyclopenta[a]phenanthrene-17-carboxylate

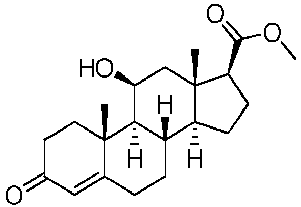

### Step 1

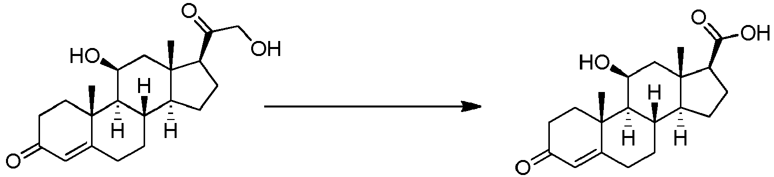

Corticosterone (1.0 g, 2.9 mmol) was dissolved in THF (8 ml) and methanol (3 ml) and treated with a hot solution of sodium metaperiodate (1.85 g, 8.8 mmol) in 7 ml water and stirred for 0.5 hr. The volatiles were removed by rotovap and the resulting suspension was filtered, washed with hot water, and dried under high vacuum to give 11-hydroxy-10,13-dimethyl-3-oxo-2,3,6,7,8,9,10,11,12,13,14,15,16,17-tetradecahydro-1H-cyclopenta[a]phenanthrene-17-carboxylic acid as a white solid (960 mg).

### Step 2

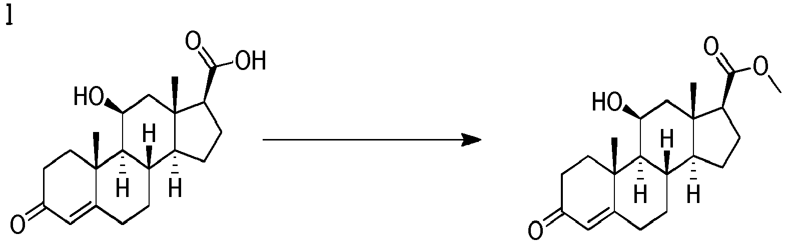

11-Hydroxy-10,13-dimethyl-3-oxo-2,3,6,7,8,9,10,11,12,13,14,15,16,17-tetradecahydro-1H-cyclopenta[a]phenanthrene-17-carboxylic acid (700 mg) was dissolved in toluene (20 ml) and methanol (2 ml) and treated dropwise with a solution of TMS-diazomethane (2.0 M in ether) until the yellow color retained. The mixture was concentrated by rotovap and purified by silica gel chromatography eluting with 30%- 50% ethyl acetate/hexanes to give 11-hydroxy-10,13-dimethyl-3-oxo-2,3,6,7,8,9,10,11,12,13,14,15,16,17-tetradecahydro-1H-cyclopenta[a]phenanthrene-17-carboxylic acid methyl ester as a white solid (718 mg). MS (ES-API) *m*/*z* (M+H⁺) 347.7.

### EXAMPLE 6 [1006]

### 1-((3S,8S,9S,10R,11S,13S,14S,17S)-3,11-dihydroxy-10,13-dimethyl-2,3,6,7,8,9,10,11,12,13,14,15,16,17-tetradecahydro-1H-cyclopenta[a]phenanthren-17-yl)ethanone

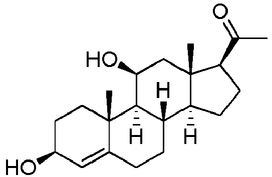

### Step 1

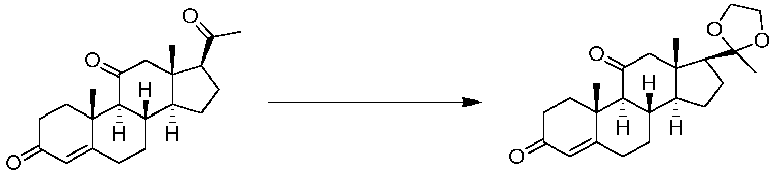

A solution of 17-acetyl-10,13-dimethyl-1,6,7,8,9,10,12,13,14,15,16,17-dodecahydro-2H-cyclopenta[a]phenanthrene-3,11-dione (500 mg, 1.5 mmol) in dichloromethane (10 ml) at-78 °C, under Ar, was treated with bistrimethoxysiloxyethane (900 µl, 3.6 mmol) and trimethylsilyl triflate (3 µl, 0.015 mmol). The reaction was placed in an ice bath, stirred for 2 hr and quenched by adding 3 drops of pyridine. The mixture was diluted with ethyl acetate, washed with 10% citric acid, saturated sodium bicarbonate and brine, dried over sodium sulfate, concentrated by rotovap, and purified by silica gel chromatography eluting with 20% - 50% ethyl acetate/hexanes to give 10,13-dimethyl-17-(2-methyl-[1,3]dioxolan-2-yl)-1,6,7,8,9,10,12,13,14,15,16,17-dodecahydro-2H-cyclopenta[a]phenanthrene-3,11-dione as a white solid (390 mg).

### Step 2

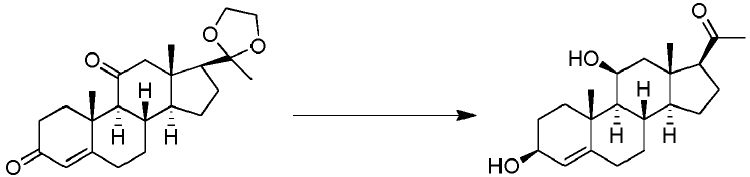

A solution of 10,13-dimethyl-17-(2-methyl-[1,3]dioxolan-2-yl)-1,6,7,8,9,10,12,13,14,15,16,17-dodecahydro-2H-cyclopenta[a]phenanthrene-3,11-dione (160 mg, 0.4 mmol) in THF (10 ml) was treated dropwise with a solution of lithium aluminum hydride (0.5 M in dimethoxyethane, 5 ml, 2.5 mmol) and stirred overnight. The reaction was quenched by dropwise addition of water until gas evolution ceased, treated with sodium sulfate, stirred until a white precipitate settles out, decanted and concentrated by rotovap to give the 11-hydroxy-allyl alcohol. The allyl alcohol was dissolved in 95% acetone/water (40 ml) and treated with pyridinium p-toluenesulfonate (30 mg) and stirred overnight. The reaction mixture was concentrated by rotovap until a precipitate formed, filtered, washed with water and dried under hard vacuum to give 10,13-dimethyl-17-(2-methyl-[1,3]dioxolan-2-yl)-2,3,4,7,8,9,10,11,12,13,14,15,16,17-tetradecahydro-1H-cyclopenta[a]phenanthrene-3,11-diol as a yellow solid (67 mg). ¹H NMR (300 MHz, DMSO-*d*₆) δ 0.62 - 0.77 (m, 2H), 0.73 (s, 3H), 0.77 - 0.91 (m, 1H), 0.92 - 1.27 (m, 4H), 1.22 (s,3H), 1.27 - 1.40 (m, 2H), 1.40 - 1.67 (m, 4H), 1.67 - 1.93 (m, 6H), 1.93 - 2.21 (m, 4H), 2.04 (s, 3H), 2.5 (m, 1-2H, under water), 3.84 - 3.95 (m, 1H), 4.11 - 4.24 (m, 2H), 4.53 (d, *J* = 5.7 Hz, 1H), 5.09 (br s, 1H) MS (ES-API) *m*/*z* (M-OH)⁺ 315.6.

### EXAMPLE 7 [1007]

### (8S,9S,10R,11S,13S,14S,17S)-17-acetyl-11-hydroxy-10,13-dimethyl-6,7,8,9,10,11,12,13,14,15,16,17-dodecahydro-1H-cyclopenta[a]phenanthren-3(2H)-one

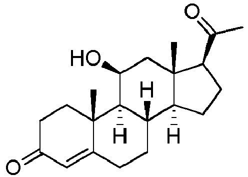

### Step 1

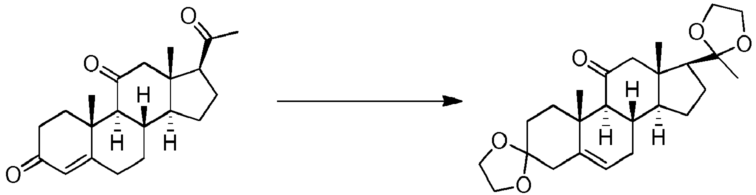

A mixture containing 17-acetyl-10,13-dimethyl-1,6,7,8,9,10,12,13,14,15,16,17-dodecahydro-2H-cyclopenta[a]phenanthrene-3,11-dione (4 g), toluenesulfonic acid monohydrate (250 mg), and ethylene glycol (10 ml) in benzene (40 ml) was refluxed with azeotropic removal of water for 8 hr. The mixture was diluted with ethyl acetate, washed with saturated sodium bicarbonate solution and brine, dried over sodium sulfate and concentrated by rotovap. Crystallization from ethyl acetate (40 ml) at -30°C gave 17-acetyl-10,13-dimethyl-1,6,7,8,9,10,12,13,14,15,16,17-dodecahydro-2H-cyclopenta[a]phenanthrene-3,11-dione-3,21-diethylene ketal as a pale yellow solid (3.0 g).

### Step 2

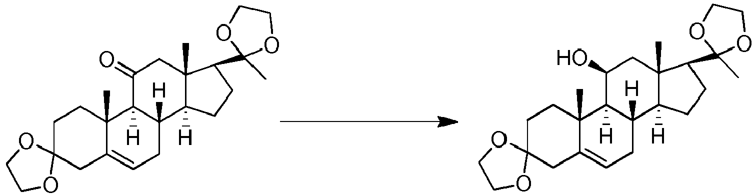

A solution of acetyl-10,13-dimethyl-1,6,7,8,9,10,12,13,14,15,16,17-dodecahydro-2H-cyclopenta[a]phenanthrene-3,11-dione-3, 21-diethylene ketal (1.0 g, 2.4 mmol) in THF was treated with a solution of lithium aluminum hydride (0.5 M in dimethoxyethane, 5.8 ml, 2.9 mmol) and stirred for 1 hours at RT. The reaction was quenched by dropwise addition of water until gas evolution ceased, treated with sodium sulfate, stirred until a white precipitate settles out, filtered and concentrated by rotovap to give an oil which was purified by silica gel chromatography eluting with 20% - 40% ethyl acetate/hexane. Acetyl-10,13-dimethyl-1,6,7,8,9,10,12,13,14,15,16,17-dodecahydro-2H-cyclopenta[a]phenanthrene-11-ol-3-one-3,21-diethylene ketal was obtained as a white solid (639 mg).

### Step 3

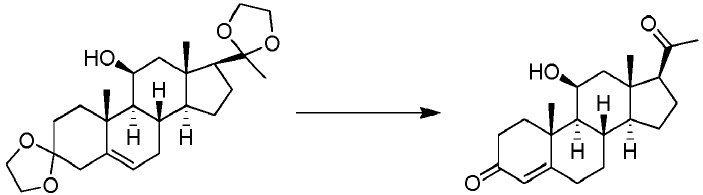

A solution of acetyl-10,13-dimethyl-1,6,7,8,9,10,12,13,14,15,16,17-dodecahydro-2H-cyclopenta[a]phenanthrene-11-ol-3-one- 3, 21-diethylene ketal (250 mg) in acetone (10 ml) and water (3 ml) was treated with concentrated sulfuric acid (0.1 ml)and stirred at RT for 4 hrs. The mixture was diluted with ethyl acetate and washed with saturated sodium carbonate solution and brine, dried over sodium sulfate and purified purified by silica gel chromatography eluting with 30% - 70% ethyl acetate/hexane. 17-Acetyl-11-hydroxy-10,13-dimethyl-1,2,6,7,8,9,10,11,12,13,14,15,16,17-tetradecahydro-cyclopenta[a]phenanthren-3-one was obtained as a white solid (201 mg).
MS (ES-API) *m*/*z* (M+H⁺) 331.7.

### EXAMPLE 8 [1058]

### (8S,9S,10R,11S,13S,14S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one

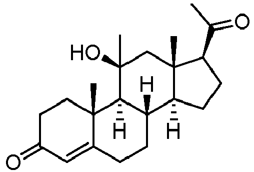

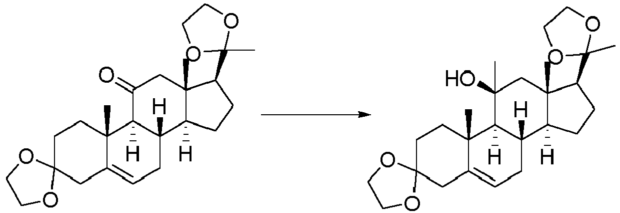

### Step 1

A solution of acetyl-10,13-dimethyl-1,6,7,8,9,10,12,13,14,15,16,17-dodecahydro-2H-cyclopenta[a]phenanthrene-3,11-dione-3,21-diethylene ketal (100mg, 0.24mmole) in THF (10ml) at 0°C was added MeLi (0.1ml, 0.31mmole) and allowed to stir for 1 hr at 0°C. Reaction mass was quenched with sat. NH₄Cl solution, extracted with EtOAc (3 X 50 ml). Organic layer was separated, dried (Na₂SO₄) and evaporated under reduced pressure and subjected to column chromatography on silica gel (100-200 mesh 1-2% MeOH:DCM) to afford the (8S,9S,10R,11S,13S,14S,17S)-10,11,13-trimethyl-17-(2-methyl-1,3-dioxolan-2-yl)-1,2,4,7,8,9,10,11,12,13,14,15,16,17-tetradecahydrospiro[cyclopenta[a]phenanthrene-3,2'-[1,3]dioxolan]-11-ol (90mg, 87%) as a white solid.
ESIMS: 433 (M⁺ + 1)

### Step 2

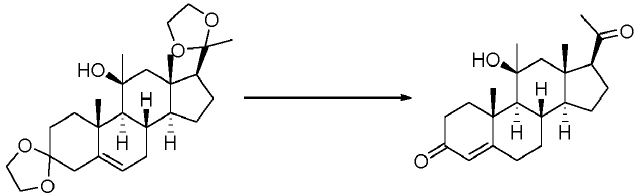

(8S,9S,10R,11S,13S,14S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one (40 mg, 56 %) was synthesized form (8S,9S,10R,11 S,13S,14S,17S)-10,11,13-trimethyl-17-(2-methyl-1,3-dioxolan-2-yl)-1,2,4,7,8,9,10,11,12,13,14,15,16,17-tetradecahydrospiro[cyclopenta[a]phenanthrene-3,2'-[1,3]dioxolan]-11-ol (90 mg, 0.2 mmol) using the same procedure as in example 7.
ESIMS: 345 (M⁺ + 1)

### EXAMPLE 9 [1008]

### 1-((3S,8S,9S,10S,11S,13S,14S,17S)-3,11-dihydroxy-10,13-dimethylhexadecahydro-1H-cyclopenta[a]phenanthren-17-yl)ethanone

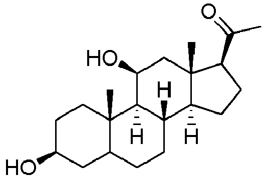

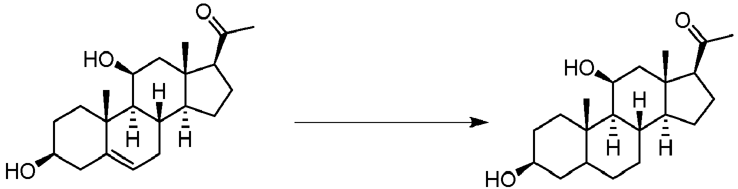

1-((3S,8S,9S,10R,11 S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one (100 mg, 0.299 mmol) and 5% Pd-C (20 mg) in ethanol (50 ml) was stirred under a balloon of hydrogen for 1 hrs. The mixture was filtered and purified by silica gel chromatography eluting with 30% - 50% ethyl acetate/hexane. 1-(3,11-Dihydroxy-10,13-dimethylhexadecahydro-cyclopenta[a]phenanthren-17-yl)-ethanone was obtained as a white solid (35 mg). ¹H NMR (300 MHz, DMSO-*d*₆) δ 0.60 (br d, *J* = 10.8 Hz, 1H), 0.70 (s, 3H), 0.8 - 1.9 (m, 23H), 0.94 (s, 3H), 1.90 - 2.18 (m, 3H), 2.04 (s, 3H), 2.5 (m, 1-2H, under DMSO), 3.34 (m, 1-2H, under water), 4.05 (br d, *J* = 3.0 Hz, 1H), 3.15 (br s, 1H), 4.42 (d, *J* = 4.8 Hz, 1H) MS (ES-API) *m*/*z* (M-OH)⁺ 317.7.
ESIMS: 335 (M⁺ + 1)

### EXAMPLE 10 [1026]

### (8S,9S,10S,11S,13S,14S,17S)-17-acetyl-11-hydroxy-10,13-dimethylhexadecahydro-3H-cyclopenta[a]phenanthren-3-one

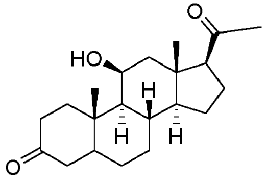

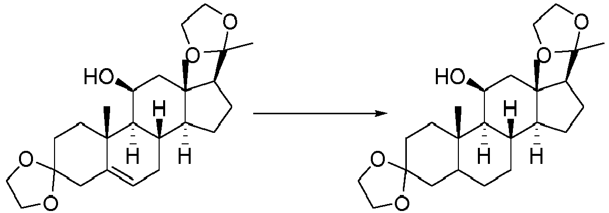

### Step 1

Acetyl-10,13-dimethyl-1,6,7,8,9,10,12,13,14,15,16,17-dodecahydro-2H-cyclopenta[a]phenanthrene-3,11-dione-3, 21-diethylene ketal (300mg, 0.71 mmol) was hydrogenated using same procedure as in example 8 to obtain (8S,9S,10S,11S,13S,14S,17S)-10,13-dimethyl-17-(2-methyl-1,3-dioxolan-2-yl)hexadecahydrospiro[cyclopenta[a]phenanthrene-3,2'-[1,3]dioxolan]-11-ol (250 mg, 83%) as light green solid.
ESIMS: 421 (M⁺ + 1)

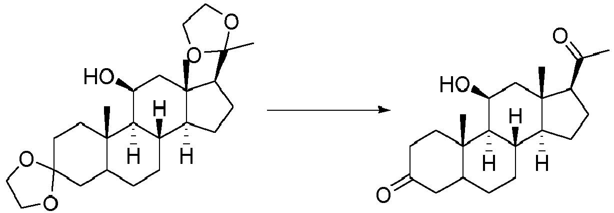

### Step 2

(8S,9S,10S,11S,13S,14S,17S)-17-acetyl-11-hydroxy-10,13-dimethylhexadecahydro-3H-cyclopenta[a]phenanthren-3-one (40 mg, 50%) was obtained from (8S,9S,10S,11S,13S,14S,17S)-10,13-dimethyl-17-(2-methyl-1,3-dioxolan-2-yl)hexadecahydrospiro[cyclopenta[a]phenanthrene-3,2'-[1,3]dioxolan]-11-ol (100 mg, 0.23 mmol) using the procedure same in example 8.
ESIMS: 333 (M⁺ + 1)

### EXAMPLE 11 [1032]

### (8S,9S,10R,11S,13S,14S,17S)-11-hydroxy-17-((R)-1-hydroxyethyl)-10,13-dimethyl-1,2,6,7,8,9,10,11,12,13,14,15,16,17-tetradecahydro-3H-cyclopenta[a]phenanthren-3-one

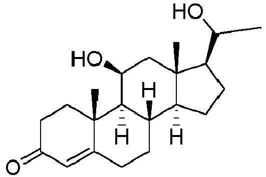

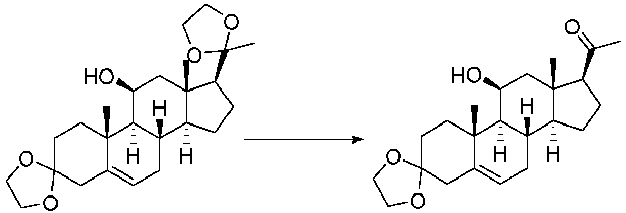

### Step 1

A solution of (8S,9S,10S,11S,13S,14S,17S)-10,13-dimethyl-17-(2-methyl-1,3-dioxolan-2-yl)hexadecahydrospiro[cyclopenta[a]phenanthrene-3,2'-[1,3]dioxolan]-11-ol (400mg, 0.95mmole) in acetone (20ml) at 0°C was added H₂SO₄ (0.4ml) in 4ml water and allowed to stir for 15min at 0°C. Reaction mass was quenched with sodium bicarbonate solution and extracted with EtOAc. Organic layer was separated; dried over Na₂SO₄ and evaporated under reduced pressure and subjected to column chromatography on silica gel (100-200 mesh, 10- 20% EtOAc:Hexane) to afforded the 1-((8S,9S,10R,11S,13S,14S,17S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one (230mg, 64%) as a white solid.
ESIMS: 375 (M⁺ + 1)

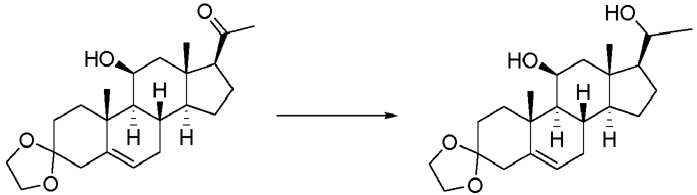

### Step 2

A solution of 1-((8S,9S,10R,11S,13S,14S,17S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one (50mg, 0.13mmole) in THF (10ml) at 0°C was added NaBH₄ (4mg, 0.013, mmol) and allowed to stir for 12hr at rt. Reaction mass was evaporated ,diluted with EtOAc and washed with sat.NH₄Cl solution. Organic layer was separated; dried over Na₂SO₄ and evaporated under reduced pressure. Crude was subjected to column chromatography on silica gel to column chromatography on silica gel (100-200 mesh, 10-20% EtOAc:Hexane) to afforded the (8S,9S,10R,11S,13S,14S,17S)-17-((R)-1-hydroxyethyl)-10,13-dimethyl-1,2,4,7,8,9,10,11,12,13,14,15,16,17-tetradecahydrospiro[cyclopenta[a]phenanthrene-3,2'-[1,3]dioxolan]-11-ol (12mg, 24%) as white solid.
ESIMS: 377 (M⁺ + 1)

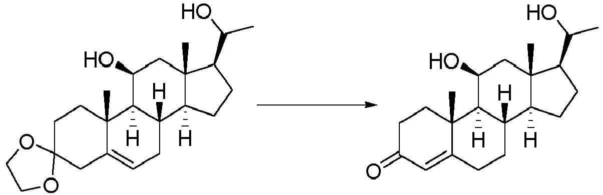

### Step 3

A solution of (8S,9S,10R,11S,13S,14S,17S)-17-((R)-1-hydroxyethyl)-10,13-dimethyl-1,2,4,7,8,9,10,11,12,13,14,15,16,17-tetradecahydrospiro[cyclopenta[a]phenanthrene-3,2'-[1,3]dioxolan]-11-ol (40mg, 0.10mmole) in acetone (7ml) at 0°C was added H₂SO₄ (0.1ml) in 1ml water and allowed to stir for 4 hrs at 0°C. Reaction mass was quenched with sodium bicarbonate solution, extracted with EtOAc. Organic layer was separated, dried (Na₂SO₄), evaporated under reduced pressure. Crude was subjected to column chromatography on silica gel silica gel (100-200 mesh, 1-2% MeOH:DCM) to obtain (8S,9S,10R,11S,13S,14S,17S)-11-hydroxy-17-((R)-1-hydroxyethyl)-10,13-dimethyl-1,2,6,7,8,9,10,11,12,13,14,15,16,17-tetradecahydro-3H-cyclopenta[a]phenanthren-3-one (15mg , 42%) as a white solid.
ESIMS: 333 (M⁺ + 1)

### EXAMPLE 12 [1036]

### (8S,9S,10S,11S,13S,14S,17S)-17-((S)-1-hydroxyethyl)-10,13-dimethylhexadecahydro-1H-cyclopenta[a]phenanthrene-3,11-diol

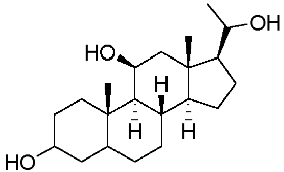

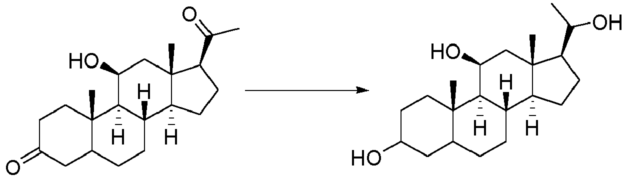

(8S,9S,10S,11S,13S,14S,17S)-17-((S)-1-hydroxyethyl)-10,13-dimethylhexa-decahydro-1H-cyclopenta[a]phenanthrene-3,11-diol (27 mg, 60%) was obtained from (8S,9S,10S,11S,13S,14S,17S)-17-acetyl-11-hydroxy-10,13-dimethylhexadecahydro-3H-cyclopenta[a]phenanthren-3-one (40 mg, 0.12 mmol) using same procedure as in exaplme 3.
ESIMS: 337 (M⁺ + 1)

### EXAMPLE 13 [1009]

### (3S,8S,9S,10R,11S,13R,14S,17S)-17-ethyl-10,13-dimethyl-2,3,4,7,8,9,10,11,12,13,14,15,16,17-tetradecahydro-1H-cyclopenta[a]phenanthrene-3,11-diol

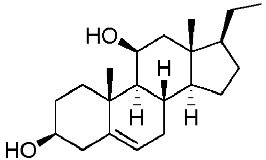

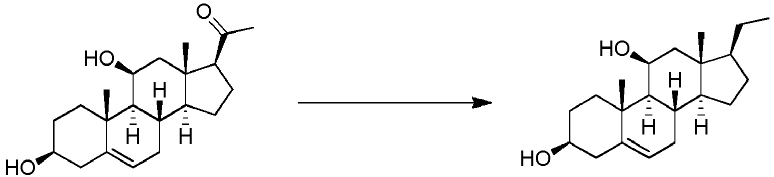

1-(3,11-dihydroxy-10,13-dimethyl-2,3,4,7,8,9,10,11,12,13,14,15,16,17-tetradecahydro-1H-cyclopenta[a]phenanthren-17-yl)-ethanone (0.2g, 0.60 mmol) was taken in ethylene glycol (5 mL). TO this, pyridine (0.12 ml, 0.072 mmol) was added followed by Hydrazine hydrate (0.044 g, 3.0 mmol). The mixture was stirred at 60°C for 2 hrs. TLC showed the consumption of starting material. NaOH (0.05g, 1.25 mmol) was added and mixture was stirred at 200oC for 1 hrs. The reaction mixture was cooled to room temperature, diluted with water and extracted with ethyl acetae (2 X 100 mL). The combined organuic extracts were dried over Na2SO4, concentrated and crude was purified by column chromatography (1% MeOH/DCM) to obtain compound **[11]** as off white solid (0.071 g, 37%)
ESIMS: 319 (M⁺ + 1)

### EXAMPLE 14 [1049]

### (10R,11S,13S,17S)-11-hydroxy-17-(2-hydroxypropan-2-yl)-10,13-dimethyl-1,2,6,7,8,9,10,11,12,13,14,15,16,17-tetradecahydro-3H-cyclopenta[a]phenanthren-3-one

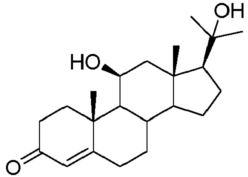

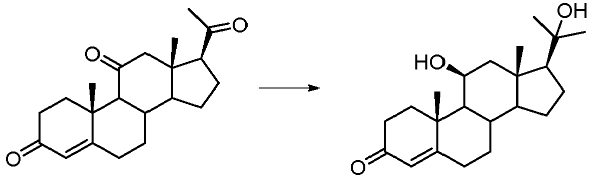

To a solution of (10R,13S,17S)-17-acetyl-10,13-dimethyl-1,6,7,8,9,10,12,13,14,15,16,17-dodecahydro-3H-cyclopenta[a]phenanthrene-3,11(2H)-dione (35mg, 0.106mmol) in a 9:1 mixture of absolute ethanol and THF (5ml) at 0°C to this was added previously cooled (at 0°C) NaBH4 (71mg, 1.87mmol) solution in a solvent 9:1 mixture of absolute ethanol and water. The reaction was stirred over night. The reaction was quenched using saturated solution of NH4CI and extracted with ethyl acetate. Ethyl acetate layer was washed with brine, dried over anhydrous sodium sulphate, and organic solvent removed under vacuum to get the product, which was triturate using DCM-n-pentane mixture to get pure product (13mg, 35%).

### EXAMPLE 15 [1010]

### (8S,9S,10R,11S,13S,14S,17R)-17-((R)-1,2-dihydroxyethyl)-11,17-dihydroxy-10,13-dimethyl-6,7,8,9,10,11,12,13,14,15,16,17-dodecahydro-1H-cyclopenta[a]phenanthren-3(2H)-one

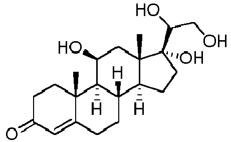

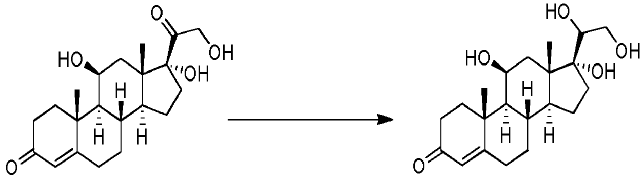

A solution of hydrocortisone (400 mg) in THF (20 ml) and water (20 ml) was treated with sodium borohydride (40 mg) and stirred for 1 hr. The mixture was quenched with acetone, diluted with ethyl acetate, washed with brine, dried over sodium sulfate and concentrated by rotovap to give 17-(1,2-dihydroxy-ethyl)-11,17-dihydroxy-10,13-dimethyl-1,2,6,7,8,9,10,11,12,13,14,15,16,17-tetradecahydro-cyclopenta[a]phenanthren-3-one as a white solid (333 mg).

### EXAMPLE 16 [1011]

### (8S,9S,10R,11S,13S,14S)-11-hydroxy-10,13-dimethyl-7,8,9,10,11,12,13,14,15,16-decahydro-1H-cyclopenta[a]phenanthrene-3,17(2H,6H)-dione

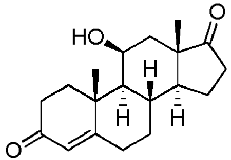

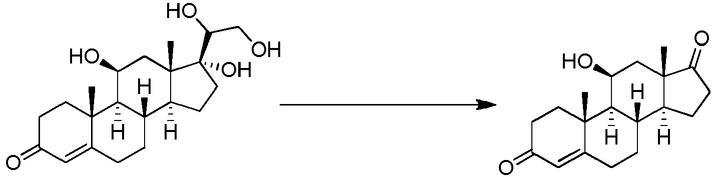

A solution of 17-(1,2-dihydroxy-ethyl)-11,17-dihydroxy-10,13-dimethyl-1,2,6,7,8,9,10,11,12,13,14,15,16,17-tetradecahydro-cyclopenta[a]phenanthren-3-one (210 mg, 0.6 mmol) in THF (4 ml) and methanol (2 ml) was treated with a hot solution of sodium metaperiodate (383 mg, 1.8 mmol) in water and stirred for 1 hr at RT. The mixture was diluted with water (10 ml) and concentrated by rotovap until a precipitate forms which is filtered, washed with water and dried under hard vacuum to give 11-hydroxy-10,13-dimethyl-1,6,7,8,9,10,11,12,13,14,15,16-dodecahydro-2H-cyclopenta[a]phenanthrene-3,17-dione as a white solid (38 mg).

### EXAMPLE 17 [1028]

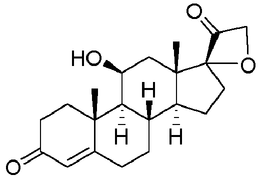

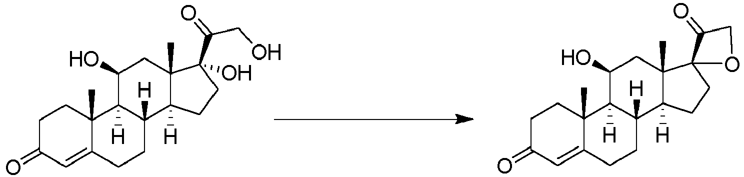

A solution of (8S,9S,10R,11S,13S,14S,17R)-11,17-dihydroxy-17-(2-hydroxyacetyl)-10,13-dimethyl-1,2,6,7,8,9,10,11,12,13,14,15,16,17-tetradecahydro-3H-cyclopenta[a]phenanthren-3-one (500 mg, 1.38 mmol mmol) in dry pyridine (10 ml) was cooled to 0°C and MsCI (0.212 mL, 1.76 mmol) was slowly added. After stirring at 0°C for 2 hrs, the solution was poured into 1N HCI (100 mL) followed by extraction with EtOAc. After drying (Na₂SO₄) the solvent was evaporated and the residue was purified by flash chromatography (2% MeOH/DCM) to yield compound **[14]** as a pale yellow foam. The compound was taken in dry THF (100 mL) To this potassium tert-butoxide (0.160 g, 1.42 mmol) was added and mixture was stirred at room temperature for 30 min. The reaction mixture was poured in to 1N HCI (100 mL), extracted with ethyl acetate (3 X 100 mL). The combined organic extract was seprtaed, dried (Na₂SO₄) and concentrated under reduced pressure to obtain (8S,9S,10R,11S,13S,14S,17R)-11-hydroxy-10,13-dimethyl-1,6,7,8,9,10,11,12,13,14,15,16-dodecahydrospiro[cyclopenta[a]phenanthrene-17,2'-oxetane]-3,3'(2H)-dione as white solid (0.350g, 95%).
ESIMS: 345 (M⁺ + 1)

### EXAMPLE 18 [1039]

### (8S,9S,10S,11S,13S,14S,17S)-17-((S)-1-hydroxyethyl)-10,13-dimethylhexadecahydrospiro[cyclopenta[a]phenanthrene-3,2'-[1,3]dioxolan]-11-ol

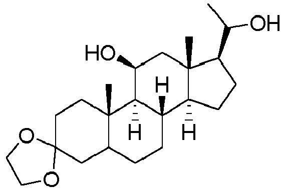

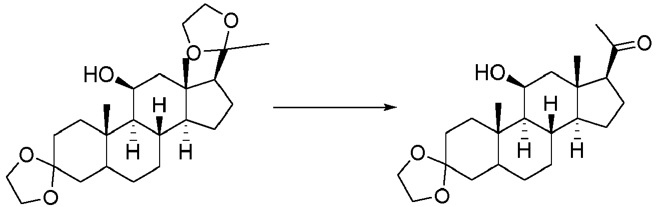

### Step 1

A solution of (8S,9S,10S,11S,13S,14S,17S)-10,13-dimethyl-17-(2-methyl-1,3-dioxolan-2-yl)hexadecahydrospiro[cyclopenta[a]phenanthrene-3,2'-[1,3]dioxolan]-11-ol (100mg, 0.23mmole) in acetone (10ml) at 0°C was added H₂SO₄ (0.1ml,) in 1 ml water and allowed to stir for 30min. at 0°C. Reaction mass was quenched with sodium bicarbonate solution and extracted with EtOAc (3 X 100 ml). Organic layer was separated, dried (Na₂SO₄) and evaporated under reduced pressure and subjected to column chromatography on silica gel (100-200 mesh, 10-20% EtOAc:Hexane) to afford the 1-((8S,9S,10S,11S,13S,14S,17S)-11-hydroxy-10,13-dimethylhexadecahydrospiro[cyclopenta[a]phenanthrene-3,2'-[1,3]dioxolan]-17-yl)ethan-1-one (40mg ,44 %) as a white solid.
ESIMS: 377(M⁺ + 1)

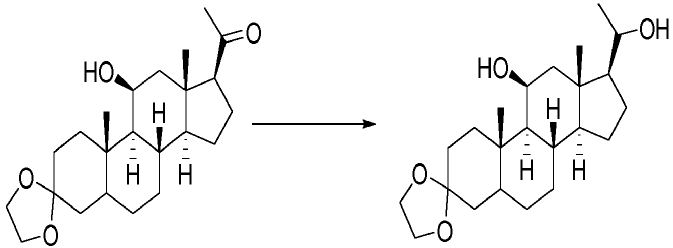

### Step 2

A solution of 1-((8S,9S,10S,11S,13S,14S,17S)-11-hydroxy-10,13-dimethylhexadecahydrospiro[cyclopenta[a]phenanthrene-3,2'-[1,3]dioxolan]-17-yl)ethan-1-one (44mg, 0.11mmole) in THF (10ml) at 0°C was added NaBH₄ (9mg, 0.23mmole) and allowed to stir for 12hr at rt. Reaction mass was evaporated under reduced pressure. Crude was diluted with EtOAc, washed with sat.NH₄Cl solution. Organic layer was separated; dried over Na₂SO₄ and evaporated under reduced pressure and subjected to column chromatography on silica gel (100-200 mesh 10-30% EtOAc:Hexane) to afford the (8S,9S,10S,11S,13S,14S,17S)-17-((S)-1-hydroxyethyl)-10,13-dimethylhexadecahydrospiro[cyclopenta[a]phenanthrene-3,2'-[1,3]dioxolan]-11-ol (10mg , 22%) as white solid.
ESIMS: 378(M⁺ + 1)

### EXAMPLE 19 [1012]

### (8S,9S,10R,11S,13S,14S,17S)-11-hydroxy-10,13-dimethyl-17-(3-methyl-1,2,4-oxadiazol-5-yl)-6,7,8,9,10,11,12,13,14,15,16,17-dodecahydro-1H-cyclopenta[a]phenanthren-3(2H)-one

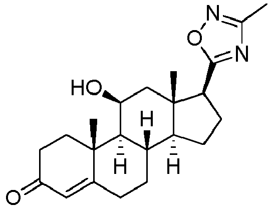

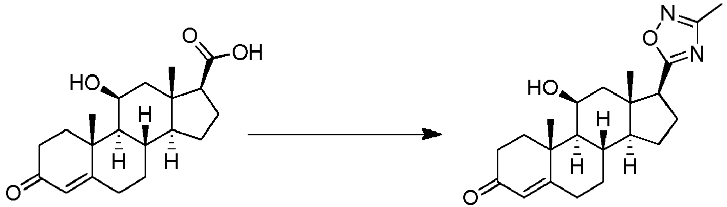

A solution of 11-hydroxy-10,13-dimethyl-3-oxo-2,3,6,7,8,9,10,11,12,13,14,15,16,17-tetradecahydro-1H-cyclopenta[a]phenanthrene-17-carboxylic acid (150 mg, 0.45 mmol) in DMF (3 ml) was treated with carbonyldiimidazole (81 mg, 0.5 mmol) and stirred at RT under Ar for 0.5 hr. Amidoxime (40 mg, 0.5 mmol) is added and the reaction is stirred for 4 hr. Carbonyldiimidazole (81 mg, 0.5 mmol) is added and the reaction is stirred for 6 hr at 115 °C for 6 hr under Ar. The mixture was diluted with ethyl acetate, washed with 10% citric acid, saturated sodium bicarbonate and brine, dried over sodium sulfate, concentrated by rotovap, and purified by silica gel chromatography eluting with 50% - 100% ethyl acetate/hexanes to give 11-hydroxy-10,13-dimethyl-17-(3-methyl-[1,2,4]oxadiazol-5-yl)-1,2,6,7,8,9,10,11,12,13,14,15,16,17-tetradecahydro-cyclopenta[a]phenanthren-3-one as a solid (35 mg). ¹H NMR (400 MHz, DMSO-*d*₆) δ 0.74 (s, 3H), 0.949 (dd, *J* = 11.6 and 3.6 Hz, 1H), 1.00 (qd, *J* = 14 and 3.6 Hz, 1H) 1.33- 1.14 (m, 2H) 1.36 (s, 3H), 1.36 - 1.42 (m, 1H), 1.48 (dd, *J* = 13.2 and 2.8 Hz, 1H), 1.72 - 1.85 (m, 2H), 1.85 - 1.99 (m, 2H), 1.99 - 2.14 (m, 2H), 2.14 - 2.27 (m, 3H), 2.31 (s, 3H), 2.34- 2.47 (m, 2H), 2.89 (t, *J* = 9.2 Hz, 1H), 4.22 (t, *J* = 3.2 Hz, 1H), 4.34 (d, *J* = 3.2 Hz, 1H), 5.57 (s, 1H) MS (ESI) *m*/*z* (M+H⁺) 371.

### EXAMPLE 20 [1013]

### Trimethylammonium (3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl sulfate

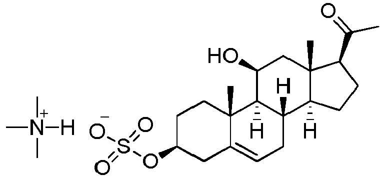

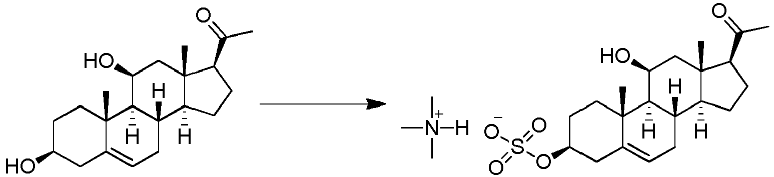

A solution of 1-(3,11-dihydroxy-10,13-dimethyl-2,3,4,7,8,9,10,11,12,13,14,15,16,17-tetradecahydro-1H-cyclopenta[a]phenanthren-17-yl)-ethanone (44 mg, 0.13 mmol) in toluene (2 ml) and DMF (0.6 ml) was treated with sulfur trioxide trimethylamine complex (18 mg, 0.14 mmol) and stirred at 40 °C overnight. Sulfur trioxide trimethylamine complex (5 mg, 0.04 mmol) was added and stirring at 40 °C was continued for 24 hr. Two drops of hexanes were added and the mixture was fitered, washed with toluene and hexanes, dried under high vacuum to give sulfuric acid mono-(17-acetyl-11-hydroxy-10,13-dimethyl-2,3,4,7,8,9,10,11,12,13,14,15,16,17-tetradecahydro-1H-cyclopenta[a]phenanthren-3-yl) ester trimethylamine salt as a white solid (25 mg). MS (ESI) *m*/*z* (M+H⁺) 413.27

### EXAMPLE 21 [1038]

### 1-((3S,10R,11S,13S,17S)-11-hydroxy-3-methoxy-10,13-dimethyl-2,3,4,7,8,9,10,11,12,13,14,15,16,17-tetradecahydro-1H-cyclopenta[a]phenanthren-17-yl)ethan-1-one

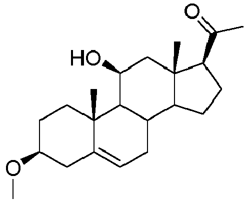

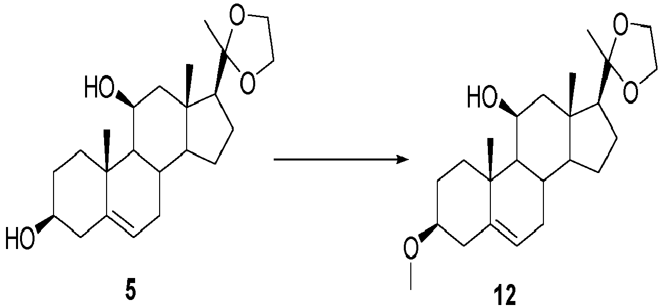

### Step 1

(3S,10R,11 S,13S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol (70 mg, 0.186 mmol) was taken in THF (10 ml) and cooled to 0oC. NaH (40 mg, 0.93 mmol) was added and mixture was stirred at room temperature for 2 hrs under nitrogen atmosphere. Mel (0.014 mL, 0.20 mmol) was added and stirring continued for 24 hrs. The reaction mixture was quenched with aq. NH₄Cl, extracted with ethyl acetate (3 X 50 mL). The combine organic extract was dried over Na₂SO₄, concentrated and crystallized by MeOH to obtain (3S,10R,11S,13S,17S)-3-methoxy-10,13-dimethyl-17-(2-methyl-1,3-dioxolan-2-yl)-2,3,4,7,8,9,10,11,12,13,14,15,16,17-tetradecahydro-1H-cyclopenta[a]phenanthren-11-ol (50mg, 71%) as white crystalline solid.
ESIMS: 391 (M⁺ + 1)

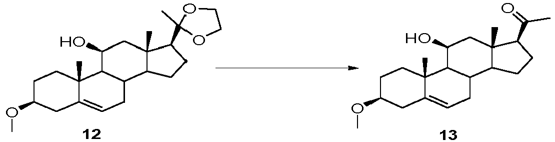

### Step 2

1-((3S,10R,11S,13S,17S)-11-hydroxy-3-methoxy-10,13-dimethyl-2,3,4,7,8,9,10,11,12,13,14,15,16,17-tetradecahydro-1H-cyclopenta[a]phenanthren-17-yl)ethan-1-one (15 mg, 43% yield) was obtained by same procedure as for 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one.
ESIMS: 328 (M⁺ - 18)

### EXAMPLE 22 [1059]

### 2-amino-N-((1S)-1-((3S,10S,11S,13S,17S)-3,11-dihydroxy-10,13-dimethylhexadecahydro-1H-cyclopenta[a]phenanthren-17-yl)ethyl)acetamide hydrochloride

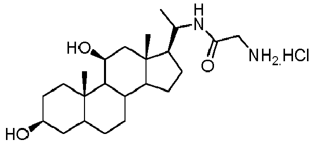

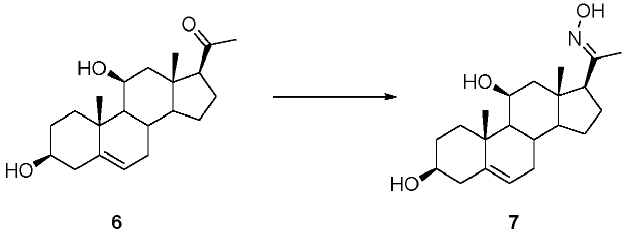

### Step 1

Compound **[6]** (0.2g, 0.60 mmol) was taken in MeOH (50 mL). TO this, pyridine (0.12 ml, 0.072 mmol) was added followed by Hydroxylamine. hydrochloride (0.044 g, 0.086 mmol). The mixture was stirred at room temperature for 2 hrs. The solvent was evaporated and crude was taken in water (50 ml), extracted with ethyl acetate (3 X 100 ml). The organic layer was separated, dried over Na₂SO₄, filtered and concentrated to obtain compound **[7]** as off white solid (0.2 g, 95%)
ESIMS: 348 (M⁺ + 1)

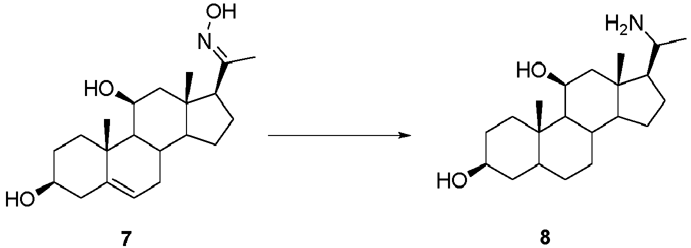

### Step 2

Compound [7] (0.2 g. 0.58 mmol) was taken in MeOH (100ml). Pd/C (0.020 g) was added and reaction was stirred at room temperature under H2 atm (60 psi, Parr shaker). After completion, The solvent was filtered, concentrated to obtain compound **[8]** as light brown solid (0.180g, 99%).
ESIMS: 334 (M⁺ + 1)

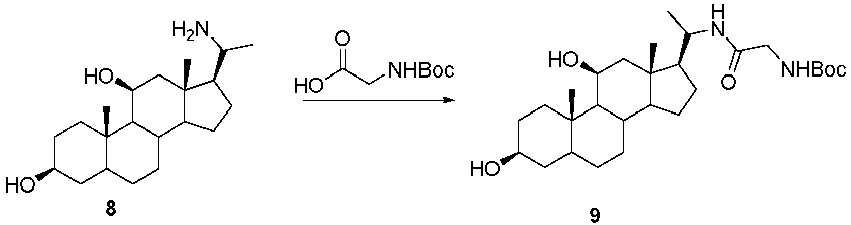

### Step 3

Acid (0.20g, 1.14 mmol) was taken in DMF (2 ml). To this EDC.HCl (0.327g, 0.45 g) was added. The mixture was stirred at room temperature for 30 min. Compound **[8]** (0.10 g, 0.30 mmol) and N-methyl morpholine (0.376 ml, 1.14 mmol) was added and mixture was stirred at room temperature for 24 hrs. The reaction was quenched with ice cold water. Reaction mixture was extracted with ethyl acetate (3 X 100ml), the organic layer was seprated, dried and concentrated to obtain crude. Compound **[9]** was obtained by column purifications (silica gel, 2-5 % MeOH/DCM as off white solid (0.190 g, 66%).
ESIMS: 477 (M⁺ + 1)

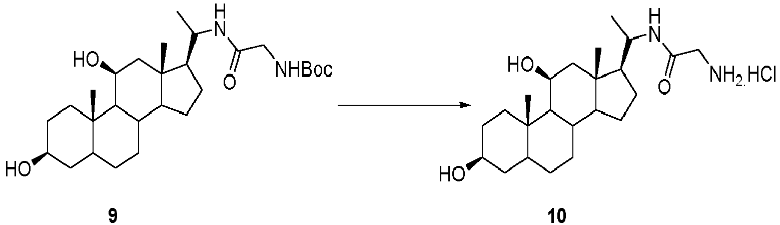

### Step 4

Compound **[9]** (0.100g, 0.21 mmol) was taken in DCM (10 ml) and cooled to 0oC. Dioxane HCI (4 N) (2 ml) was added to it and mixture was stirred at same temperature for 2 hrs. After completion, the solvent was evaporated, and crude was purified by ether, pentabe washing to obtain compound **[10]** as white solid (0.075g, 96%)
ESIMS: 377 (M⁺ + 1)

### EXAMPLE 23 [1030]

### (8S,9S,10R,11S,13S,14S,17S)-11-hydroxy-N,N,10,13-tetramethyl-3-oxo-2,3,6,7,8,9,10,11,12,13,14,15,16,17-tetradecahydro-1H-cyclopenta[a]phenanthrene-17-carboxamide

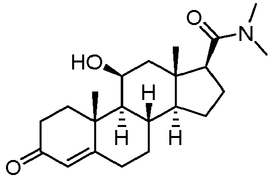

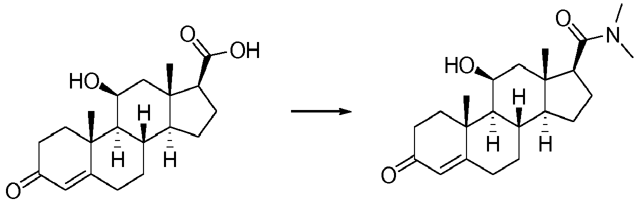

A solution of 11-hydroxy-10,13-dimethyl-3-oxo-2,3,6,7,8,9,10,11,12,13,14,15,16,17-tetradecahydro-1H-cyclopenta[a]phenanthrene-17-carboxylic acid (180 mg, 0.54 mmol) in DMF (3 ml). EDC.HCl (156 mg, 0.812 mmol) was added and mixture was stirred at room temperature for 30 min. Amine (0.81 mmol) was added followed by N-methyl morpholine (0.12 ml, 1.08 mmol) and stirring was continued for 24 hrs at same temperature. The reaction mixture was quenched with brine (50 ml), extracted with ethyl acetate (3 X 100 ml). The combined organic extract was dried (Na₂SO₄) concentrated to obtain crude which was purified by column chromatography (silica gel 100-200 mesh) to obtain compound **(8S,9S,10R,11S,13S,14S,17S)-11-hydroxy-*N,N*,10,13-tetramethyl-3-oxo-2,3,6,7,8,9,10,11,12,13,14,15,16,17-tetradecahydro-*1H-*cyclopenta[a]phenanthrene-17-carboxamide** (60-70% yield) as off white solid.
In a similar manner, the compounds 1057 and 1060 may be prepared by suitably varying the substituents.

### EXAMPLE 24 [1033]

### (8S,9S,10S,11S,13S,14S,17S)-11-hydroxy-N,N-10,13-tetramethyl-3-oxohexadecahydro-1H-cyclopenta[a]phenanthrene-17-carbaxamide

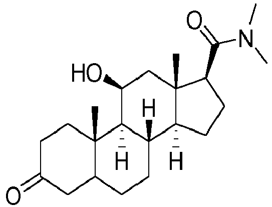

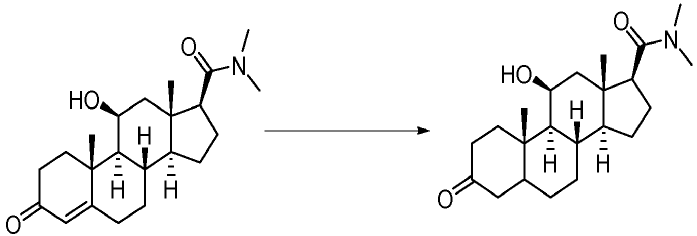

(8S,9S,10R,11S,13S,14S,17S)- 11-hydroxy-*N,N*,10,13-tetramethyl-3-oxo-2,3,6,7,8,9,10,11,12,13,14,15,16,17-tetradecahydro-*1H*-cyclopenta[a]phenanthrene-17-carboxamide (30 mg, 0.083 mmol) was taken in ethyl acetate: Methanol (9:1). Pd/C (3 mg, 10%) was added to it and mixture was stirred under H₂ atmosphere for 10 min. The reaction mixture was filtered, concentrated to obtain (8S,9S,10S,11S,13S,14S,17S)-11-hydroxy-*N,N*-10,13-tetramethyl-3-oxohexadecahydro-*1H-*cyclopenta[a]phenanthrene-17-carbaxamide (26 mg, 87% yield) as white solid.
ESIMS: 362 (M⁺ + 1)

### EXAMPLE 25 [1046]

### (3S,8S,9S,10R,11S,13S,14S,17S)-3,11-dihydroxy-N,N,10,13-tetramethyl-2,3,6,7,8,9,10,11,12,13,14,15,16,17-tetradecahydro-1H-cyclopenta[a]phenanthrene-17-carboxamide

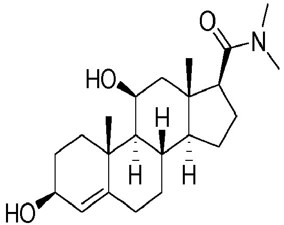

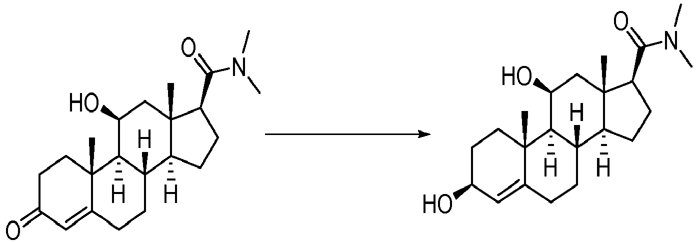

(8S,9S,10R,11S,13S,14S,17S)-11-hydroxy-*N,N*,10,13-tetramethyl-3-oxo-2,3,6,7,8,9,10,11,12,13,14,15,16,17-tetradecahydro-*1H*-cyclopenta[a]phenanthrene-17-carboxamide (20 mg, 0.055 mmol) was taken in ethanol (10 ml). NaBH4 (10 mg, 0.27 mmol) was added and mixture was stirred at room temperature for 2 hrs. The reaction was quenched with ice cold water, extracted with ethyl acetate (2 X 25 ml). The organic extract was seperated, dried (Na2SO4) and concentrated to obtain crude which was purified with ether, pentane washing to obtain (3S,8S,9S,10R,11S,13S,14S,17S)-3,11-dihydroxy-*N,N*,10,13-tetramethyl-2,3,6,7,8,9,10,11,12,13,14,15,16,17-tetradecahydro-*1H*-cyclopenta[a]phenanthrene-17-carboxamide (15 mg, 75%) as off white solid.
ESIMS: 362 (M⁺ + 1)

### EXAMPLE 26 [1043]

### (3S,8S,9S,10R,11S,13S,14S,17S)-3,11-dihydroxy-N,N,10,13-tetramethyl-2,3,4,7,8,9,10,11,12,13,14,15,16,17-tetradecahydro-1H-cyclopenta[a]phenanthrene-17-carboxamide

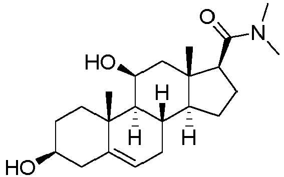

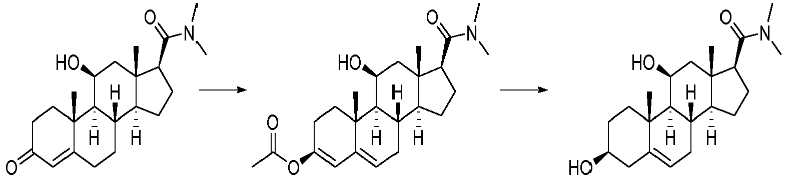

A solution of 70% perchloric acid (10 µl) in ethyl acetate (10 ml) is added to a solution of acetic anhydride (4.8 mL) in ethyl acetate (30 ml) and the final volume is brought up to 50 ml. 25ml of this solution was used in the following step.

To (8S,9S,10R,11S,13S,14S,17S)-11-hydroxy-N,N,10,13-tetramethyl-3-oxo-2,3,6,7,8,9,10,11,12,13,14,15,16,17-tetradecahydro-1H-cyclopenta[a]phenanthrene-17-carboxamide (0.1g, 0.278mmol) was added the above solution and the reaction stirred at RT for 3.5h. The reaction was quenched with saturated sodium bicarbonate solution and extracted with ethyl acetate. Ethyl acetate layer was concentrated to get oil. The oil was taken in methanol (1ml), followed by addition of pyridine (0.5ml). The reaction was then concentrated to get a paste. The paste was taken in ethyl acetate, washed with citric acid solution, then with brine, dried over anhydrous sodium sulphate and removed under vacuum to get crude product. The product was purified over silica column using 0-1% methanol and DCM. 3-acetylated compound (55mg) and 3,11-diacetylated (26mg) was obtained.

A solution of 3-acetylated compound (0.055g) from above step in absolute ethanol and THF (7:3) at 0°C was added to a stirred solution of sodium borohydride (0.1g) in absolute ethanol and water (7:3) at 0°C. The reaction was gradually allowed to attend room temperature. The reaction was concentrated under vacuum and taken in ethyl acetate and washed with saturated solution of NH4CI, dried and concentrated to get crude product. The compound was purified on a silica column using 0-2% methanol and DCM (5.5mg 5%). ESIMS: 362 (M⁺ + 1)

### EXAMPLE 27 [1052]

### (3S,8S,9S,10R,11S,13S,14S,17S)- 17-((E)-1-hydrazonoethy)-10,13-dimethyl- 2,3,4, 7,8,9,10,11,12,13,14,15,16,17-tetradecahydro-1H-cyclopenta[a]phenanthrene-3,11-diol

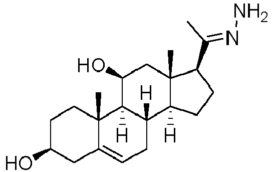

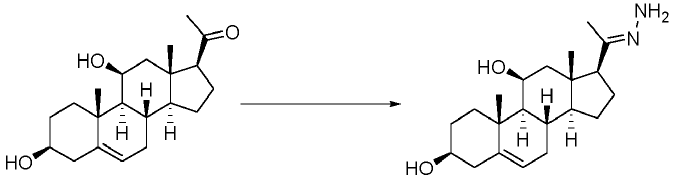

1-(3,11-dihydroxy-10,13-dimethyl-2,3,4,7,8,9,10,11,12,13,14,15,16,17-tetradecahydro-1H-cyclopenta[a]phenanthren-17-yl)-ethanone (50 mg, 0.15 mmol) was taken in ethanol (50 ml). Hydrazine (1M THF solution, 2 ml) was added and mixture was heated at 50oC for 24 hrs. The solvent was evaporated and crude was purified by ether washing to obtain (3S,8S,9S,10R,11S,13S,14S,17S)- 17-((E)-1-hydrazonoethy)-10,13-dimethyl-2,3,4,7,8,9,10,11,12,13,14,15,16,17-tetradecahydro-1*H*-cyclopenta[a]phenanthrene-3,11-diol as white solid (30 mg, 60%).
ESIMS: 347 (M⁺ + 1)

### EXAMPLE 28 [1051]

### (10R,11S,13S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-11-d-3,11-diol

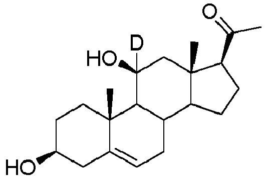

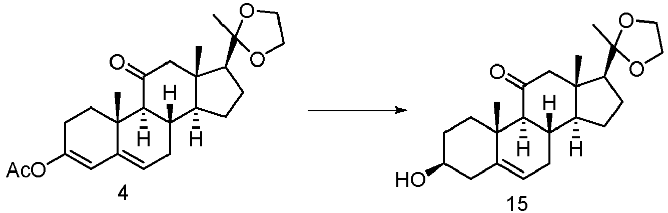

### Step 1

To a stirred solution of (10*R*,13*S*,17*S*)-10,13-dimethyl-17-(2-methyl-1,3-dioxolan-2-yl)-11-oxo-2,7,8,9,10,11,12,13,14,15,16,17-dodecahydro-1*H*-cyclopenta[*a*]-phenanthren-3-yl acetate (200mg, 0.48 mmol) in EtOH (15ml) at 0°C was added NaBH₄ (32mg, 0.86 mmol) and allowed to stir for 2 hr at room temperature. After completion, the reaction mass was evaporated under reduced pressure. Crude was diluted with EtOAc (100 ml) and washed with sat. NH₄Cl solution. Organic layer was separated, dried over Na₂SO₄ and evaporated under reduced pressure. Crude was purified by column chromatography on silica gel (100-200 mesh, 20-30% EtOAc/Cyclohexane) to afforded the (10*R*,13*S*,17*S*)-10,13-dimethyl-17-(2-methyl-1,3-dioxolan-2-yl)-1,2,3,4,7,8,9,10,12,13,14,15,16,17-tetradecahydro-11*H*-cyclopenta[*a*]-phenanthren-11-one (60mg , 32%) as off-white solid.
ESIMS: 375 (M⁺ + 1)

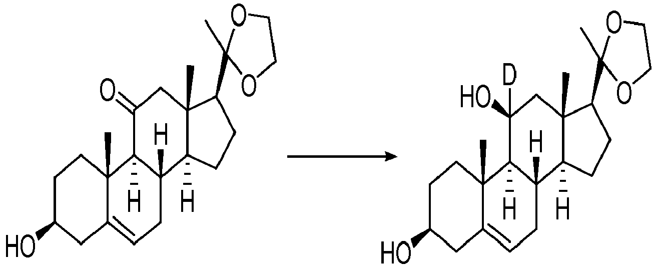

### Step 2

To a stirred solution of (10*R*,13*S*,17*S*)-10,13-dimethyl-17-(2-methyl-1,3-dioxolan-2-yl)-1,2,3,4,7,8,9,10,12,13,14,15,16,17-tetracahydro-11*H*-cyclopenta[a]-phenanthren-11-one (50mg, 0.13 mmol) in EtOH (10ml) at 0°C was added NaBD₄ (0.011g, 0.26 mmol) and allowed to stir for 12 hrs at room temperature. Completion of reaction was monitored by checking TLC. Reaction mass was evaporated under reduced pressure. Crude was diluted with EtOAc and washed with sat. NH₄Cl solution. Combined organic layer was separated, dried over Na₂SO₄ and evaporated under reduced pressure. Crude was by column chromatography on silica gel (100-200 mesh, 2-3% MeOH/DCM) to obtain (10*R*,11*S*,13*S*,17*S*)-10,13-dimethyl-17-(2-methyl-1,3-dioxolan-2-yl)-2,3,4,7,8,9,10,11,12,13,14,15,16,17-tetradecahydro-1*H-*cyclopenta[a]phenanthrene-11-*d*-3,11-diol as desired product.(30mg ,60%) as off-white solid.
ESIMS: 378(M⁺ + 1)

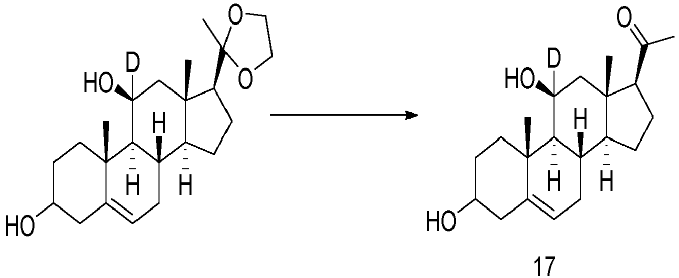

### Step 3

1-((10*R*,11S,13S,17S)-3,11-dihydro-10,13-dimethyl-2,3,4,7,8,9,10,11,12,13,14,15,16,17-tetradecahydro-1*H-*cyclopenta[a]phenanthren-17-yl-*d*)ethan-1-one (16 mg, 60% yield) was obtained from (10*R*,11*S*,13*S*,17*S*)-10,13-dimethyl-17-(2-methyl-1,3-dioxolan-2-yl)-2,3,4,7,8,9,10,11,12,13,14,15,16,17-tetradecahydro-1*H-*cyclopenta[a]phenanthrene-11-*d*-3,11-diol (30 mg, 0.082 mmol) using the procedure same in example 2.
ESIMS: 334(M⁺ + 1)

### EXAMPLE 29 [1045]

### 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3-d)ethan-1-one

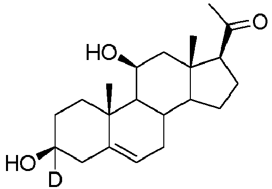

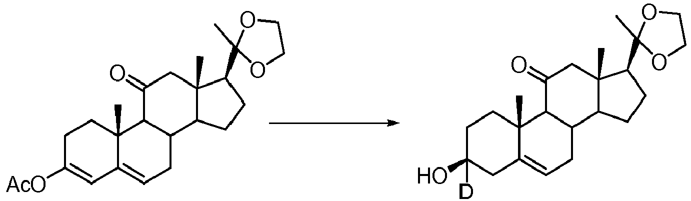

### Step 1

A solution of 10*R*,13*S*,17*S*)-10,13-dimethyl-17-(2-methyl-1,3-dioxolan-2-yl)-11-oxo-2,7,8,9,10,11,12,13,14,15,16,17-dodecahydro-1*H*-cyclopenta[*a*]-phenanthren-3-yl acetate (100mg, 0.24 mmol) in EtOH (10ml) at 0°C was added NaBD₄ (18mg, 0.43mmol) and allowed to stir for 12hr at rt. Reaction mass was evaporated under reduced pressure, diluted with EtOAc and washed with sat. NH₄Cl solution. Organic layer was separated; dried over Na₂SO₄ and evaporated. Crude was subjected to column chromatography on silica gel [100-200 mesh 10-30 % EtOAc:Hexane) to afford (3S,10R,13S,17S)-3-hydroxy-10,13-dimethyl-17-(2-methyl-1,3-dioxolan-2-yl)-1,2,3,4,7,8,9,10,12,13,14,15,16,17-tetradecahydro-11H-cyclopenta[a]phenanthren-11-one-3-d (25mg ,27 %) as off-white solid.
ESIMS: 376(M⁺ + 1)

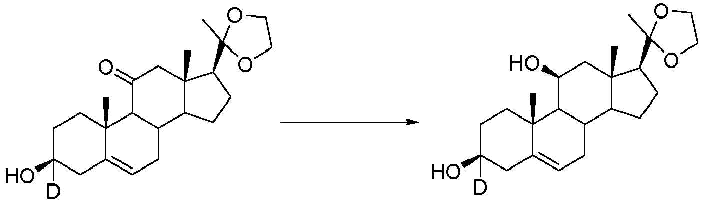

### Step 2

A solution of (3S,10R, 13S,17S)-3-hydroxy-10,13-dimethyl-17-(2-methyl-1,3-dioxolan-2-yl)-1,2,3,4,7,8,9,10,12,13,14,15,16,17-tetradecahydro-11H-cyclopenta[a]phenanthren-11-one-3-d (25mg, 0.06 mmol) in EtOH (5ml) at 0°C was added NaBH₄ (5mg, 0.11 mmol) and allowed to stir for 6 hrs at RT. Reaction mass was evaporated under reduced pressure, diluted with EtOAc and washed with sat.NH₄Cl solution. Organic layer was separated; dried over Na₂SO₄ and evaporated, to afford (3S,10R,11S,13S,17S)-10,13-dimethyl-17-(2-methyl-1,3-dioxolan-2-yl)-2,3,4,7,8,9,10,11,12,13,14,15,16,17-tetradecahydro-1 H-cyclopenta[a]phenanthrene-3-d-3,11-diol. (20mg , 80%) as off-white solid. Crude was directly used for next step.
ESIMS: 378(M⁺ + 1)

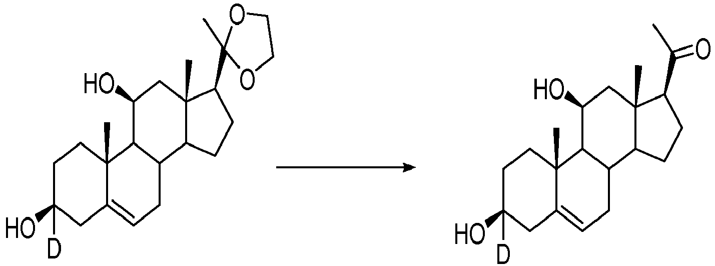

### Step 3

A solution of (3S,10R,11S,13S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3-d-3,11-diol (20mg, 0.05 mmol) in acetone (5ml) at 0°C was added H₂SO₄ (0.1ml) in 1ml water and allowed to stir for 15min. at 0°C. Reaction mass was quenched with sodium bicarbonate solution, extracted with EtOAc. Organic layer was separated; dried over Na₂SO₄, evaporated under reduced pressure. Crude was subjected to column chromatography on silica gel (100-200 mesh 1-2% MeOH:DCM) to obtain 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3-d)ethan-1-one (14mg , 82%) as a white solid.
ESIMS:334 (M⁺ + 1)

### EXAMPLE 30 [1061]

### 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3,11-d₂)ethan-1-one

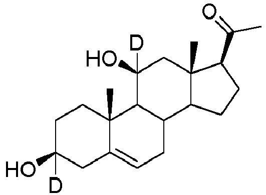

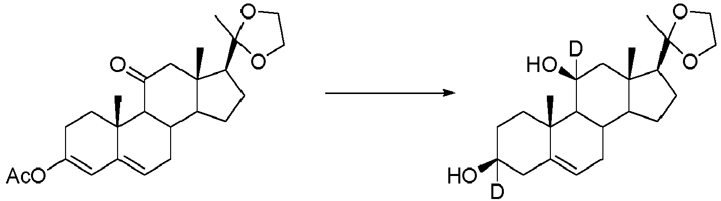

### Step 1

A solution of 10*R*,13*S*,17*S*)-10,13-dimethyl-17-(2-methyl-1,3-dioxolan-2-yl)-11-oxo-2,7,8,9,10,11,12,13,14,15,16,17-dodecahydro-1*H*-cyclopenta[a]-phenanthren-3-yl acetate (100mg, 0.24 mmol) in EtOH (10ml) at 0°C was added NaBD₄ (18mg, 0.43mmol) and allowed to stir for 12hr at rt. Reaction mass was evaporated under reduced pressure, diluted with EtOAc and washed with sat. NH₄Cl solution. Organic layer was separated; dried over Na₂SO₄ and evaporated. Crude was subjected to column chromatography on silica gel [100-200 mesh 10-30 % EtOAc:Hexane) to afford (3S,10R,11S,13S,17S)-10,13-dimethyl-17-(2-methyl-1,3-dioxolan-2-yl)-2,3,4,7,8,9,10,11,12,13,14,15,16,17-tetradecahydro-1 H-cyclopenta[a]phenanthrene-3,11-d₂-3,11-diol (30 mg ,33 %) as off-white solid.
ESIMS: 379(M⁺ + 1)

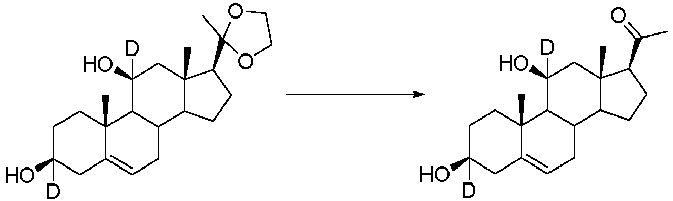

1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3,11-d₂)ethan-1-one (15mg ,57 %) was obtained as white solid from (3S,10R,11S,13S,17S)-10,13-dimethyl-17-(2-methyl-1,3-dioxolan-2-yl)-2,3,4,7,8,9,10,11,12,13,14,15,16,17-tetradecahydro-1H-cydopenta[a]phenanthrene-3,11-d₂-3,11-diol (30 mg, 0.079 mmol) using same procedure as above.

### EXAMPLE 31 [1047]

### 1-((3S,10R,11S,13S,17S)-3,11-dihydroxy-10,11,13-trimethyl-2,3,4,7,8,9,10,11,12,13,14,15,16,17-tetradecahydro-1H-cyclopenta[a]phenanthren-17-yl)ethan-1-one

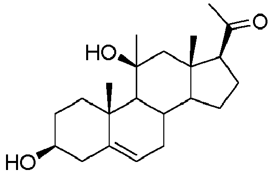

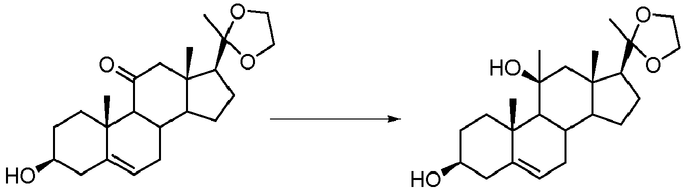

### Step 1

A solution of (10*R*,13*S*,17*S*)-10,13-dimethyl-17-(2-methyl-1,3-dioxolan-2-yl)-1,2,3,4,7,8,9,10,12,13,14,15,16,17-tetracahydro-11*H*-cyclopenta[a]-phenanthren-11-one compound (30mg, 0.08 mmol) in THF (10ml) at 0°C was added MeLi (0.03ml, 0.08 mmol) and allowed to stir for 1 hr at 0°C. Reaction mass was quenched with sat. NH₄Cl solution and extracted with EtOAc. Organic layer was separated; dried over Na₂SO₄ and evaporated under reduced pressure to obtain (10R,11S,13S,17S)-10,11,13-trimethyl-17-(2-methyl-1,3-dioxolan-2-yl)-2,3,4,7,8,9,10,11,12,13,14,15,16,17-tetradecahydro-1 H-cyclopenta[a]phenanthrene-3,11-diol (25 mg , 80%) as a white solid.
ESIMS: 391 (M⁺ + 1)

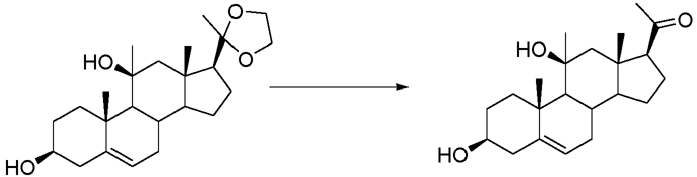

### Step 2

1-((3S,10R,11S,13S,17S)-3,11-dihydroxy-10,11,13-trimethyl-2,3,4,7,8,9,10,11,12,13,14,15,16,17-tetradecahydro-1H-cyclopenta[a]phenanthren-17-yl)ethan-1-one (7mg ,31 %) was obtained from (10R,11S,13S,17S)-10,11,13-trimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol using the same procedure for deprotection of ketal mentioned above.
ESIMS: 347 (M⁺ + 1)

### EXAMPLE 32[1023]

### (10R,11S,13S,17R)-11,17-dihydroxy-10,13-dimethyl-3-oxo-2,3,6,7,8,9,10,11,12,13,14,15,16,17-tetradecahydro-1H-cyclopenta[a]phenathrene-17-carboxylic acid

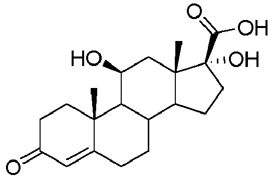

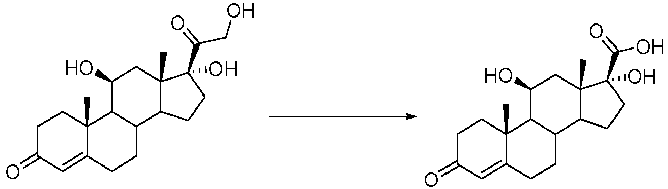

Cortisol (1.0 g, 2.76 mmol) was taken in THF:MeOH (16:4 ml). NalO₄ (1.72 g, 8.03 mmol) in water (15 ml) was preheated at 50°C and added to it. The reaxction mixture was stirred at room temperature at 2 hrs. After completion, the reaction mixture was concentrated under reduced pressure. The solid residue was triturated with water. The solid was filtered and washed with water, dried under vaccum to obtain (10R,11S,13S,17R)-11,17-dihydroxy-10,13-dimethyl-3-oxo-2,3,6,7,8,9,10,11,12,13,14,15,16,17-tetradecahydro-1H-cyclopenta[a]phenathrene-17-carboxylic acid as white solid (810 mg, 84%)
ESIMS: 334(M⁺ + 1)

### Example 33 [1018]

### (10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one

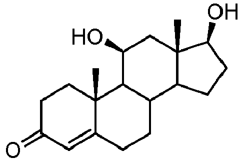

(10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one (24.8 mg, 55% yield) was obtained from (8S,9S,10R,11S,13S,14S)-11-hydroxy-10,13-dimethyl-7,8,9,10,11,12,13,14,15,16-decahydro-1H-cyclopenta[a]phenanthrene-3,17(2H,6H)-dione (50 mg,) using the procedure same in (8S,9S,10R,11S,13S,14S,17R)-17-((R)-1,2-dihydroxyethyl)-11,17-dihydroxy-10,13-dimethyl-6,7,8,9,10,11,12,13,14,15,16,17-dodecahydro-1H-cyclopenta[a]phenanthren-3(2H)-one.
ESIMS: 334(M⁺ + 1)

### Example 34 [1017]

### (3S,10R,11S,13S,17S)-10,13-dimethyl-2,3,4,7,8,9,10,11,12,13,14,15,16,17-tetradecahydro-1H-cyclopenta[a]phenanthrene-3,11,17-triol

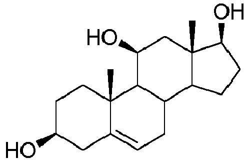

(3S,10R,11S,13S,17S)-10,13-dimethyl-2,3,4,7,8,9,10,11,12,13,14,15,16,17-tetradecahydro-1H-cyclopenta[a]phenanthrene-3,11,17-triol (9 mg, 90% yield) was obtained from (10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one (10mg, 32.89mmol) using procedure as in example 1.

### Example 35 [1062]

### (3S,10R,11S,13S,17S)-10,13-dimethyl-2,3,6,7,8,9,10,11,12,13,14,15,16,17-tetradecahydro-1H-cyclopenta[a]phenanthrene-3,11,17-triol

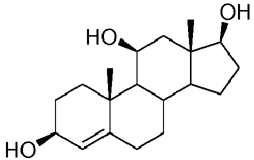

(3S,10R,11S,13S,17S)-10,13-dimethyl-2,3,6,7,8,9,10,11,12,13,14,15,16,17-tetradecahydro-1H-cyclopenta[a]phenanthrene-3,11,17-triol was obtained from (10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one (10mg, 32.89mmol) using method for synthesis of (3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol

### EXAMPLE 36 [1020]

### (10R,11S,13S,17S)-11-hydroxy-10,13-dimethyl-3-oxo-2,3,6,7,8,9,10,11,12,13,14,15,16,17-tetradecahydro-1H-cyclopenta[a]phenanthrene-17-carboxylic acid

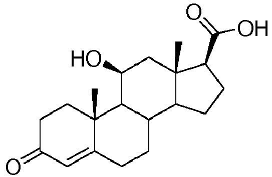

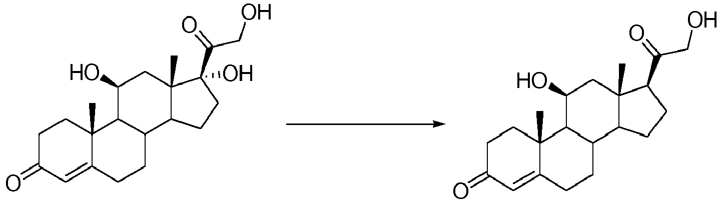

### Step 1:

Hydrocortisone (500 mg, 1.38 mmol) was taken in ACN (200 ml) was added trimethyl silyl iodide (1.17ml, 8.28mmol) dropwise. The reaction mixture was stirred at room temperature for 20 min. After completion, the reaction mixture was quenched by 10% sodium thiosulfate (50 ml), washed with sat. NaHCO₃, extracted with ethyl acetate. Organic layer was separated; dried over Na₂SO₄ and evaporated under reduced pressure to obtain (10R,11S,13S,17S)-11-hydroxy-17-(2-hydroxyacetyl)-10,13-dimethyl-1,2,6,7,8,9,10,11,12,13,14,15,16,17-tetradecahydro-3H-cyclopenta[a]phenanthren-3-one as light yellow solid (450 mg, 94%)
ESIMS: 347(M⁺ + 1)

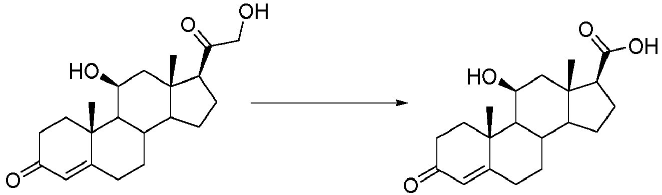

### Step 2:

To a stirred solution of (10R,11S,13S,17S)-11-hydroxy-17-(2-hydroxyacetyl)-10,13-dimethyl-1,2,6,7,8,9,10,11,12,13,14,15,16,17-tetradecahydro-3H-cyclopenta[a]phenanthren-3-one (1.0 g, 2.89mmol) in acetone:water (50 ml) was added NalO₄ (2.47 g, 11.5 mmol) and stirred the reaction mixture for 2 hr at rt. Reaction mixture was quenched with water (25 ml), extracted with EtOAc. Organic layer was separated; dried over Na₂SO₄ and evaporated under reduced pressure to obtain (10R,11S,13S,17S)-11-hydroxy-10,13-dimethyl-3-oxo-2,3,6,7,8,9,10,11,12,13,14,15,16,17-tetradecahydro-1H-cyclopenta[a]phenanthrene-17-carboxylic acid as white solid (850 mg, 88%)
ESIMS: 333(M⁺ + 1)

### EXAMPLE 37 [1063]

### (3S,8S,9S,10S,11S,13S,14S,17S)-3,11-dihydroxy-N,10,13-trimethylhexadecahydro-1H-cyclopenta[a]phenanthrene-17-carboxamide

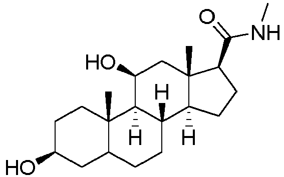

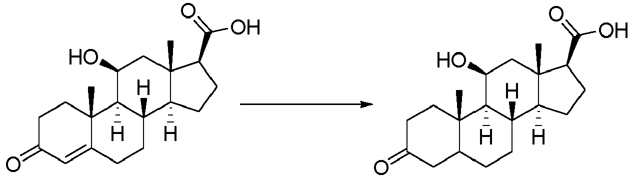

### Step 1:

(8S,9S,10R,11 S,13S,14S,17S)-11-hydroxy-10,13-dimethyl-3-oxo-2,3,6,7,8,9,10,11,12,13,14,15,16,17-tetradecahydro-1H-cyclopenta[a]phenanthrene-17-carboxylic acid (800 mg, 2.40 mmol) was taken in ethyl acetate. Pd/C (80mg, 10%) was added to it and mixture was stirred under H₂ atmosphere for 1 hr. The reaction mixture was filtered, concentrated to obtain (8S,9S,10S,11S,13S,14S,17S)-11-hydroxy-10,13-dimethyl-3-oxohexadecahydro-1H-cyclopenta[a]phenanthrene-17-carboxylic acid (750 mg, 93% yield) as white solid.
ESIMS: 335 (M⁺ + 1)

### Step 2:

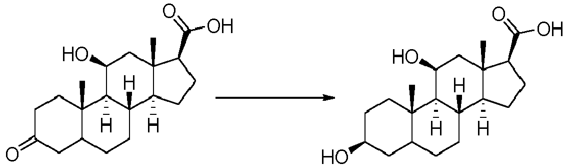

A solution of (8S,9S,10S,11S,13S,14S,17S)-11-hydroxy-10,13-dimethyl-3-oxohexadecahydro-1H-cyclopenta[a]phenanthrene-17-carboxylic acid (750mg, 2.24mmole) in THF:EtOH (30ml) at 0°C was added NaBH₄ (170mg, 4.49, mmol) and allowed to stir for 12hr at rt. Reaction mass was evaporated, diluted with EtOAc and washed with sat.NH₄Cl solution. Organic layer was separated; dried over Na₂SO₄ and evaporated under reduced pressure. Crude was subjected to column chromatography on silica gel to column chromatography on silica gel (100-200 mesh, 3-4% MeOH:DCM) to afforded the (3S,8S,9S,10S,11S,13S,14S,17S)-3,11-dihydroxy-10,13-dimethylhexadecahydro-1H-cyclopenta[a]phenanthrene-17-carboxylic acid (600mg, 79%) as white solid.
ESIMS: 337 (M⁺ + 1)

### Step 3:

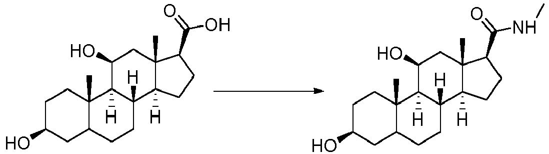

A solution of (3S,8S,9S,10S,11S,13S,14S,17S)-3,11-dihydroxy-10,13-dimethylhexadecahydro-1H-cyclopenta[a]phenanthrene-17-carboxylic acid (70 mg, 0.20 mmol) in DMF (1 ml). EDC.HCl (60 mg, 0.31 mmol), HOBt (42mg, 0.31mmol) and N-methyl morpholine (0.06ml, 0.52mmol) was added along with methylamine HCI (35mg, 0.52mmol) and mixture was stirred at room temperature for 12 hr. The reaction mixture was quenched with brine (20 ml), extracted with ethyl acetate (2 X 50 ml). The combined organic extract was dried (Na₂SO₄) concentrated to obtain crude which was purified by column chromatography (silica gel 100-200 mesh) to obtain (3S,8S,9S,10S,11S,13S,14S,17S)-3,11-dihydroxy-N,10,13-trimethylhexadecahydro-1H-cyclopenta[a]phenanthrene-17-carboxamide (60-70% yield) as off white solid.
ESIMS: 350 (M⁺ + 1)

### EXAMPLE 38[1064]

### (3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide

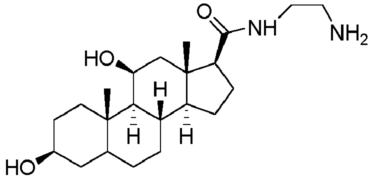

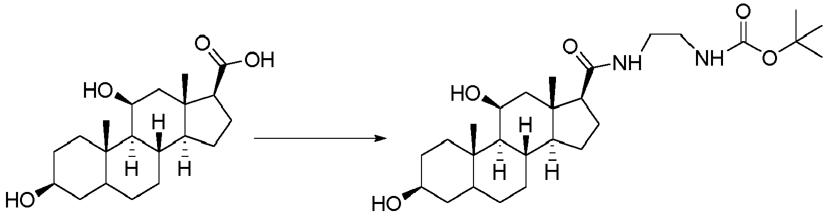

### Step 1:

A solution of (3S,8S,9S,10S,11S,13S,14S,17S)-3,11-dihydroxy-10,13-dimethylhexadecahydro-1H-cyclopenta[a]phenanthrene-17-carboxylic acid (70 mg, 0.20 mmol) in DMF (1 ml). EDC.HCl (60 mg, 0.31 mmol), HOBt (42mg, 0.31 mmol) and N-methyl morpholine (0.06ml, 0.52mmol) was added along with tert-butyl (2-aminoethyl)carbamate (83mg, 0.52mmol) and mixture was stirred at room temperature for 12 hr. The reaction mixture was quenched with brine (20 ml), extracted with ethyl acetate (2 X 50 ml). The combined organic extract was dried (Na₂SO₄) concentrated to obtain crude which was purified by column chromatography (silica gel 100-200 mesh) to obtain tert-butyl (2-((3S,8S,9S,10S,11S,13S,14S,17S)-3,11-dihydroxy-10,13-dimethylhexadecahydro-1H-cyclopenta[a]phenanthrene-17-carboxamido)ethyl)carbamate (60-70% yield) as off white solid.
ESIMS: 479 (M⁺ + 1)

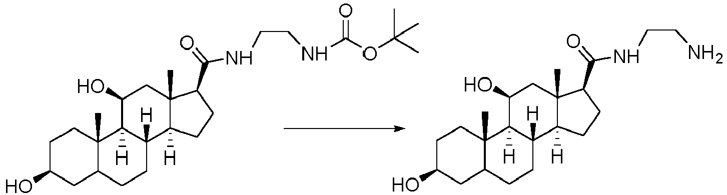

### Step 2:

To a stirred solution of tert-butyl (2-((3S,8S,9S,10S,11S,13S,14S,17S)-3,11-dihydroxy-10,13-dimethylhexadecahydro-1H-cyclopenta[a]phenanthrene-17-carboxamido)ethyl)carbamate (50mg, 0.10mmol) in DCM (10ml) was added trifluoro acetic acid (12 mg, 0.10mmol) at 0°C and allowed to stirred at rtfor 1 hr. Then all volatiles were evaporated and crude compound was washed with diethyl ether (10ml) to obtain (3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide (30-40% yield) as off white solid.
ESIMS: 379 (M⁺ + 1)

### EXAMPLE 39 [1065]

### (3S,10R,11S,13S,17S)-5-methoxy-10,13-dimethyl-17-(2-methyl-1,3-dioxolan-2-yl)hexadecahydro-1H-cyclopenta[a]phenanthrene-3,6,11-triol

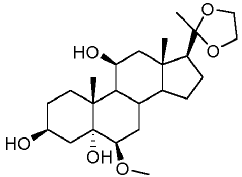

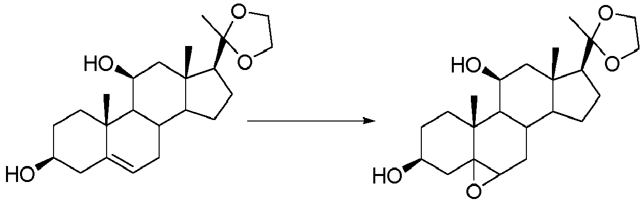

### Step 1:

To a stirred solution of (3S,10R,11S, 13S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol (300mg, 0.79mmol) in DCM (20ml) was added m-CPBA (205 mg 1.19 mmol) at 0°C and allowed to stirred at rt for 4 hr. Then reaction mixture was diluted with DCM (50ml) washed with water (2X 50ml) .The combined organic extract was dried (Na₂SO₄) concentrated . Crude which was purified by column chromatography (silica gel 100-200 mesh, 20-30% EtOAc/Cyclohexane) to obtain (3S,9S,9aS,11S,11bR)-9a,11b-dimethyl-9-(2-methyl-1,3-dioxolan-2-yl)hexadecahydrocyclopenta[1,2]phenanthro[8a,9-b]oxirene-3,11-diol (30-40% yield) as off white solid.
ESIMS: 393 (M⁺ + 1)

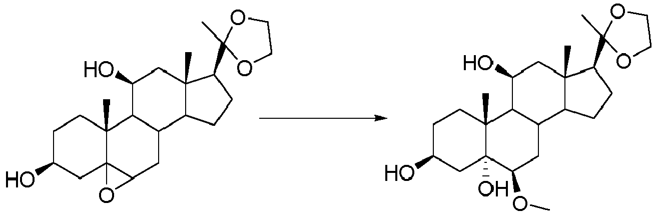

### Step 2:

To a stirred solution of (3S,9S,9aS,11S,11bR)-9a,11b-dimethyl-9-(2-methyl-1,3-dioxolan-2-yl)hexadecahydrocyclopenta[1,2]phenanthro[8a,9-b]oxirene-3,11-diol (50mg, 0.12mmol) in MeOH (10ml) was added p-TSA (33 mg 0.19 mmol) at 0°C and allowed to stirred at rt for 1 hr. All volatiles were evaporated , diluted with EtOAc and washed with water (2X25ml).The combined organic extract was dried (Na₂SO₄) concentrated to obtain (3S,10R,11S,13S,17S)-5-methoxy-10,13-dimethyl-17-(2-methyl-1,3-dioxolan-2-yl)hexadecahydro-1H-cyclopenta[a]phenanthrene-3,6,11-triol (30-40% yield) as white solid.
ESIMS: 425 (M⁺ + 1)

### EXAMPLE 40 [1055]

### 1-((3R,8S,9S,10R,11S,13S,14S,17S)-3-fluoro-11-hydroxy-10,13-dimethy)-2,3,4,7,8,9,10,11,12,13,14,15,16,17-tetradecahydro-1H-cyclopenta[a]phenanthren-17-yl)ethan-1-one

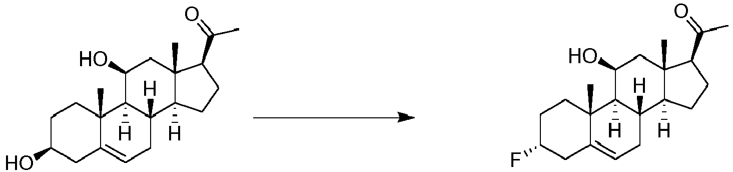

A solution of 1-(3,11-dihydroxy-10,13-dimethyl-2,3,4,7,8,9,10,11,12,13,14,15,16,17-tetradecahydro-1H-cyclopenta[a]phenanthren-17-yl)-ethanone (100 mg, 0.3012 mmols) in DCM (5 ml) was treated with DAST (0.065 ml,1.5eq) at -78°C and stirred at RT for 6 hr. The mixture was concentrated under vacuum and crude was by silica gel chromatography eluting with 2% Methanol /DCM. 1-((3R,8S,9S,10R,11S,13S,14S,17S)-3-fluoro-11-hydroxy-10,13-dimethyl-2,3,4,7,8,9,10,11,12,13,14,15,16,17-tetradecahydro-1H-cyclopenta[a]phenanthren-17-yl)ethan-1-one was obtained as a white solid (30 mg). MS (ES-API) *m*/*z* (M+H⁺) 335.7.

### EXAMPLE 41 [1053]

### 1-((8S,9S,10S,11S,13S,14S,17S)-11-hydroxy-10,13-dimethylhexadecahydro-1H-cyclopenta[a]phenanthren-17-yl)ethan-1-one

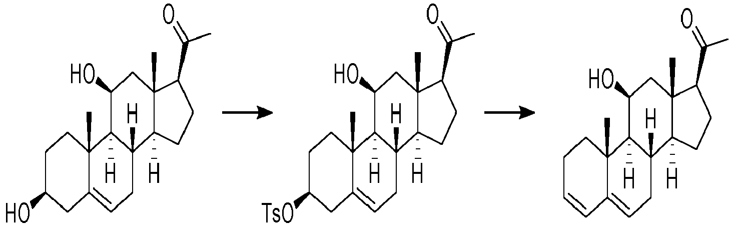

### Step 1:

A solution of 1-(3,11-dihydroxy-10,13-dimethyl-2,3,4,7,8,9,10,11,12,13,14,15,16,17-tetradecahydro-1H-cyclopenta[a]phenanthren-17-yl)-ethanone (200 mg,0.602 mmols) in pyridine (2 ml) was treated with p-toluene sulphonic chloride (343 mg, 3eq) at 0°C temperature and then stirred at RT for 8hrs. The reaction was quenched with crashed ice and stirred for 30 mins.Sodis was filtered and dried under vacuum to get crude product. Crude was by silica gel chromatography eluting with 20 % Ethylacetate /Hexene. (3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl 4-methylbenzenesulfonate was obtained as a white solid (180 mg). MS (ES-API) *m*/*z* (M+H⁺) 487.7.

### Step 2:

A solution of (3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl 4-methylbenzenesulfonate (150 mg,0.308 mmols) in THF (5 ml) was treated with potassium carbonate (127 mg, 3eq) at 60°C temperature for 8hrs. The reaction mixture was filtered and filtrate was dried under vacuum to get crude product. Crude was by silica gel chromatography eluting with 10 % Ethylacetate /Hexene. 1-((8S,9S,10R,11S,13S,14S,17S)-11-hydroxy-10,13-dimethyl-2,7,8,9,10,11,12,13,14,15,16,17-dodecahydro-1H-cyclopenta[a]phenanthren-17-yl)ethan-1-one was obtained as a white solid (80 mg). MS (ES-API) *m*/*z* (M+H⁺) 315.2.

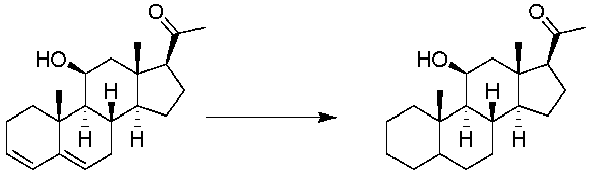

### Step 3:

A solution of 1-((8S,9S,10R,11S,13S,14S,17S)-11-hydroxy-10,13-dimethyl-2,7,8,9,10,11,12,13,14,15,16,17-dodecahydro-1H-cyclopenta[a]phenanthren-17-yl)ethan-1-one (50 mg, 0.158,mmols) and 10% Pd-C (10 mg) in ethanol (10 ml) was stirred under a balloon of hydrogen for 1 hr. The reaction mixture was filtered and filtrate was dried under vacuum to get crude product.Crude was by silica gel chromatography eluting with 8 % Ethyl acetate /Hexene. 1-((8S,9S,10S,11S,13S,14S,17S)-11-hydroxy-10,13-dimethylhexadecahydro-1H-cyclopenta[a]phenanthren-17-yl)ethan-1-one was obtained as a white solid (20 mg). MS (ES-API) *m*/*z* (M+H⁺) 319.2.

### EXAMPLE 42 [1070]

### (3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol

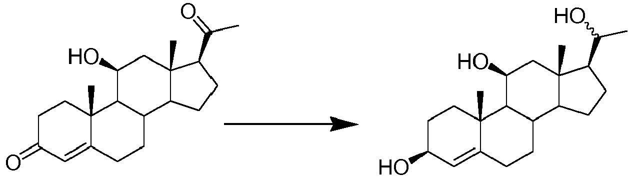

A solution of 17-Acetyl-11-hydroxy-10,13-dimethyl-1,2,6,7,8,9,10,11,12,13,14,15,16,17-tetradecahydro-cyclopenta[a]phenanthren-3-one (100 mg, 0.303 mmols) in methanol (5 ml) was treated with NaBH4(35 mg ,3eq) and stirred at RT for 4 hr. The mixture was concentrated under vacuum and crude was by silica gel chromatography eluting with 2% Methanol /DCM . (3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol was obtained as a white solid (60 mg). MS (ES-API) *m*/*z* (M+H⁺) 335.7.

### EXAMPLE 43 [1022]

### (3E,8S,9S,10R,11S,13S,14S,17Z)-11-hydroxy-10,13-dimethyl-1,6,7,8,9,10,11,12,13,14,15,16-dodecahydro-3H-cyclopenta[a]phenanthrene-3,17(2H)-dione dioxime

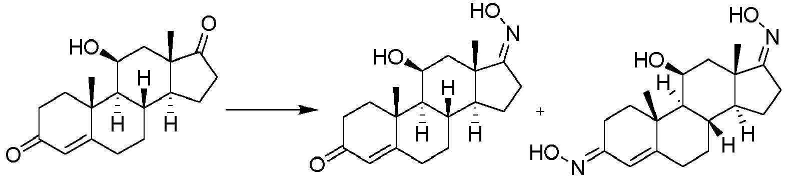

A solution of 11-hydroxy-10,13-dimethyl-1,6,7,8,9,10,11,12,13,14,15,16-dodecahydro-2H-cyclopenta[a]phenanthrene-3,17-dione (300 mg, 0.909 mmols) in methanol (4 ml) was treated with Pyridine (0.22 ml, 3 eq) and hydroxylamine hydrochloride (81mg, 1.3 Eq) at 0 °C temperature and stirred for 6 hrs at RT. The mixture was concentrated to get crude product. Crude was by silica gel chromatography eluting with 1.5 % Methanol /DCM . (8S,9S,10R,11S,13S,14S,Z)-11-hydroxy-17-(hydroxyimino)-10,13-dimethyl-1,2,6,7,8,9,10,11,12,13,14,15,16,17-tetradecahydro-3H-cyclopenta[a]phenanthren-3-one (80 mg) and (3E,8S,9S,10R,11 S,13S,14S,17Z)-11-hydroxy-10,13-dimethyl-1,6,7,8,9,10,11,12,13,14,15,16-dodecahydro-3H-cyclopenta[a]phenanthrene-3,17(2H)-dione dioxime (60 mg) was obtained as a white solid (mg).

### EXAMPLE 44 [1056]

### (3S,8S,9S,10S,11S,13S,14S)-17-amino-10,13-dimethylhexadecahydro-1H-cyclopenta[a]phenanthrene-3,11-diol

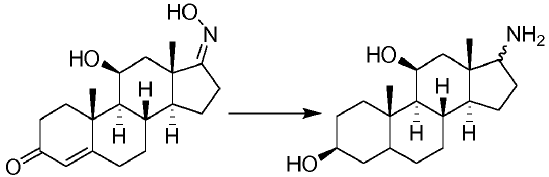

A solution of (8S,9S,10R,11S,13S,14S,Z)-11-hydroxy-17-(hydroxyimino)-10,13-dimethyl-1,2,6,7,8,9,10,11,12,13,14,15,16,17-tetradecahydro-3H-cyclopenta[a]phenanthren-3-one (50 mg,0.157 mmols ) and 10% Pd-C (10 mg) in Methanol (20 ml) was stirred under 60 psi hydrogen pressure in a parr shaker for 16 hrs.Reaction mixture was filtered through celite and filtrate for was treated with NaBH4(18 mg, 3eq) and stirred at RT for 4 hr. The mixture was concentrated under vacuum and crude was by silica gel chromatography eluting with 2% Methanol /DCM. (3S,8S,9S,10S,11S,13S,14S)-17-amino-10,13-dimethylhexadecahydro-1H-cyclopenta[a]phenanthrene-3,11-diol was obtained as a white solid (10 mg). MS (ES-API) *m*/*z* (M+H⁺) 308.2.

**3-(4,5-Dimethylthiazol-2-yl)-2,5-diphenyltetrazolium bromide (MTT) assay.** 6,000 BCAEC were seeded into clear 96-well flat bottom plates at 100 µl per well. After starvation, cells were treated for 48 hours with the corresponding compounds. Compounds were changed every 12 and 24 hours (with 100 µl per well of fresh medium). Following the treatment medium was aspirated from plates, and washed once with 100 µl HBSS, subsequently 100 µl per well 0.5 mg/ml MTT in phenol red-free DMEM was added. Plates were incubated for 1 hour at 37 oC, and this was followed by aspiration of MTT solution, addition of 100 µl per well DMSO to dissolve formazan crystals, and incubation at 37°C for 15 minutes. Mitochondrial function was quantified by absorbance was measured at 540 nm with background substraction at 670 nm using. After MTT absorbance measure, media was removed and cells quantify in each well as described elsewhere (Oliver et al, 1989) with some modifications. In brief, 100 µl of 10 % formol saline to each well was added, and incubated at 4°C for 24 hours. Next, formol solution was removed from the wells and 100 µl of filtered 0.1 % (w/v) Methylene Blue in 0.01 M borate buffer (pH 8.5) was added to each well. After 30 minutes, excess dye was removed and washed off 3 times with 0.01 M borate buffer (pH 8.5). The stained dye on the cells was eluted by the addition of 100 µl of 1:1 (v/v) ethanol and 0.1 M-HCl in each well. The plates were then gently shaken and incubated for 15 minutes under a bench rocker. Absorbance was measured at 650 nm. The MTT/Methylene Blue ratio was used to quantify the mitochondrial function. Oliver MH, Harrison NK, Bishop JE, Cole PJ, Lauren GJ. A rapid and convenient assay for counting cells cultured in microwell plates: application for assessment of growth factors. J Cell Sci. 1989,3:513-8.

**Slot Blot Method.** Following treatment, bovine aortic endothelial cells grown in 12 well plates are scrapped in 50 µl of lysis buffer (1% Triton X-100, 20 mm Tris, 140 mm NaCl, 2 mm EDTA, and 0.1% SDS) with protease and phosphatase inhibitor cocktails (P2714 and P2850, Sigma-Aldrich) supplemented with 0.15 mm PMSF, 5 mm Na₃VO₄ and 3 mm NaF. Homogenates are passed through an insulin syringe five times, sonicated for 30 min at 4°C and centrifuged (12,000 g) for 10 min. The total protein content is measured in the supernatant using the Bradford method. A total of 10-20 µg of protein are loaded into a pre-assembled slot blot apparatus to be transferred under vacuum onto polyvinyl membranes, incubated for 1 h in blocking solution (5% non-fat dry milk in TBS plus 0.1% Tween 20 (TBS-T)), followed by a overnight incubation at 4° C with primary antibodies (oxidative phosphorylation complex I or GAPDH). Primary antibodies are diluted in TBS-T plus 5% non-fat dry milk. Membranes are washed (3 X for 5 min) in TBS-T and incubated 1 h at room temperature in the presence of HRP-conjugated secondary antibodies diluted in blocking solution. Membranes are again washed 3 times in TBS-T, and the immunoblots developed using an ECL Plus detection kit (Amersham-GE). Band intensities are digitally quantified using ImageJ software (http://www.nih.gov). Complex I values are normalized for loading differences using GAPDH after stripping and reprobing of the membrane. All Western blot images to be obtained will be within the linear range of X-ray film to ensure computer-assisted densitometry accuracy.

Compounds of the invention were tested in the above assays and the values are set out in the Table 1 below. Unless indicated otherwise, the data refer to the title compounds in the examples.

**Table 1. Biological Activity**

| **Example #** | **MTT Assay** | **Slot Blot Assay** |
|---|---|---|
| | **- indicates ≤ 110%** | **- indicates ≤ 150% HCAEC** |
| | **+ indicates > 110%** | **+ indicates > 150% HCAEC** |
| 1001 | + | NT |
| 1002 | + | + |
| 1003 | + | NT |
| 1004 | NT | + |
| 1005 | NT | NT |
| 1006 | NT | NT |
| 1007 | + | NT |
| 1008 | NT | NT |
| 1009 | NT | + |
| 1059 | NT | NT |
| 1010 | NT | NT |
| 1011 | NT | NT |
| 1012 | NT | + |
| 1013 | NT | NT |
| 1016 | - | NT |
| 1014 | NT | NT |
| 1015 | NT | NT |
| 1025 | NT | NT |

| | | |
|---|---|---|
| NT - Not Tested | | |

### AMPK activation protocol:

AMPK activation potential of the compounds was evaluated using cell based ELISA.

Hepatoma (Hep G2) liver cells were maintained in a T 75 culture flask-containing 25 mM DMEM+10% fetal calf serum. The cells were maintained in a T 75 culture flask-containing medium (DMEM+10% fetal calf serum). On reaching a confluence of 70 to 80%, the cells were seeded in a 96 well plate at a density of 40,000 cells per well in 25mM DMEM+10% FCS medium. The plates were then incubated at 37° C. with 5% CO₂ for 24hours. Various concentrations of drugs were prepared in DMSO and diluted to required concentration with the medium and incubated at 37° C. with 5% CO₂ for 30 min and 1h for Epicatechin analogs and 11-BHP analogs respectively. Metformin was used as positive control .Cells were fixed with 4% formaldehyde for 30 minutes at room temperature and washed three times with PBS containing 0.1% Triton X-100. Endogenous peroxidase was quenched with 1 % H₂O₂ in PBS-T(0.1% Tween 20) for 30 minutes and washed three times in PBS-T. Cells were blocked with 1% BSA in PBS-T for 1 hour. The cells were incubated with 1:1000 dilution primary antibody(Phospho-AMPKα (Thr172) Rabbit mAb,CellSignaling in PBS-T containing 5% BSA at 4° C overnight. The cells were then washed three times with PBS-T for 5 minutes and incubated with 1:1000 dilution secondary antibody (Anti-rabbit IgG, HRP-linked Antibody, Cell Signaling) in PBS-T with 1% BSA for 1 hour at RT.Cells were washed three times with PBS-T for 5 minutes The cells were incubated with 100 µlTMBsubstrate solution for 30minutes and the reaction was stopped with 100 µl of 2N H₂SO₄. Then the plate was read at 450 nM using ELISA plate reader and absorbance recorded. % activity was calculated using DMSO control as 100%.

Compounds of the present invention were tested for the above AMPK activation potential and the percentage actication pAMPK at 1nM values are set out in Table 2 below. Unless indicated otherwise, the data refer to the title compounds in the examples.

**TABLE 2**

| **S.No.** | **Compound No** | **% activation pAMPK at 1nM** | **SPR No** | **% activation pAMPK at 1 nM** |
|---|---|---|---|---|
| 1. | 1016 | 104 | 1018 | 106 |
| 2. | 1023 | 127 | 1017 | 97 |
| 3. | 1020 | 132 | 1024 | 133 |
| 4. | 1030 | 105 | 1022 | 129 |
| 5. | 1033 | 107 | 1035 | 102 |
| 6. | 1048 | 108 | 1036 | 102 |
| 7. | 1021 | 129 | 1037 | 95 |
| 8. | 1026 | 110 | 1038 | 103 |
| 9. | 1025 | 118 | 1039 | 100 |
| 10. | 1028 | 103 | 1040 | 95 |
| 11. | 1032 | 105 | 1041 | 95 |
| 12. | 1029 | 105 | 1042 | 96 |
| 13. | 1031 | 109 | 1045 | 116 |
| 14. | 1019 | 103 | 1047 | 119 |
| 15. | 1034 | 104 | | |

For reasons of clompleteness, various aspects of the invention are set out in the following numbered clauses:
Clause 1. A compound of structural Formula I: 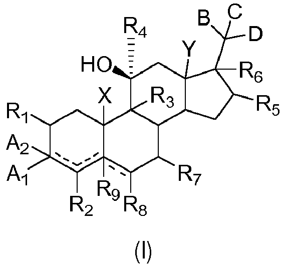 or a salt thereof, wherein:
   a dashed line represents an optional double bond or a methylene group attached to the atoms on either side such that a fused cyclopropyl ring results, provided that cumulated double bonds (=C=) are not allowed; n is an integer from 1 to 6;
   A₁ is selected from the group consisting of hydrogen, deuterium, halogen, -OH, PO₄³⁻, -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁;
   A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom of the heterocylic ring or heteroaryl ring is selected from N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
   B and C are each independently selected from the group consisting of hydrogen, -OH, -OR₁₀, -NR₁₀R₁₁, -OQ,-NR₁₀Q, -NR₁₁COR₁, -OCOOR₁, -NR₁₁COOR₁₀, -NR₁₁CONR₁₀;-COR₁₀, -COOR₁₀,-CONR₁₀R₁₁, _{,}-NH(CH₂)ₙNH₂;_{,} -NR₁₁CO(CH₂)ₙNH₂ and C₁-C₁₂ alkyl, wherein said C₁-C₁₂ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁; or B and C, taken together with the atom to which they are attached, may form a 3-7 membered cycloalkyl, 4-7 membered heterocycloalkyl, or 5-6 membered heteroaryl, wherein heteroatom of the heterocylic ring or heteroaryl ring is selected from N, O or S;, any of which may be optionally substituted with one or more C₁-C₁₂ alkyl; wherein said C₁-C₁₂ alkyl may be optionally substituted with one or more substituents selected from the group consisting of fluorine, chlorine, -OR₁₀, -OCOR₁₀, -NR₁₁COR₁₀, -OCOOR₁₀, -NR₂COOR₁₀, -NR₁₁CONR₁₀R₁₁,-COR₁₀, -COOR₁₀, and -CONR₁₀R₁₁; or B and C, taken together, are =O, =N-OH or =N-NH₂;
   D is selected from the group consisting of hydrogen, hydroxyl, methyl, ethyl, propyl, isopropyl, cyclopropyl, amino, hydroxyalkyl, -CD₃, -CF₃, -OR₁₀, -OCF₃, phenyl, 4-6 membered heterocycloalkyl, and 5-6 membered heteroaryl; wherein heteroatom in the said heteroalkyl or heteroaryl ring is selected from the group of N, O or S; wherein said 5-6 membered heterocycloalkyl or 5-6 membered heteroaryl may be optionally substituted with one or more substituents selected from the group consisting of C₁-C₄ alkyl, -R₁₀, -OR₁₀, -NR₁₀R₁₁, fluorine, chlorine, and -CF₃ or a C=O group; or when taken together with B or C, may represent a double bond; or B, C and D, taken together with the atom to which they are attached, may form a carbonyl group, oxime group, phenyl, 4-6 membered heterocycloalkyl, or 5-6 membered heteroaryl; any of which may be optionally substituted with one or more substituents selected from the group consisting of -C₁-C₄ alkyl, -R₁₀,-OR₁₀, -NR₁₀R₁₁, fluorine, chlorine, -C=O and -CF₃;
   R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀, - NR₁₁CONR₁₀R₁₁, -COR₁₀, -COOR₁₀, and -CONR₁₀R₁₁;
   R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₃ is selected from the group consisting of hydrogen, fluorine, chlorine, methyl, and -OR₁₀;
   R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
   R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
   R₈ and Rg are each selected from hydrogen, hydroxy, methoxy, amines, alkyl or acyl.
   R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
   R₁₂ and R₁₃ are each independently selected from the group consisting of hydrogen, C₁-C₆ alkyl, C₁-C₆ haloalkyl, amino, substituted amino, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
   R₁₄ and R₁₅ are each independently selected from the group consisting of hydrogen and C₁-C₆ alkyl;
   R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl;
   Q is selected from the group consisting of: 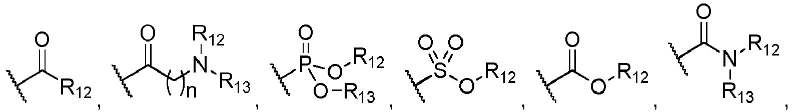 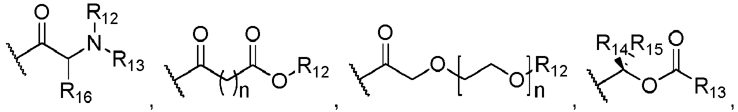 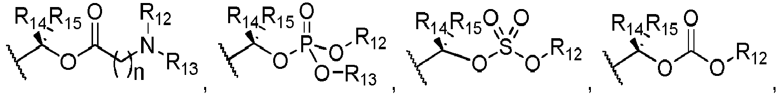 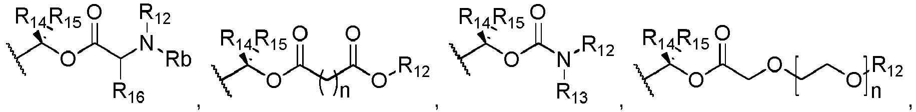 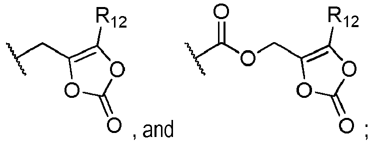 and
   X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀,-NR₁₁CONR₁₀R₁₁, -COR₁₀, -COOR₁₀, and -CONR₁₀R₁₁.
Clause 2. The compound as claused in clause 1, having structural Formula II: 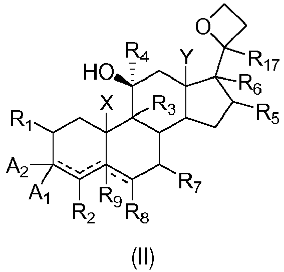 or a salt thereof, wherein:
   a dashed line represents an optional double bond or a methylene group attached to the atoms on either side such that a fused cyclopropyl ring results, provided that cumulated double bonds (=C=) are not allowed;
   n is an integer from 1 to 6;
   m is an integer from 1 to 6;
   A₁ is selected from the group consisting of hydrogen, -OH, deuterium, halogen, PO₄³⁻ , -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁;
   A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom in the said heteroalkyl or heteroaryl ring is selected from the group of N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀,-NR₁₁COR₁₀, -OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
   R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₃ is selected from the group consisting of hydrogen, fluorine, chlorine, methyl, and -OR₁₀;
   R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
   R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
   R₈ and Rg are each selected from hydrogen, hydroxy, methoxy, amines, alkyl or acyl.R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
   R₁₂ and R₁₃ are each independently selected from the group consisting of hydrogen, C₁-C₆ alkyl, C₁-C₆ haloalkyl, amino, substituted amino, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
   R₁₄ and R₁₅ are each independently selected from the group consisting of hydrogen and C₁-C₆ alkyl;
   R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl; Q is selected from the group consisting of: 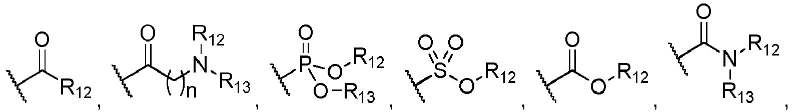 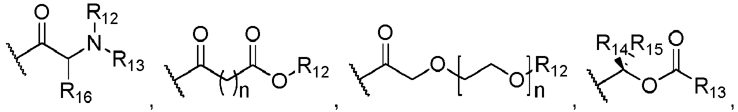 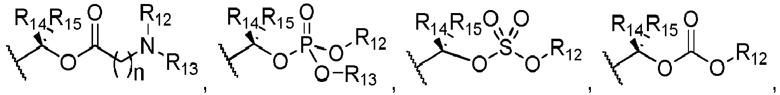 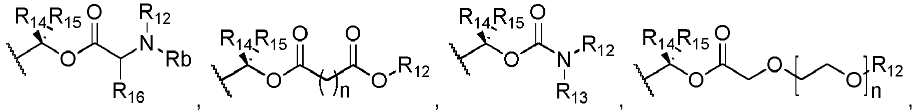 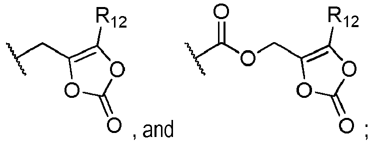 and
   X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀,-NR₁₁CONR₁₀R₁₁, -COR₁₀, -COOR₁₀, and -CONR₁₀R₁₁.
Clause 3. The compound as claused in clause 1, having structural Formula III: 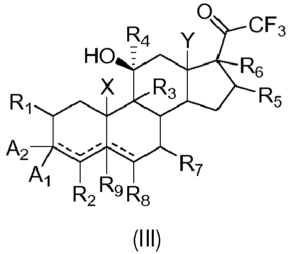 or a salt thereof, wherein:
   a dashed line represents an optional double bond or a methylene group attached to the atoms on either side such that a fused cyclopropyl ring results, provided that cumulated double bonds (=C=) are not allowed;
   n is an integer from 1 to 6;
   A₁ is selected from the group consisting of hydrogen, deuterium, halogen, -OH, PO₄³⁻ , -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁;
   A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom in the said heteroalkyl or heteroaryl ring is selected from the group of N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀,-NR₁₁COR₁₀, -OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
   R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 4-7 membered cycloalkyl, 5-7 membered heterocycloalkyl, and 5-6 membered heteroaryl, wherein heteroatom in the said heteroalkyl or heteroaryl ring is selected from the group of N, O or S; any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀, - NR₁₁CONR₁₀R₁₁, -COR₁₀, -COOR₁₀, and -CONR₁₀R₁₁;
   R₃ is selected from the group consisting of hydrogen, fluorine, chlorine, methyl, and -OR₁₀;
   R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
   R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
   R₈ and Rg are each selected from hydrogen, hydroxy, methoxy, amines, alkyl or acyl.
   R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
   R₁₂ and R₁₃ are each independently selected from the group consisting of hydrogen, C₁-C₆ alkyl, C₁-C₆ haloalkyl, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
   R₁₄ and R₁₅ are each independently selected from the group consisting of hydrogen and C₁-C₆ alkyl;
   R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl; Q is selected from the group consisting of: 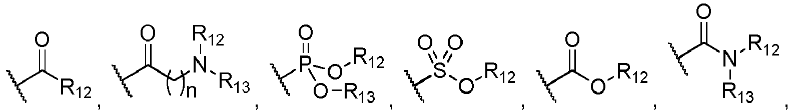 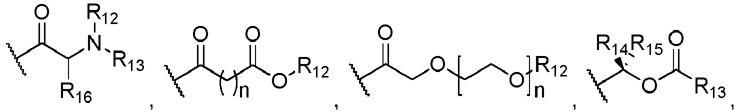 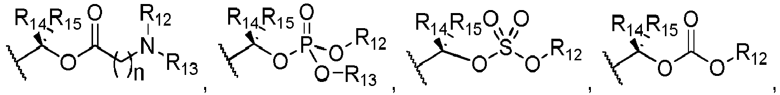 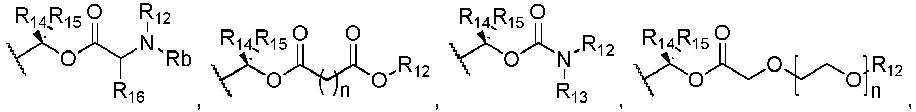 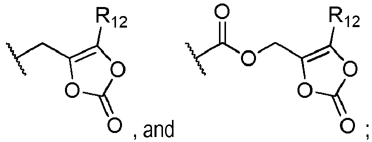 and
   X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀, - NR₁₁CONR₁₀R₁₁, -COR₁₀, -COOR₁₀, and -CONR₁₀R₁₁.
Clause 4. The compound as claused in clause 1, having structural Formula IV: 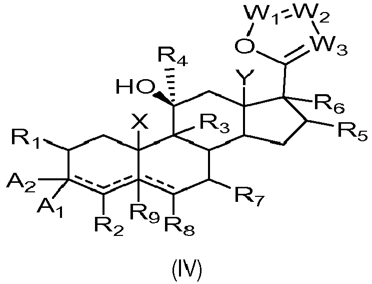 or a salt thereof, wherein:
   a dashed line represents an optional double bond or a methylene group attached to the atoms on either side such that a fused cyclopropyl ring results, provided that cumulated double bonds (=C=) are not allowed;
   n is an integer from 1 to 6;
   A₁ is selected from the group consisting of hydrogen, deuterium, halogen, -OH, PO₄³⁻ , -SO₄²⁻, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀, -COOR₁₀, and-CONR₁₀R₁₁,;
   A₂ is selected from the group consisting of hydrogen, deuterium, halogen, PO₄³⁻, -SO₄²⁻, C₁-C₁₂ alkyl, 3-7 membered cycloalkyl, 4-7 membered heterocycloalkyl, and 5-6 membered heteroaryl, wherein heteroatom of the heterocylic ring or heteroaryl ring is selected from N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀,-OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
   R₁ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀, -COR₁₀, -COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₂ is selected from the group consisting of hydrogen, fluorine, chlorine, -CF₃, -OR₁₀, -NR₁₀R₁₁, -OQ, -NR₁₀Q,-NR₁₁COR₁₀, -OCOOR₁₀, -NR₁₁COOR₁₀, -NR₁₁CONR₁₀R₁₁, -COR₁₀, -COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
   R₃ is selected from the group consisting of hydrogen, fluorine, chlorine, methyl, and -OR₁₀;
   R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
   R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀, -NR₁₁CONR₁₀R₁₁, -COR₁₀, -COOR₁₀,-CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
   R₈ and Rg are each selected from hydrogen, hydroxy, methoxy, amines, alkyl or acyl.
   R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀,-OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
   R₁₂ and R₁₃ are each independently selected from the group consisting of hydrogen, C₁-C₆ alkyl, C₁-C₆ haloalkyl, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
   R₁₄ and R₁₅ are each independently selected from the group consisting of hydrogen and C₁-C₆ alkyl;
   R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl), -CONH₂, and phenyl, wherein said phenyl may be optionally substituted with one or more substituents selected from the group consisting of halo and hydroxyl;
   Q is selected from the group consisting of: 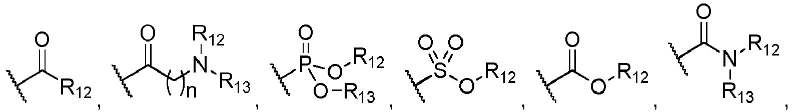 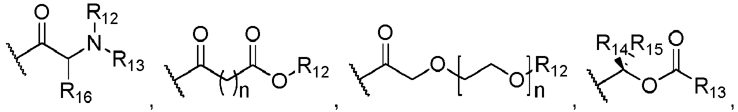 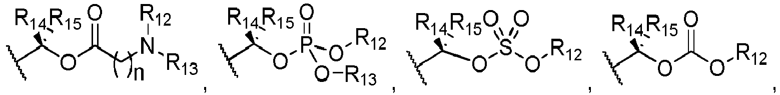 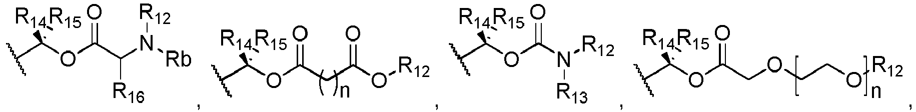 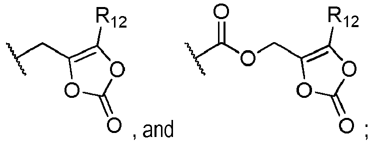
   W₁, W₂ and W₃ are each independently selected from the group consisting of CH₂, CH, C, NH, N, NR₁₀, NQ, and O; and
   X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀, -NR₁₁COR₁₀, -OQ, -NR₁₀Q, -OCOOR₁₀, -NR₁₁COOR₁₀,-NR₁₁CONR₁₀R₁₁, -COR₁₀, -COOR₁₀, and -CONR₁₀R₁₁.
Clause 5. The compound as claused in clause 1, having structural Formula V: 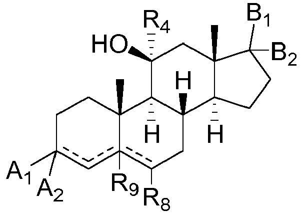 or a salt thereof, wherein:
   a dashed line represents an optional double bond or a methylene group attached to the atoms on either side such that a fused cyclopropyl ring results, provided that cumulated double bonds (=C=) are not allowed; and it is understood by a person skilled in the art that the atoms are placed inside the molecule such that their valency is suitably satisfied;
   A₁ and A₂ are each independently selected from the group consisting of hydrogen, deuterium, halogen, C₁-C₃ alkyl, hydroxy, C₁-C₃ alkoxy, -NH₂, PO₄³⁻ or -SO4²⁻;
   or A₁ and A₂, taken together, is selected from the group consisting of carbonyl or Oxime; or A₁ and A₂, taken together with the atom to which they are attached, may form a 3-7 membered cycloalkyl, 4-7 membered heterocycloalkyl comprising 1-3 heteroatoms selected from O or N, or 5-6 membered heteroaryl comprising 1-3 hereoatoms; wherein the heteroatom is selected from the group consisting of O, N and S.
   R₁ and R₂ are each independently selected from the group consisting of hydrogen, deuterium, halogens, hydroxy, C₁-C₃ alkyl, C₁-C₃ alkoxy or -NH₂; or R₁ and R₂ taken together may be selected from an oxo group or an oxime group.
   R₃ is selected from the group consisting of hydrogen, fluorine, chlorine, methyl, and -OR₁₀;
   R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
   B₁ and B₂ are each independently selected from the group consisting of hydrogen, deuterium, an -NH₂, a carbonyl, a C₁₋₅ alkoxy, -OH, -OR₃, -COR₃, -CONR₃R₄, -CONH(CH₂)ₙNH₂, -C(R₃)=N-OH or -C(R₃)=N-NH₂; or a C₁₋₅alkyl optionally substituted with one or more substituents selected from the group consisting of an alkyl, amino, a hydroxyalkyl, a carbonyl group, a C₁₋₅alkoxy, -OH, -OR₃, -COR₃, -CONR₃R₄, -CONH(CH₂)ₙNH₂,-C(R₃)=N-OH or-NHCOCH₂NH₂; or B₁ is a 3-7 membered cycloalkyl or 4-7 membered heterocycloalkyl or heterocycloalkenyl group comprising 1-3 heteroatoms selected from O or N, or 5-6 membered heteroaryl comprising 1-3 hereoatoms selected from the group consisting of O, N and S; any of which may be optionally substituted with one or more C₁-C₃ alkyl or a carbonyl group; or B₁ and B₂, taken together, is an oxo or an oxime group; or B₁ and B₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of hydrogen, -NH₂, hydroxyl, carbonyl, C₁₋₅ alkyl or a C₁₋₅ alkoxy group;
   R₈ and R₉ are each selected from hydrogen, hydroxy, methoxy, amines, alkyl or acyl.
   With the proviso that the 11^{th} position is β in configuration.
Clause 6. The compound as claused in clause 1, wherein the compound is selected from the group consisting of
   i. (3S,8S,9S,10R,11S,13S,14S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol;
   ii. 1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethanone
   iii. (3S,8S,9S,10R,11S,13S,14S,17S)-17-(1-hydroxyethyl)-10,13-dimethyl-2,3,4,7,8,9,10,11,12,13,14,15,16,17-tetradecahydro-1H-cyclopenta[a]phenanthrene-3,11-diol
   iv. (3S,8S,9S,10R,11S,13S,14S,17S)-methyl 3,11-dihydroxy-10,13-dimethyl-2,3,4,7,8,9,10,11,12,13,14,15,16,17-tetradecahydro-1H-cyclopenta[a]phenanthrene-17-carboxylate
   v. (8S,9S,10R,11S,13S,14S,17S)-methyl 11-hydroxy-10,13-dimethyl-3-oxo-2,3,6,7,8,9,10,11,12,13,14,15,16,17-tetradecahydro-1H-cyclopenta[a]phenanthrene-17-carboxylate
   vi. 1-((3S,8S,9S,10R,11S,13S,14S,17S)-3,11-dihydroxy-10,13-dimethyl-2,3,6,7,8,9,10,11,12,13,14,15,16,17-tetradecahydro-1H-cyclopenta[a]phenanthren-17-yl)ethanone
   vii. (8S,9S,10R,11S,13S,14S,17S)-17-acetyl-11-hydroxy-10,13-dimethyl-6,7,8,9,10,11,12,13,14,15,16,17-dodecahydro-1H-cyclopenta[a]phenanthren-3(2H)-one
   viii. 1-((3S,8S,9S,10S,11S,13S,14S,17S)-3,11-dihydroxy-10,13-dimethylhexadecahydro-1H-cyclopenta[a]phenanthren-17-yl)ethanone
   ix. (3S,8S,9S,10R,11S,13R,14S,17S)-17-ethyl-10,13-dimethyl-2,3,4,7,8,9,10,11,12,13,14,15,16,17-tetradecahydro-1H-cyclopenta[a]phenanthrene-3,11-diol
   x. (8S,9S,10R,11S,13S,14S,17R)-17-((R)-1,2-dihydroxyethyl)-11,17-dihydroxy-10,13-dimethyl-6,7,8,9,10,11,12,13,14,15,16,17-dodecahydro-1H-cyclopenta[a]phenanthren-3(2H)-one
   xi. (8S,9S,10R,11 S,13S,14S)-11-hydroxy-10,13-dimethyl-7,8,9,10,11,12,13,14,15,16-decahydro-1H-cyclopenta[a]phenanthrene-3,17(2H,6H)-dione
   xii. (8S,9S,10R,11S,13S,14S,17S)-11-hydroxy-10,13-dimethyl-17-(3-methyl-1,2,4-oxadiazol-5-yl)-6,7,8,9,10,11,12,13,14,15,16,17-dodecahydro-1H-cyclopenta[a]phenanthren-3(2H)-one;
   xiii. Trimethylammonium (3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl sulfate;
   xiv. (8S,9S,10R,11S,13S,14S,17S)-11-hydroxy-17-(2-hydroxyacetyl)-10,13-dimethyl-6,7,8,9,10,11,12,13,14,15,16,17-dodecahydro-1H-cyclopenta[a]phenanthren-3(2H)-one;
   xv. (8S,9S,10R,11 S,13S,14S,17R)-11,17-dihydroxy-17-(2-hydroxyacetyl)-10,13-dimethyl-6,7,8,9,10,11,12,13,14,15,16,17-dodecahydro-1H-cyclopenta[a]phenanthren-3(2H)-one;
   xvi. 1-((3S,10R,11S,13S,17S)-3,11-dihydroxy-1 0,13-dimethyl-2,3,4,7,8,9,10,11,12,13,14,15,16,17-tetradecahydro-1H-cyclopenta[a]phenanthren-17-yl)ethan-1-one;
   xvii. (3S,10R,11S,13S,17S)-10,13-dimethyl-2,3,4,7,8,9,10,11,12,13,14,15,16,17-tetradecahydro-1H-cyclopenta[a]phenanthrene-3,11,17 -triol;
   xviii. (10R,11S,13S,17S)-11,17-dihydroxy-10,13-dimethyl-1,2,6,7,8,9,10,11,12,13,14,15,16,17-tetradecahydro-3H-cyclopenta[a]phenanthren-3-one;
   xix. (10R,11S,13S)-11-hydroxy-10,13-dimethyl-1,6,7,8,9,10,11,12,13,14,15,16-dodecahydro-3H-cyclopenta[a]phenanthrene-3,17(2H)-dione;
   xx. (10R,11S,13S)-11-hydroxy-10,13-dimethyl-3-oxo-2,3,6,7,8,9,10,11,12,13,14,15,16,17-tetradecahydro-1H-cyclopenta[a]phenanthrene-17-carboxylic acid;
   xxi. (10R,11S,13S)-17-(1-hydroxyethyl)-10,13-dimethyl-1,2,4,7,8,9,10,11,12,13,14,15,16,17-tetradecahydrospiro[cyclopenta[a]phenanthrene-3,2'-[1,3]dioxolan]-11-ol;
   xxii. (3Z,10R,11S,13S,17E)-11-hydroxy-10,13-dimethy!-1,6,7,8,9,10,11,12,13,14,15,16-dodecahydro-3H-cyclopenta[a]phenanthrene-3,17(2H)-dione dioxime;
   xxiii. (10R,11S,13S,17R)-11,17-dihydroxy-10,13-dimethyl-3-oxo-2,3,6,7,8,9,10,11,12,13,14,15,16,17-tetradecahydro-1H-cyclopenta[a]phenanthrene-17-carboxylic acid;
   xxiv. (10R,11S,13S,E)-11-hydroxy-17-(hydroxyimino)-10,13-dimethyl-1,2,6,7,8,9,10,11,12,13,14,15,16,17-tetradecahydro-3H-cyclopenta[a]phenanthren-3-one;
   xxv. (10R,11S,13S,17S)-17-acetyl-11-hydroxy-10,13-dimethyl-1,2,6,7,8,9,10,11,12,13,14,15,16,17-tetradecahydro-3H-cyclopenta[a]phenanthren-3-one;
   xxvi. (10S,11S,13S,17S)-17-acetyl-11-hydroxy-10,13-dimethylhexadecahydro-3H-cyclopenta[a] phenanthren-3-one;
   xxvii. (3S,10R,11S,13S,17S)-10,13-dimethyl-2,3,6,7,8,9,10,11,12,13,14,15,16,17-tetradecahydro-1H-cyclopenta[a]phenanthrene-3,11,17 -triol;
   xxviii. (10R,11S,13S,17R)-11-hydroxy-10,13-dimethyl-1,6,7,8,9,10,11,12,13,14,15,16-dodecahydrospiro[cyclopenta[a]phenanthrene-17,2'-oxetane]-3,3'(2H)-dione;
   xxix. 1-((10R,11S,13S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one;
   xxx. (10R,11S,13S)-11-hydroxy-N,N,10,13-tetramethyl-3-oxo-2,3,6,7,8,9,10,11,12,13,14,15,16,17-tetradecahydro-1H-cyclopenta[a]phenanthrene-17-carboxamide;
   xxxi. (10R,11S,13S,17S,Z)-11-hydroxy-17-((Z)-1-(hydroxyimino)ethyl)-10,13-dimethyl-1,2,6,7,8,9,10,11,12,13,14,15,16,17-tetradecahydro-3H-cyclopenta[a]phenanthren-3-one oxime;
   xxxii. (10R,11S,13S)-11-hydroxy-17-(1-hydroxyethyl)-10,13-dimethyl-1,2,6,7,8,9,10,11,12,13,14,15,16,17-tetradecahydro-3H-cyclopenta[a]phenanthren-3-one;
   xxxiii. (10S,11S,13S)-11-hydroxy-N,N,10,13-tetramethyl-3-oxohexadecahydro-1H-cyclopenta[a]phenanthrene-17 -carboxamide;
   xxxiv. (10S,11S,13S)-11-hydroxy-10,13-dimethyltetradecahydro-3H-cyclopenta[a]phenanthrene-3,17(2H)-dione;
   xxxv. (10R,11S,13S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one;
   xxxvi. (3S,10R,11S,13S)-17-(1-hydroxyethyl)-10,13-dimethyl-2,3,4,7,8,9,10,11,12,13,14,15,16,17-tetradecahydro-1H-cyclopenta[a]phenanthrene-3,11-diol;
   xxxvii. (3S,8S,9S,10R,11S, 13S,14S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol;
   xxxviii. 1-((3S,10R,11S,13S,17S)-11-hydroxy-3-methoxy-10,13-dimethyl-2,3,4,7,8,9,10,11,12,13,14,15,16,17-tetradecahydro-1H-cyclopenta[a]phenanthren-17-yl)ethan-1-one;
   xxxix. (10S,11S,13S)-17-(1-hydroxyethyl)-10,13-dimethylhexadecahydrospiro[cyclopenta[a]phenanthrene-3,2'-[1,3]dioxolan]-11-ol;
   xl. (3S,10S,11S,13S,17S)-17-(1-hydroxyethyl)-10,13-dimethylhexadecahydro-1H-cyclopenta[a]phenanthrene-3,11-diol;
   xli. (3S,10R,11S,13R,17S)-17-ethyl-10,13-dimethyl-2,3,4,7,8,9,10,11,12,13,14,15,16,17-tetradecahydro-1H-cyclopenta[a]phenanthrene-3,11-diol;
   xlii. 1-((3S,10S,11S,13S,17S)-3,11-dihydroxy-10,13-dimethylhexadecahydro-1H-cyclopenta[a]phenanthren-17-yl)ethan-1-one;
   xliii. (3S,10R,11S,13S,17S)-3,11-dihydroxy-N,N,10,13-tetramethyl-2,3,4,7,8,9,10,11,12,13,14,15,16,17-tetradecahydro-1H-cyclopenta[a]phenanthrene-17-carboxamide;
   xliv. 1-((8S,9S,10R,11S,13S,14S,17S)-11-hydroxy-10,13-dimethyl-2,7,8,9,10,11,12,13,14,15,16,17-dodecahydro-1H-cyclopenta[a]phenanthren-17-yl)ethan-1-one;
   xlv. 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3-d)ethan-1-one;
   xlvi. (3S,10R,11S,13S,17S)-3,11-dihydroxy-N,N,10,13-tetramethyl-2,3,6,7,8,9,10,11,12,13,14,15,16,17-tetradecahydro-1H-cyclopenta[a]phenanthrene-17-carboxamide;
   xlvii. 1-((3S,10R,11S,13S,17S)-3,11-dihydroxy-10,11,13-trimethyl-2,3,4,7,8,9,10,11,12,13,14,15,16,17-tetradecahydro-1H-cyclopenta[a]phenanthren-17-yl)ethan-1-one;
   xlviii. (10R,11S,13S)-11-hydroxy-17-(2-hydroxyacetyl)-10,13-dimethyl-1,2,6,7,8,9,10,11,12,13,14,15,16,17-tetradecahydro-3H-cyclopenta[a]phenanthren-3-one;
   xlix. (10R,11S,13S,17S)-11-hydroxy-17-(2-hydroxypropan-2-yl)-1 0,13-dimethyl-1,2,6,7,8,9,10,11,12,13,14,15,16,17-tetradecahydro-3H-cyclopenta[a]phenanthren-3-one;
   l. (E)-1-((3S,10R,11S,13S)-3,11-dihydroxy-10,13-dimethyl-2,3,4,7,8,9,10,11,12,13,14,15,16,17-tetradecahydro-1H-cyclopenta[a]phenanthren-17-yl)ethan-1-one oxime;
   li. 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-11-d)ethan-1-one;
   lii. (3S,10R,11S,13S,17S)-17-((E)-1-hydrazonoethyl)-10,13-dimethyl-2,3,4,7,8,9,10,11,12,13,14,15,16,17-tetradecahydro-1H-cyclopenta[a]phenanthrene-3,11-diol;
   liii. 1-((10S,11S,13S,17S)-11-hydroxy-10,13-dimethylhexadecahydro-1H-cyclopenta[a]phenanthren-17-yl)ethan-1-one;
   liv. (3S,10S,11S,13S)-17-(1-aminoethyl)-10,13-dimethylhexadecahydro-1H-cyclopenta[a]phenanthrene-3,11-diol;
   Iv. 1-((3R,10R,11S,13S,17S)-3-fluoro-11-hydroxy-10,13-dimethyl-2,3,4,7,8,9,10,11,12,13,14,15,16,17-tetradecahydro-1H-cyclopenta[a]phenanthren-17-yl)ethan-1-one;
   Ivi. (3S,10S,11S,13S)-17-amino-10,13-dimethylhexadecahydro-1H-cyclopenta[a]phenanthrene-3,11-diol;
   Ivii. (10R,11S,13S)-11-hydroxy-10,13-dimethyl-17-(piperazine-1-carbonyl)-1,2,6,7,8,9,10,11,12,13,14,15,16,17-tetradecahydro-3H-cyclopenta[a]phenanthren-3-one;
   Iviii. (8S,9S,10R,11S,13S,14S,17S)-17-acetyl-11-hydroxy-10,11,13-trimethyl-1,2,6,7,8,9,10,11,12,13,14,15,16,17-tetradecahydro-3H-cyclopenta[a]phenanthren-3-one;
   lix. (8S,9S,10R,11S,13S,14S,17S)-N-(2-aminoethyl)-11-hydroxy-10,13-dimethyl-3-oxo-2,3,6,7,8,9,10,11,12,13,14,15,16-tetradecahydro-1H-cyclopenta[a]phenanthrene-17-carboxamide;
   lx. 1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-3,11-d2)ethan-1-one;
   lxi. (3S,8S,9S,10S,11S,13S,14S,17S)-3,11-dihydroxy-N,10,13-trimethylhexadecahydro-1H-cyclopenta[a]phenanthrene-17 -carboxamide;
   Ixii. (3S,10R,11S,13S,17S)-2,3,6,7,8,9,10,11,12,13,14,15,16,17-tetradecahydro-10,13-dimethyl-1H-cyclopenta[a]phenanthrene-3,11,17 -triol;
   lxiii. (3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide;
   lxiv. (3S,10R,11S,13S,17S)-5-methoxy-10,13-dimethyl-17-(2-methyl-1,3-dioxolan-2-yl)hexadecahydro-1H-cyclopenta[a]phenanthrene-3,6,11-triol;
   Ixv. 1-((3S,5R,6R,10R,11S,13S,17S)-3,5,11-trihydroxy-6-methoxy-10,13-dimethylhexadecahydro-1H-cyclopenta[a]phenanthren-17-yl)ethan-1-one;
   lxvi. 1-((3S,5R,6R,10R,11S,13S,17S)-3,5,11-trihydroxy-6-methoxy-10,13-dimethylhexadecahydro-1H-cyclopenta[a]phenanthren-17-yl)ethan-1-one;
   Ixvii. 1-((3S,5R,6R,10R,11S,13S,17S)-3,5,6,11-tetrahydroxy-10,13-dimethylhexadecahydro-1H-cyclopenta[a]phenanthren-17-yl)ethan-1-one;
   lxviii. 3S,10R,11S,13S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol;
Clause 7. A pharmaceutical composition comprising a compound as claused in clause 1 together with a pharmaceutically acceptable carrier.
Clause 8. Use of the compounds as claused in clauses 1 to 6 for the activation AMPK.
Clause 9. Use of the compounds as claused in clauses 1 to 6 for the subsequent activation transcription factors such as PCG-1 alpha associated with mitochondrial biogenisis.
Clause 10. Use of the compounds as claused in clauses 1 to 6 for the activation mitochondrial biogenisis and functions.
Clause 11. Use of the compounds as claused in clauses 1 to 6 for treatment of diseases associated with mitochondrial depletion and/ or dysfunctionselected, but not limited to, the group consisting of skeletal muscle diseases, cardiac muscle diseases associated with ischemia, or impaired or inadequate blood flow, diseases associated with genetic disorders that directly or indirectly affect the number, structure, or function of mitochondria, diseases associated with impaired neurological function associated with decreased mitochondrial number or function, diseases associated with loss of number, loss of function, or loss of correct, optimally efficient internal organization of skeletal muscle cells or cardiac muscle cells, metabolic diseases, and conditions associated with liver cell injury and altered fatty acid metabolism.
Clause 12. Use of the compounds as claused in clauses 1 to 6 for mitochondrial biogenesis-mediated disease is selected from the group consisting of acute coronary syndrome, myocardial infarction, angina, renal injury, renal ischemia, diseases of the aorta and its branches, injuries arising from medical interventions, atherosclerosis, trauma, diabetes, hyperlipidemia, vascular stenosis, peripheral arterial disease, vasculopathy, and vasculitis, Friedreich's ataxia, muscular dystrophy, Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, spinal muscular atrophy, Emery-Dreifuss muscular dystrophy, Huntington's disease, Parkinson's disease, Alzheimer's disease, and amyotrophic lateral sclerosis, sarcopenia, congestive heart failure, aging, myocarditis, myositis, polymyalgia rheumatic, polymyositis, HIV, cancer and/or the side effects of chemotherapy targeting the cancer, malnutrition, aging, inborn errors of metabolism, trauma, and stroke or other types of neurological impairment, hepatic steatosis, hepatic fibrosis, cirrhosis, and hepatocyte or stellate cell injury.
Clause 13. Use of the compounds as claused in clauses 1 to 6 in a dosage in the range of 0.01 to 100mg/Kg.
Clause 14. The compounds as claused in clauses 1 to 6 when administered as orally or parenterally.
Clause 15. A method of manufacturing the compound as claused in clause 1, as described herein.

## Claims

1. A compound of structural Formula I: 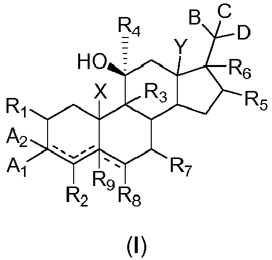 or a salt thereof, wherein:
a dashed line represents an optional bond or a methylene group attached to the atoms on either side such that a fused cyclopropyl ring results, provided that cumulated double bonds (=C=) are not allowed;
n is an integer from 1 to 6;
A₁ is selected from the group consisting of hydrogen, deuterium, halogen, -OH, PO₄³⁻, -SO₄²⁻, -OR₁₀, -NR₁₀R₁₁, -OQ, -NR₁₀Q, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
A₂ is selected from the group consisting of hydrogen, deuterium, C₁-C₁₂ alkyl, 3-7 membered cycloalkyl, 4-7 membered heterocycloalkyl, and 5-6 membered heteroaryl, wherein heteroatom of the heterocylic ring or heteroaryl ring is selected from N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀, -OCOOR₁₀,-NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀, -OCOOR₁₀, -NR₁₁COOR₁₀, -NR₁₁CONR₁₀;-COR₁₀, -COOR₁₀, and -CONR₁₀R₁₁; or A₁ and A₂, taken together, are =O or oxime (=O-NH);
B and C are each independently selected from the group consisting of hydrogen, -OH, -OR₁₀, -NR₁₀R₁₁, -OQ, -NR₁₀Q, -NR₁₁COR₁, -OCOOR₁, -NR₁₁COOR₁₀, -NR₁₁CONR₁₀;-COR₁₀, -COOR₁₀,-CONR₁₀R₁₁, ,-NH(CH₂)ₙNH_{2;}, -NRₙCO(CH₂)ₙNH₂ and C₁-C₁₂ alkyl, wherein said C₁-C₁₂ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁; or B and C, taken together with the atom to which they are attached, may form a 3-7 membered cycloalkyl, 4-7 membered heterocycloalkyl, or 5-6 membered heteroaryl, wherein heteroatom of the heterocylic ring or heteroaryl ring is selected from N, O or S;, any of which may be optionally substituted with one or more C₁-C₁₂ alkyl; wherein said C₁-C₁₂ alkyl may be optionally substituted with one or more substituents selected from the group consisting of fluorine, chlorine, -OR₁₀, -OCOR₁₀,-NR₁₁COR₁₀, -OCOOR₁₀, -NR₂COOR₁₀, -NR₁₁CONR₁₀R₁₁, -COR₁₀, -COOR₁₀, and-CONR₁₀R₁₁; or B and C, taken together, are =O, =N-OH or =N-NH₂;
D is selected from the group consisting of hydrogen, hydroxyl, methyl, ethyl, propyl, isopropyl, cyclopropyl, amino, hydroxyalkyl, -CD₃, -CF₃, -OR₁₀, -OCF₃, phenyl, 4-6 membered heterocycloalkyl, and 5-6 membered heteroaryl; wherein heteroatom in the said heteroalkyl or heteroaryl ring is selected from the group of N, O or S; wherein said 5-6 membered heterocycloalkyl or 5-6 membered heteroaryl may be optionally substituted with one or more substituents selected from the group consisting of C₁-C₄ alkyl, -R₁₀, -OR₁₀, -NR₁₀R₁₁, fluorine, chlorine, and -CF₃ or a C=O group ;
Or when taken together with B or C, may represent a double bond, or B, C and D taken together with the atom to which they are attached may form a carbonyl group, oximegroup, phenyl, 4-6 membered heterocycloalkyl, or 5-6 membered heteroaryl; any of which may be optionally substituted with one or more substituents selected from the group consisting of -C₁-C₄ alkyl, -R₁₀, -OR₁₀, -NR₁₀R₁₁, fluorine, chlorine, [[=O]]-C=O and -CF₃;
R₁ is selected from the group consisting of hydrogen, fluorine, chlorine, -CF₃, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀, -COR₁₀,-COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 5-7 membered cycloalkyl, 5-7 membered heterocycloalkyl, and 5-6 membered heteroaryl, any of which may be optionally substituted with one or more substituents selected from the group consisting of fluorine, chlorine, -R₁₀,-OR₁₀, -OCOR₁₀, -NR₁₁COR₁₀, -OCOOR₁₀, -NR₁₁COOR₁₀, -NR₁₁CONR₁₀R₁₁, -COR₁₀,-COOR₁₀, and -CONR₁₀R₁₁;
R₂ is selected from the group consisting of hydrogen, fluorine, chlorine, -CF₃, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀,-COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
R₃ is selected from the group consisting of hydrogen, fluorine, chlorine, methyl, and-OR₁₀;
R₄ is selected from the group consisting of hydrogen, deuterium, and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀,-NR₁₁CONR₁₀R₁₁, -COR₁₀, -COOR₁₀, -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₈ and R₉ are each hydrogen, hydroxy, methoxy, amines, alkyl or acyl.
R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, amino, substituted amino, phenyl, and phenymethyl, wherein said phenyl group or the phenyl portion of the phenylmethyl group may be optionally substituted with one or more substituents selected from the group consisting of halogen, methoxy, trifluormethoxy, and amino; and, R₁₂ and R₁₃ taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl),-CONH₂, and phenyl, wherein said phenyl may be optionally substituted with one or more substituents selected from the group consisting of halo and hydroxyl;
Q is selected from the group consisting of: 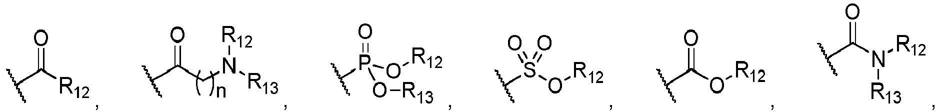 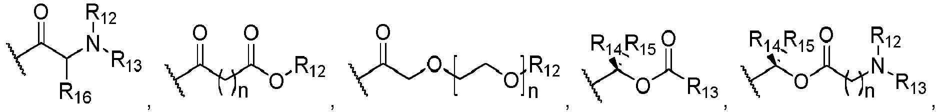 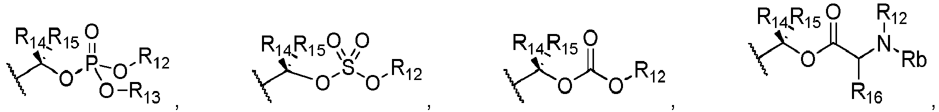 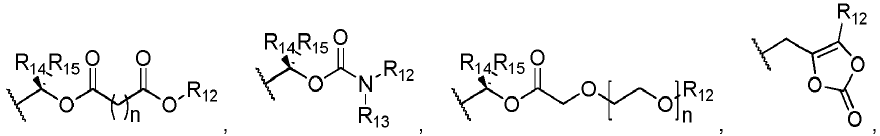 and 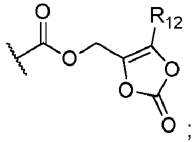 and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀,-NR₁₁COR₁₀, -OQ, -NR₁₀Q, -OCOOR₁₀, -NR₁₁COOR₁₀, -NR₁₁CONR₁₀R₁₁, -COR₁₀, -COOR₁₀, and -CONR₁₀R₁₁.
Provided that when A₁ and A₂ form =O and R6 is hydrogen or hydroxyl be acetyl. 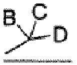 cannot

2. The compound as claimed in claim 1, having structural Formula III: 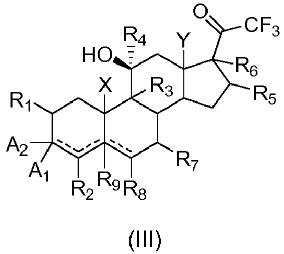 or a salt thereof, wherein:
a dashed line represents an optional double bond or a methylene group attached to the atoms on either side such that a fused cyclopropyl ring results, provided that cumulated double bonds (=C=) are not allowed;
n is an integer from 1 to 6;
A₁ is selected from the group consisting of hydrogen, deuterium, halogen, -OH, PO₄³⁻, -SO₄²⁻, -OR₁₀, -NR₁₀R₁₁, -OQ, -NR₁₀Q, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
A₂ is selected from the group consisting of hydrogen, deuterium, halogen, PO₄³⁻,-S0₄²⁻, C₁-C₁₂ alkyl, 3-7 membered cycloalkyl, 4-7 membered heterocycloalkyl, and 5-6 membered heteroaryl, wherein heteroatom in the said heteroalkyl or heteroaryl ring is selected from the group of N, O or S; wherein said 3-7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀,-NR₁₁COR₁₀, -OCOOR₁₀, -NR₁₁COOR₁₀, -NR₁₁CONR₁₀R₁₁, -COR₁₀, -COOR₁₀, -CONR₁₀R₁₁; or A₁ and A₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of fluorine, chlorine, -R₁₀, -OR₁₀, -OCOR₁, -NR₁₁COR₁₀, -OCOOR₁₀, -NR₁₁COOR₁₀, -NR₁₁CONR₁₀; -COR₁₀, -COOR₁₀, and -CONR₁₀R₁₁; or A₁ and A₂, taken together, are =O;
R₁ is selected from the group consisting of hydrogen, fluorine, chlorine, -CF₃, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀, -COR₁₀,-COOR₁₀, or -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁; or R₁ and A₁ or R₁ and A₂, taken together with the atoms to which they are attached, can form a 4-7 membered cycloalkyl, 5-7 membered heterocycloalkyl, and 5-6 membered heteroaryl, wherein heteroatom in the said heteroalkyl or heteroaryl ring is selected from the group of N, O or S; any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR₁₀R₁₁;
R₂ is selected from the group consisting of hydrogen, fluorine, chlorine, -CF₃, -OR₁₀,-NR₁₀R₁₁, -OQ, -NR₁₀Q, -NR₁₁COR₁₀, -OCOOR₁₀, -NR₁₁COOR₁₀, -NR₁₁CONR₁₀R₁₁, -COR₁₀,-COOR₁₀, - CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀, -NR₁₁CONR₁₀R₁₁,-COR₁₀, -COOR₁₀, and -CONR-₁₀R₁₁; or R₂ and A₁ or R₂ and A₂, taken together with the atoms to which they are attached, can form 5-7 membered cycloalkyl, 5-7 membered heterocycloalkyl, or 5-6 membered heteroaryl, any of which may be optionally substituted with one or more substituents selected from the group consisting of fluorine, chlorine, -R₁₀, -OR₁₀, -OCOR₁₀, -NR₁₁COR₁₀, -OCOOR₁₀, -NR₁₁COOR₁₀, -NR₁₁CONR₁₀R₁₁, -COR₁₀, -COOR₁₀, and -CONR₁₀R₁₁;
R₃ is selected from the group consisting of hydrogen, fluorine, chlorine, methyl, and-OR₁₀;
R₄ is selected from the group consisting of hydrogen, hydroxy and C₁-C₆ alkyl, wherein said C₁-C₆ alkyl may be optionally substituted with one or more substituents selected from the group consisting of fluorine, chlorine, and -OR₁₀;
R₅, R₆ and R₇ are each independently selected from hydrogen, fluorine, chlorine, -CF₃, -OH, -OR₁₀, -OQ, NR₁₀R₁₁, -NR₁₀Q, -NR₁₀Q, -NR₁₁COR₁₀, -OCOOR₁₀, -NR₁₁COOR₁₀,-NR₁₁CONR₁₀R₁₁, -COR₁₀, -COOR₁₀, -CONR₁₀R₁₁, and C₁-C₆ alkyl, wherein said C₁-C₆ alkyl may be optionally substituted with one or more substituents selected from the group consisting of fluorine, chlorine, -OR₁₀, -OCOR₁₀, -NR₁₁COR₁₀, -OCOOR₁₀, -NR₁₁COOR₁₀,-NR₁₁CONR₁₀R₁₁, -COR₁₀, -COOR₁₀, and -CONR₁₀R₁₁;
R₈ and R₉ are each selected from hydrogen, hydroxy, methoxy, amines, alkyl or acyl.
R₁₀ and R₁₁ are each independently selected from the group consisting of hydrogen, C₁-C₁₂ alkyl, 3-7 membered cycloalkyl, 4-7 membered heterocycloalkyl, and 5-6 membered heteroaryl, wherein said -7 membered cycloalkyl, 4-7 membered heterocycloalkyl, or 5-6 membered heteroaryl may be optionally substituted with one or more substituents selected from the group consisting of fluorine, chlorine, -R₁₀, -OR₁₀, -OCOR₁₀, -NR₁₁COR₁₀,-OCOOR₁₀, -NR₁₁COOR₁₀, -NR₁₁CONR₁₀R₁₁, -COR₁₀, -COOR₁₀, and -CONR₁₀R₁₁;
R₁₂ and R₁₃ are each independently selected from the group consisting of hydrogen, C₁-C₆ alkyl, C₁-C₆ haloalkyl, phenyl, and phenymethyl, wherein said phenyl group or the phenyl portion of the phenylmethyl group may be optionally substituted with one or more substituents selected from the group consisting of halogen, methoxy, trifluormethoxy, and amino; and R₁₄ and R₁₅, taken together with the atoms to which they are attached, form 5-7 membered cycloalkyl, 5-7 membered heterocycloalkyl, or 5-6 membered heteroaryl, any of which may be optionally substituted with one or more substituents selected from the group consisting of halo, methyl and methoxy;
R₁₄ and R₁₅ are each independently selected from the group consisting of hydrogen and C₁-C₆ alkyl;
R₁₆ is selected from the group consisting of hydrogen and C₁-C₆ alkyl, wherein said C₁-C₆ alkyl may be optionally substituted with one or more substituents selected from the group consisting of hydroxyl, methoxy, amino, thio, methylthio, -C(O)OH, -C(O)O-(C₁-C₃ alkyl),-CONH₂, and phenyl, wherein said phenyl may be optionally substituted with one or more substituents selected from the group consisting of halo and hydroxyl;
Q is selected from the group consisting of: 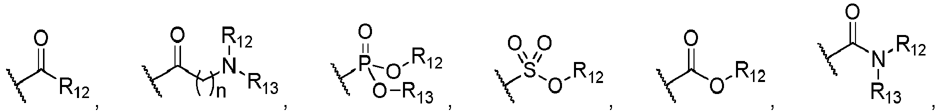 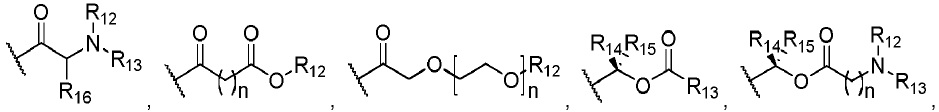 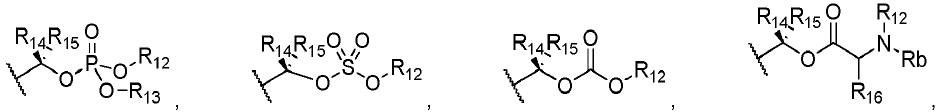 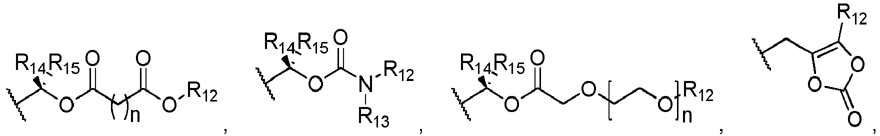 and 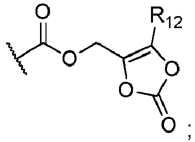 and
X and Y are each independently selected from the group consisting of hydrogen and C₁-C₁₂ alkyl, wherein said C₁-C₁₂ alkyl that may be optionally substituted with one or more substituents selected from the group consisting of fluorine, chlorine, -OR₁₀, -OCOR₁₀,-NR₁₁COR₁₀, -OQ, -NR₁₀Q, -OCOOR₁₀, -NR₁₁COOR₁₀, -NR₁₁CONR₁₀R₁₁, -COR₁₀, -COOR₁₀, and -CONR₁₀R₁₁.

3. The compound as claimed in claim 1, having structural Formula V: 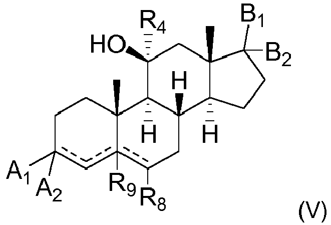 or a salt thereof, wherein:
a dashed line represents an optional double bond or a methylene group attached to the atoms on either side such that a fused cyclopropyl ring results, provided that cumulated double bonds (=C=) are not allowed; and it is understood by a person skilled in the art that the atoms are placed inside the molecule such that their valency is suitably satisfied;
n is an integer from 1 to 6;
A₁ and A₂ are each independently selected from the group consisting of hydrogen, deuterium, halogen, C₁-C₃ alkyl, hydroxy, C₁-C₃alkoxy, -NH₂, -OC(O)R₁₂orOPO₃³⁻ , OSO₃²⁻;
or A₁ and A₂, taken together, is O or oxime; or A₁ and A₂, taken together with the atom to which they are attached, may form a 3-7 membered cycloalkyl, 4-7 membered heterocycloalkyl comprising 1-3 heteroatoms selected from O or N, or 5-6 membered heteroaryl comprising 1-3 hereoatoms; wherein the heteroatom is selected from the group consisting of O, N and S.
R₃ is selected from the group consisting of hydrogen, fluorine, chlorine, methyl, and-OR₁₀;
R₄ is selected from the group consisting of hydrogen,- deuterium, hydroxy and C₁-C₆ alkyl, wherein said C₁-C₆ alkyl may be optionally substituted with one or more substituents selected from the group consisting of fluorine, chlorine, and -OR₁₀;
B₁ and B₂ are each independently selected from the group consisting of hydrogen, deuterium, NH₂, a C₁₋₅ alkoxy, -OH, -COR₃, -CONR₃R₄, -CONH(CH₂)ₙNH₂, -C(R₃)=N-OH or-C(R₃)=N-NH₂; or a C₁₋₅ alkyl optionally substituted with one or more substituents selected from the group consisting of an alkyl, amino, a hydroxyalkyl, a carbonyl group, a C₁₋₅ alkoxy,-OH, -COR₃, -CONR₃R₄, -CONH(CH₂)ₙNH₂, -C(R₃)=N-OH or -NHCOCH₂NH₂; or B₁ is a 3-7 membered cycloalkyl or 4-7 membered heterocycloalkyl or heterocycloalkenyl group comprising 1-3 heteroatoms selected from O or N, or 5-6 membered heteroaryl comprising 1-3 hereoatoms selected from the group consisting of O, N and S; any of which may be optionally substituted with one or more C₁-C₃ alkyl or a carbonyl group; or B₁ and B₂, taken together, is an oxo or an oxime group; or B₁ and B₂, taken together with the atom to which they are attached, may form a 3-7 membered cycloalkyl, 4-7 membered heterocycloalkyl, or 5-6 membered heteroaryl, any of which may be optionally substituted with one or more substituents selected from the group consisting of hydrogen, -NH₂, hydroxyl, carbonyl, C₁₋₅ alkyl and a C₁₋₅ alkoxy group;or B₁ and B₂ together with the carbon to which they are attached form a carbonyl;
R₈ and R₉ are each hydrogen, chlorine, fluorine, hydroxy, methoxy, amines, alkyl or acyl.
R₁₀ is hydrogen or C₁-C₁₂ alkyl; and
R₁₂ is hydrogen, C₁-C₆ alkyl, C₁-C₆haloalkyl, amino, substituted amino, phenyl, and phenymethyl, wherein said phenyl group or the phenyl portion of the phenylmethyl group may be optionally substituted with one or more substituted selected from the group consisting of halogen, methoxy, trifluormethoxy, and amino.

4. A compound being selected from the group consisting of
(3S,8S,9S,10R,11S,13S,14S,17S)-10,13-dimethyl-17-(2-methyl-1,3-dioxolan-2-yl)-2,3,4,7,8,9,10,11,12,13,14,15,16,17-tetradecahydro-1H-cyclopenta[a]phenanthrene-3,11-diol;
1-((3S,8S,9S,10R,11S,13S,14S,17S)-3,11-dihydroxy-10,13-dimethyl-2,3,4,7,8,9,10,11,12,13,14,15,16,17-tetradecahydro-1H-cyclopenta[a]phenanthren-17-yl)ethanone
(3S,8S,9S,10R,11S,13S,14S,17S)-17-(1-hydroxyethyl)-10,13-dimethyl-2,3,4,7,8,9,10,11, 12,13,14,15,16,17-tetradecahydro-1H-cyclopenta[a]phenanthrene-3,11-diol
(3S,8S,9S,10R,11S,13S,14S,17S)-methyl3,11-dihydroxy-10,13-dimethyl-2,3,4,7,8,9,10,11,12,13,14,15,16,17-tetradecahydro-1H-cyclopenta[a]phenanthrene-17-carboxylate
(8S,9S,10R,11S,13S,14S,17S)-methyl11-hydroxy-10,13-dimethyl-3-oxo-2,3,6,7,8,9,10,11,12,13,14,15,16,17-tetradecahydro-1H-cyclopenta[a]phenanthrene-17-carboxylate
1-((3S,8S,9S,10R,11S,13S,14S,17S)-3,11-dihydroxy-10,13-dimethyl-2,3,6,7,8,9,10,11,12,13,14,15,16,17-tetradecahydro-1H-cyclopenta[a]phenanthren-17-yl)ethanone
1-((3S,8S,9S,10S,11S,13S,14S,17S)-3,11-dihydroxy-10,13-dimethylhexadecahydro-1H-cyclopenta[a]phenanthren-17-yl)ethanone
(3S,8S,9S, 10R,11S,13R,14S, 17S)-17-ethyl-10,13-dimethyl-2,3,4,7,8,9,10,11,12,13,14, 15,16,17-tetradecahydro-1H-cyclopenta[a]phenanthrene-3,11-diol
(8S,9S,10R,11S,13S,14S,17R)-17-((R)-1,2-dihydroxyethyl)-11,17-dihydroxy-10,13-dimethyl-6,7,8,9,10,11,12,13,14,15,16,17-dodecahydro-1H-cyclopenta[a]phenanthren-3(2H)-one
(8S,9S,10R,11S,13S,14S)-11-hydroxy-10,13-dimethyl-7,8,9,10,11,12,13,14,15,16-decahydro-1H-cyclopenta[a]phenanthrene-3,17(2H,6H)-dione
(8S,9S,10R,11S,13S, 14S,17S)-11-hydroxy-10,13-dimethyl-17-(3-methyl-1,2,4-oxadiazol-5-yl)-6,7,8,9,10,11,12,13,14,15,16,17-dodecahydro-1H-cyclopenta[a]phenanthren-3(2H)-one;
Trimethylammonium(3S,8S,9S,10R,11S,13S,14S,17S)-17-acetyl-11-hydroxy-10,13-dimethyl-2,3,4,7,8,9,10,11,12,13,14,15,16,17-tetradecahydro-1H-cyclopenta[a]phenanthren-3-yl sulfate;
(8S,9S,10R,11S,13S,14S,17S)-11-hydroxy-17-(2-hydroxyacetyl)-10,13-dimethyl-6,7,8,9,10,11,12,13,14,15,16,17-dodecahydro-1H-cyclopenta[a]phenanthren-3(2H)-one;
(8S,9S,10R,11S,13S,14S,17R)-11,17-dihydroxy-17-(2-hydroxyacetyl)-10,13-dimethyl-6,7,8,9,10,11,12,13,14,15,16,17-dodecahydro-1H-cyclopenta[a]phenanthren-3(2H)-one;
1-((3S,10R,11S,13S,17S)-3,11-dihydroxy-10,13-dimethyl-2,3,4,7,8,9,10,11, 12,13,14,15,16,17-tetradecahydro-1H-cyclopenta[a]phenanthren-17-yl)ethan-1-one;
(3S,10R,11S,13S,17S)-10,13-dimethyl-2,3,4,7,8,9,10,11,12,13,14,15,16,17-tetradecahydro-1H-cyclopenta[a]phenanthrene-3,11,17-triol;
(10R,11S,13S,17S)-11,17-dihydroxy-10,13-dimethyl-1,2,6,7,8,9,10,11, 12,13,14,15,16, 17-tetradecahydro-3H-cyclopenta[a]phenanthren-3-one;
(10R,11S,13S)-11-hydroxy-10,13-dimethyl-1,6,7,8,9,10,11,12,13,14,15,16-dodecahydro-3H-cyclopenta[a]phenanthrene-3,17(2H)-dione;
(10R,11S,13S)-11-hydroxy-10,13-dimethyl-3-oxo-2,3,6,7,8,9,10,11, 12,13,14,15,16,17-tetradecahydro-1H-cyclopenta[a]phenanthrene-17-carboxylic acid;
(10R,11S,13S)-17-(1-hydroxyethyl)-10,13-dimethyl-1,2,4,7,8,9,10,11,12, 13,14,15,16, 17 -tetradecahydrospiro[cyclopenta[a]phenanthrene-3,2'-[1,3]dioxolan]-11-ol;
(3Z,10R,113,133,17E)-11-hydroxy-10,13-dimethyl-1,6,7,8,9,10,11,12,13,14,15,16-dodecahydro-3H-cyclopenta[a]phenanthrene-3,17(2H)-dione dioxime;
(10R,11S,13S,17R)-11,17-dihydroxy-10,13-dimethyl-3-oxo-2,3,6,7,8,9,10,11,12,13, 14,15,16,17-tetradecahydro-1 H-cyclopenta[a]phenanthrene-17-carboxylic acid;
(10R,11S,13S,E)-11-hydroxy-17-(hydroxyimino)-10,13-dimethyl-1,2,6,7,8,9,10,11,12, 13,14,15,16,17-tetradecahydro-3H-cyclopenta[a]phenanthren-3-one;
(3S,10R,11S,13S,17S)-10,13-dimethyl-2,3,6,7,8,9,10,11,12,13,14,15,16,17-tetradecahydro-1H-cyclopenta[a]phenanthrene-3,11,17-triol;
(10R,11S,13S,17R)-11-hydroxy-10,13-dimethyl-1,6,7,8,9,10,11,12,13,14,15,16-dodecahydrospiro[cyclopenta[a]phenanthrene-17,2'-oxetane]-3,3'(2H)-dione;
1-((10R,11S,13S)-11-hydroxy-10,13-dimethyl-1,2,4,7,8,9,10,11,12,13,14,15,16,17-tetradecahydrospiro[cyclopenta[a]phenanthrene-3,2'-[1,3]dioxolan]-17-yl)ethan-1-one;
(10R,11S,13S)-11-hydroxy-N,N,10,13-tetramethyl-3-oxo-2,3,6,7,8,9,10,11,12, 13,14,15,16,17-tetradecahydro-1H-cyclopenta[a]phenanthrene-17-carboxamide;
(10R,11S,13S,17S,Z)-11-hydroxy-17-((Z)-1-(hydroxyimino)ethyl)-10,13-dimethyl-1,2,6,7,8,9,10,11,12,13,14,15,16,17-tetradecahydro-3H-cyclopenta[a]phenanthren-3-one oxime;
(10R,11S,13S)-11-hydroxy-17-(1-hydroxyethyl)-10,13-dimethyl-1,2,6,7,8,9,10,11,12, 13,14,15,16,17-tetradecahydro-3H-cyclopenta[a]phenanthren-3-one;
(10S,11S,13S)-11-hydroxy-N,N,10,13-tetramethyl-3-oxohexadecahydro-1H-cyclopenta-[a]phenanthrene-17-carboxamide;
(10S,11S,13S)-11-hydroxy-10,13-dimethyltetradecahydro-3H-cyclopenta[a]phenanthrene-3,17(2H)-dione;
(3S,10R,11S,13S)-17-(1-hydroxyethyl)-10,13-dimethyl-2,3,4,7,8,9,10,11,12,13,14,15,16,17-tetradecahydro-1H-cyclopenta[a]phenanthrene-3,11-diol;
(3S,8S,9S,10R,11S,13S,14S,17S)-17-(1-hydroxyethyl)-10,13-dimethyl-2,3,6,7,8,9,10,11,12,13,14,15,16,17-tetradecahydro-1H-cyclopenta[a]phenanthrene-3,11-diol;
1-((3S,10R,11S,13S,17S)-11-hydroxy-3-methoxy-10,13-dimethyl-2,3,4,7,8,9,10,11,12, 13,14,15,16,17-tetradecahydro-1H-cyclopenta[a]phenanthren-17-yl)ethan-1-one;
(10S,11S,13S)-17-(1-hydroxyethyl)-10,13-dimethylhexadecahydrospiro-[cyclopenta[a]-phenanthrene-3,2'-[1,3]dioxolan]-11-ol;
(3S,10S,11S,13S,17S)-17-(1-hydroxyethyl)-10,13-dimethylhexadecahydro-1H-cyclopenta-[a]phenanthrene-3,11-diol;
(3S,10R,11S,13R,17S)-17-ethyl-10,13-dimethyl-2,3,4,7,8,9,10,11,12,13,14,15,16,17-tetradecahydro-1H-cyclopenta[a]phenanthrene-3,11-diol;
1-((3S,10S,11S,13S,17S)-3,11-dihydroxy-10,13-dimethylhexadecahydro-1H-cyclopenta-[a]phenanthren-17-yl)ethan-1 -one; (3S,10R,11S,13S,17S)-3,11-dihydroxy-N,N,10,13-tetramethyl-2,3,4,7,8,9,10,11,12,-13,14,15,16,17-tetradecahydro-1H-cyclopenta[a]phenanthrene-17-carboxamide;
1-((8S,9S,10R,11S,13S,14S,17S)-11-hydroxy-10,13-dimethyl-2,7,8,9,10,11,12,13, 14,15,16,17-dodecahydro-1H-cyclopenta[a]phenanthren-17-yl)ethan-1-one;
1-((3S,10R,11S,13S,17S)-3,11-dihydroxy-10,13-dimethyl-2,3,4,7,8,9,10,11,12,13, 14,15,16,17-tetradecahydro-1H-cyclopenta[a]phenanthren-17-yl-3-d)ethan-1-one;
(3S,10R,11S,13S,17S)-3,11-dihydroxy-N,N,10,13-tetramethyl-2,3,6,7,8,9,10,11,12,13, 14,15,16,17-tetradecahydro-1H-cyclopenta[a]phenanthrene-17-carboxamide;
1-((3S,10R,11S,13S,17S)-3,11-dihydroxy-10,11,13-trimethyl-2,3,4,7,8,9,10,11,12,13,14,15,16,17-tetradecahydro-1H-cyclopenta[a]phenanthren-17-yl)ethan-1-one;
(10R,11S,13S)-11-hydroxy-17-(2-hydroxyacetyl)-10,13-dimethyl-1,2,6,7,8,9,10,11,12, 13,14,15,16,17-tetradecahydro-3H-cyclopenta[a]phenanthren-3-one;
(10R,11S,13S,17S)-11-hydroxy-17-(2-hydroxypropan-2-yl)-10,13-dimethyl-1,2,6,7,8,9, 10,11,12,13,14,15,16,17-tetradecahydro-3H-cyclopenta[a]phenanthren-3-one;
(E)-1-((3S,10R,11S,13S)-3,11-dihydroxy-10,13-dimethyl-2,3,4,7,8,9,10,11,12, 13,14,15,16,17-tetradecahydro-1H-cyclopenta[a]phenanthren-17-yl)ethan-1-one oxime;
1-((3S,10R,11S,13S,17S)-3,11-dihydroxy-10,13-dimethyl-2,3,4,7,8,9,10,11,12,13,14,15,16,17-tetradecahydro-1H-cyclopenta[a]phenanthren-17-yl-11-d)ethan-1-one;
(3S,10R,11S,13S,17S)-17-((E)-1-hydrazonoethyl)-10,13-dimethyl-2,3,4,7,8,9,10,11,12,13,14,15,16,17-tetradecahydro-1H-cyclopenta[a]phenanthrene-3,11-diol;
1-((10S,11S,13S,17S)-11-hydroxy-10,13-dimethylhexadecahydro-1H-cyclopenta-[a]phenanthren-17-yl)ethan-1-one;
(3S,10S,11S,13S)-17-(1-aminoethyl)-10,13-dimethylhexadecahydro-1H-cyclopenta-[a]phenanthrene-3,11-diol;
1-((3R,10R,11S,13S,17S)-3-fluoro-11-hydroxy-10,13-dimethyl-2,3,4,7,8,9,10,11,12,13,14,15,16,17-tetradecahydro-1H-cyclopenta[a]phenanthren-17-yl)ethan-1-one;
(3S,10S,11S,13S)-17-amino-10,13-dimethylhexadecahydro-1H-cyclopenta-[a]phenan-threne-3,11-diol;
(10R,11S,13S)-11-hydroxy-10,13-dimethyl-17-(piperazine-1-carbonyl)-1,2,6,7,8,9,10,11, 12,13,14,15,16,17-tetradecahydro-3H-cyclopenta[a]phenanthren-3-one;
(8S,9S,10R,11S,13S,14S,17S)-N-(2-aminoethyl)-11-hydroxy-10,13-dimethyl-3-oxo-2,3,6,7,8,9,10,11,12,13,14,15,16-tetradecahydro-1H-cyclopenta[a]phenanthrene-17-carboxamide;
1-((3S,10R,11S,13S,17S)-3,11-dihydroxy-10,13-dimethyl-2,3,4,7,8,9,10,11,12, 13,14,15,16,17-tetradecahydro-1H-cyclopenta[a]phenanthren-17-yl-3,11-d2)ethan-1-one;
(3S,8S,9S,10S,11S,13S,14S,17S)-3,11-dihydroxy-N,10,13-trimethyl-hexadecahydro-1H-cyclopenta[a]phenanthrene-17-carboxamide;
(3S,10R,11S,13S,17S)-2,3,6,7,8,9,10,11,12,13,14,15,16,17-tetradecahydro-10,13-dimethyl-1H-cyclopenta[a]phenanthrene-3,11,17-triol;
(3S,8S,9S,10S,11S,13S,14S,17S)-N-(2-aminoethyl)-3,11-dihydroxy-10,13-dimethylhexadecahydro-1H-cyclopenta[a]phenanthrene-17-carboxamide;
(3S,10R,11S,13S,17S)-5-methoxy-10,13-dimethyl-17-(2-methyl-1,3-dioxolan-2-yl)-hexadecahydro-1H-cyclopenta[a]phenanthrene-3,6,11-triol;
1-((3S,5R,6R,10R,11S,13S,17S)-3,5,11-trihydroxy-6-methoxy-10,13-dimethylhexadecahydro-1 H-cyclopenta[a]phenanthren-17-yl)ethan-1-one;
1-((3S,5R,6R,10R,11S,13S,17S)-3,5,11-trihydroxy-6-methoxy-10,13-dimethylhexadecahydro-1 H-cyclopenta[a]phenanthren-17-yl)ethan-1-one;
1-((3S,5R,6R,10R,11S,13S, 17S)-3,5,6,11-tetrahydroxy-10,13-dimethylhexadecahydro-1H-cyclopenta[a]phenanthren-17-yl)ethan-1-one;and
(3S,10R,11S,13S,17S)-17-(1-hydroxyethyl)-10,13-dimethyl-2,3,6,7,8,9,10,11,12,13, 14,15,16,17-tetradecahydro-1H-cyclopenta[a]phenanthrene-3,11-diol;

5. A pharmaceutical composition comprising a compound as claimed in Claim 1 together with a pharmaceutically acceptable carrier.

6. A compound as claimed in claim 1 for use in the treatment of diseases associated with mitochondrial depletion and/or dysfunction selected from the group consisting of skeletal muscle diseases, cardiac muscle diseases associated with ischemia, or impaired or inadequate blood flow, diseases associated with genetic disorders that directly or indirectly affect the number, structure, or function of mitochondria, diseases associated with impaired neurological function associated with decreased mitochondrial number or function, diseases associated with loss of number, loss of function, or loss of correct, optimally efficient internal organization of skeletal muscle cells or cardiac muscle cells, metabolic diseases, and conditions associated with liver cell injury and altered fatty acid metabolism.

7. A compound as claimed in claim 1 for use in the treatment of a mitochondrial biogenesis-mediated disease selected from the group consisting of acute coronary syndrome, myocardial infarction, angina, renal injury, renal ischemia, diseases of the aorta and its branches, injuries arising from medical interventions, atherosclerosis, trauma, diabetes, hyperlipidemia, vascular stenosis, peripheral arterial disease, vasculopathy, and vasculitis, Friedreich's ataxia, muscular dystrophy, Duchenne muscular dystrophy, Becker muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, spinal muscular atrophy, Emery-Dreifuss muscular dystrophy, Huntington's disease, Parkinson's disease, Alzheimer's disease, and amyotrophic lateral sclerosis, sarcopenia, congestive heart failure, aging, myocarditis, myositis, polymyalgia rheumatic, polymyositis, HIV, cancer and/or the side effects of chemotherapy targeting the cancer, malnutrition, aging, inborn errors of metabolism, trauma, and stroke or other types of neurological impairment, hepatic steatosis, hepatic fibrosis, cirrhosis, and hepatocyte or stellate cell injury.

8. The compound as claimed in claim 1 for use in a dosage in the range of 0.01 to 100mg/Kg for oral or parenteral administration.
